# EUROPEAN PATENT APPLICATION

(11) **EP 3 835 306 A1**
(43) Date of publication of application: **16.06.2021**
(21) Application number: 19216206.3
(22) Date of filing: 13.12.2019
(51) Int. Cl.: C07H 1/00, C07H 3/06, B01J 19/12

(54) **MICROWAVE-ASSISTED METHOD FOR SYNTHESIS OF OLIGO- AND POLYSACCHARIDES ON SOLID PHASE**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a method for synthesizing oligo- and polysaccharides using microwave radiation, in particular to a method and a device for automated synthesis of oligo- and polysaccharides.

## Description

### Field of the invention

The present invention relates to a method for synthesizing oligo- and polysaccharides using microwave radiation, in particular to a method and a device for automated synthesis of oligo- and polysaccharides.

### Background of the invention

Certain biopolymers, including polypeptides and polynucleotides are routinely synthesized by solid-phase methods in which individual subunits are added stepwise to a growing chain tethered to a solid support. Those approaches can be carried out using commercially available synthesizers in an automated or semi-automated manner. However, those general synthesizers do not allow the efficient synthesis of oligosaccharides on a solid support and typically result in poor yields and selectivity, due to the lack of control of reaction temperature.

Polysaccharides are the most abundant biomolecules on Earth and are essential for many vital biological functions. Energy, structural support, cell proliferation and differentiation, host-pathogen recognition and cancer invasion are among the numerous areas that oligosaccharides and glycoconjugates participate and regulate. Because biochemical techniques to purify pure oligosaccharides from natural sources are difficult and inefficient, chemical and enzymatic approaches, or combinations thereof, are often the only reliable methods to access pure oligosaccharides. Efforts to reduce the time and resources spent on traditional chemical syntheses have focused mainly on automated glycan assembly (AGA) on solid supports.

The advantages of solid-support synthesis have been appreciated since the development of peptide synthesis by Merrifield and oligonucleotide synthesis on polymer supports developed by Caruthers. Automated synthesis machines carry out the reaction sequences and the reactions are carried out in high yields by using excess reagents, which are simply removed by washing steps. Separation from the solid carrier and purification as post-automation steps are typically standardized. The keys to successful automated synthesis are reliable building blocks and resins, high-yield couplings and reliable instrumentation.

Since the introduction of AGA by Seeberger in 2001, many aspects of the synthesis process have been systematically improved. Synthetic glycans of increasing length and complexity up to a chain length of 50 units were assembled with the help of AGA. The quality control of the stereo- and regiochemical composition of synthetic products was greatly accelerated by the use of ion mobility mass spectrometry (IM-MS). The overall AGA process was greatly improved and led to the development of the first commercial Glyconeer 2.1® automated oligosaccharide synthesizer.

Depending on the molecular weight of the sugar, 10-50 mg of completely protected oligosaccharide is obtained from the resin in this batch size. After deprotection and purification of the final product, often only 2-15 mg of the desired oligosaccharide product remains. These amounts are often sufficient for biochemical investigations and have been successfully used to produce glycan arrays. However, significantly more material is required for studies in other scientific areas. Carbohydrates in the form of cellulose and chitin are the two dominant biopolymers that require 100 mg or more of synthetic compounds to investigate their material properties.

Microwave-assisted synthesis has been a fundamental incorporation into automated synthesizers providing higher yields while suppressing the formation of byproducts. Shimizu et al. (Tetrahedron 64 (2008) 10091-10096) have utilized microwave irradiation in glycosylation reactions of disaccharide and trisaccharide building blocks for Lewis X oligosaccharide in a microwave system equipped with a cooling unit. The authors have shown that cooling was necessary for carrying out the glycosylation reactions.

Besides the glycosylation reaction, during a typical AGA cycle there are also several auxiliary reaction steps (e.g. protecting group manipulations including, capping reactions with acetyl groups and deprotection reactions). These auxiliary steps increase the overall yield of the final oligo- or polysaccharide by terminating unreacted acceptors (in case of capping reactions) as well as they remove temporary protecting groups which is a prerequisite for the next coupling cycle. These auxiliary steps have been an unnecessary bottleneck in AGA, as prolonged reaction times are required in each AGA cycle, rendering the duration of a single cycle to over 100 minutes. Moreover, often only moderate yields were observed in auxiliary steps.

It is therefore the objective of the present invention to provide an improved and rapid method for synthesizing oligo- and polysaccharides on a solid support. In particular the objective of the present invention is to provide an improved and rapid automated method for synthesizing oligo- and polysaccharides on a solid support. Especially desired are a method and a device for easily scaling up the synthesis of oligo- and polysaccharides on a solid support, shortening the reaction times, increasing yield and decreasing side and decomposition products.

The objective of the present invention is solved by the teachings of the independent claims. Further advantageous features, aspects and details of the invention are evident from the dependent claims, the description, the figures, and the examples of the present application.

### Brief description of the invention

The present invention relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support; and
c) performing removal of the at least one protecting group of the further saccharide under microwave irradiation.

The inventors have found that the chemical reaction time for synthesis of oligo- and polysaccharides on a solid support can be significantly reduced if the auxiliary steps are conducted under microwave irradiation. These auxiliary steps have been a bottleneck in the preparation of longer oligo- and polysaccharides, as they consume a significant amount of the overall time required for each AGA cycle. A typical coupling cycle in a glycan synthesis comprises a glycosylation step and several protecting group manipulations including a capping reaction in order to terminate unreacted saccharides and deprotection of a temporary protecting group. The term "auxiliary steps" as used herein is referred to the protecting group manipulations. The glycosylation or coupling reaction step is not an auxiliary step. The usual reaction time of auxiliary steps for an AGA cycle is 50 minutes, whereas in the inventive methods described herein, the reaction time is reduced to 10 - 15 minutes. In addition to the beneficial shorter reaction time, the reaction steps performed under microwave irradiation remained orthogonal and only few side reactions were observed. Thus, the present invention relates to a method for synthesizing oligo- and polysaccharides on a solid support, wherein the auxiliary steps of the coupling cycle are performed under microwave irradiation.

Surprisingly, the inventors could further show that performing the glycosylation reaction, i.e. the coupling of the further saccharide to the saccharide immobilized on the solid support, without microwave irradiation requires reaction times between 5-20 min, which is already significantly shorter than the observations reported in the prior art with reaction times between 60-360 min for glycosylation coupling performed under microwave irradiation. In addition, the inventors observed that the main advantage of using microwave radiation during the coupling cycle is the fast adjustment of the temperature of the reagents inside of the microwave transparent reaction vessel when the microwave heating is combined with simultaneous active cooling. Thus, in one embodiment of the inventive method described herein, step b) is performed without microwave irradiation.

In a further embodiment of the inventive methods described herein, the solid support obtained in step b) is treated with a capping reagent in order to block or cap free hydroxyl groups of the immobilized saccharide. By using a capping step elongation of failure sequences of the oligo- or polysaccharide during the synthesis cycles is avoided and the difficult separation of deletion sequences missing only one saccharide unit (n-1) can be circumvented. Thus, in one embodiment the method comprises the steps of:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
b') treating the solid support obtained in step b) with a capping reagent in order to deactivate or block free hydroxyl groups at the immobilized saccharide; and
c) performing removal of the at least one protecting group of the further saccharide under microwave irradiation.

Preferably, the capping step b') is performed under microwave irradiation.

In one embodiment of the present invention the saccharide is immobilized on the solid support via a photo-cleavable linker.

Preferably, the coupling reaction in step b) is performed at a reaction temperature below 0°C. Preferably, the glycosylation reagent is cooled to a temperature of at least 0°C before addition to the further saccharide and the solid support.

Preferably, the removal of the at least one protecting group in step c) is performed at a reaction temperature of at least 40°C.

In a preferred embodiment of the present invention, the method further comprises the steps:
d) cleaving the saccharide obtained in step c) from the solid support;
e) performing removal of at least one permanent protecting group of the saccharide obtained in step d); and
f) purifying the saccharide obtained in step e).

The present invention further relates to an automated synthesizer (100) comprising a microwave transparent reaction vessel (400), a microwave generator component (500), a reagent storing component (660), a reagent delivery system (600), and a cooling device (350) for cooling the microwave transparent reaction vessel. Preferably, the automated synthesizer may further comprise a pre-cooling device (300) for pre-cooling the reagents to be supplied.

### Description of the invention

Solid phase synthesis of oligo- or polysaccharides has been proven as suitable synthesis strategy for synthesizing oligo- and polysaccharides, in particular in connection with automation of the synthesis process. For solid phase synthesis of oligo- or polysaccharides the repetitive nature of glycosylation and deprotection can easily be framed into a coupling cycle. There is a long-felt need for an efficient method for synthesizing oligo- or polysaccharides, in particular an efficient automated method for synthesizing oligo- or polysaccharides.

As used herein, the term **"glycosylation reaction"** or **"coupling reaction"** relates in the most general sense to a formation of an acetal connecting two sugar units. The anomeric substituent of a glycosyl donor acts as a leaving group thereby generating an electrophilic intermediate. Reaction of this species with a nucleophile, typically a hydroxyl group or amine group of a glycosyl acceptor, leads to the formation of a glycosidic linkage. Thus, the term "glycosylation reaction" or "coupling reaction" refers to reactions between a glycosyl donor and a glycosyl acceptor, wherein the reducing end of the donor reacts with a free hydroxy or amine group of the acceptor. Thus, O-glycosylation or *N*-glycosylation methods are preferably employed in the coupling reaction of the method according to the invention. More preferably, O-glycosylation methods are employed in the coupling reaction of the method according to the invention. These glycosylation methods are known from the state of the art. Generally, they require a leaving group at the reducing end of the donor, which is activated in the presence of a catalyst. The glycosylation reactions take place upon treatment of a donor and an acceptor with a **"glycosylation reagent"** which acts as an activator or an activating agent. Glycosylation reagents known to the skilled person include, but are not restricted to: AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST).

The term **"coupling cycle"** or **"synthesis cycle"** or **"glycosylation cycle"** as used herein relates to an iterative or repetitive sequence of coupling reactions and deprotection steps in the synthesis of oligo- or polysaccharides. In this synthesis strategy, the carbohydrate chain is elongated in a stepwise manner and thus gradually extended through each synthesis cycle until the desired oligo- or polysaccharide is obtained.

As used herein, the term **"saccharide"** refers to but is not restricted to monosaccharide, disaccharide, trisaccharide, tetrasaccharide, pentasaccharide, hexasaccharide, heptasaccharide, octasaccharide..., oligosaccharide, polysaccharide and glycan. The saccharide comprises preferably monosaccharide units selected from:
D-Arabinose, D-Lyxose, D-Ribose, D-Xylose, L-Arabinose, L-Lyxose, L-Ribose, L-Xylose, D-Ribulose, D-Xylulose, L-Ribulose, L-Xylulose, D-Deoxyribose, L-Deoxyribose, D-Erythrose, D-Threose, L-glycero-D-manno-Heptose, D-glycero-D-manno-Heptose, D-Allose, D-Altrose, D-Glucose, D-Mannose, D-Gulose, D-ldose, D-Galactose, D-Talose, D-psicose, D-fructose, D-sorbose, D-tagatose, 6-Deoxy-L-altrose, 6-Deoxy-D-talose, D-Fucose, L-Fucose, D-Rhamnose, L-Rhamnose, D-Quinovose, Olivose, Tyvelose, Ascarylose, Abequose, Paratose, Digitoxose, Colitose, D-Glucosamine, D-Galactosamine, D-Mannosamine, D-Allosamine, L-Altrosamine, D-Gulosamine, L-Idosamine, D-Talosamine, N-Acetyl-D-glucosamine, N-Acetyl-D-galactosamine, N-Acetyl-D-mannosamine, N-Acetyl-D-allosamine, N-Acetyl-L-altrosamine, N-Acetyl-D-gulosamine, N-Acetyl-L-idosamine, N-Acetyl-D-talosamine, N-Acetyl-D-fucosamine, N-Acetyl-L-fucosamine, N-Acetyl-L-rhamnosamine, N-Acetyl-D-quinovosamine, D-Glucuronic acid, D-Galacturonic acid, D-Mannuronic acid, D-Alluronic acid, L-Altruronic acid, D-Guluronic acid, L-Guluronic acid, L-Iduronic acid, D-Taluronic acid, Neuraminic acid, N-Acetylneuraminic acid, N-Glycolylneuraminic acid, Apiose, Bacillosamine, Thevetose, Acofriose, Cymarose, Muramic acid, N-Acetylmuramic acid, N-Glycolylmuramic acid, 3-Deoxy-lyxo-heptulosaric acid, Ketodeoxyoctonic acid, and Ketodeoxynononic acid. Preferably the monosaccharide units belong to the following group of α- and β-D/L-carbohydrates comprising or consisting of:
α -D-ribopyranose, α-D-arabinopyranose, α-D-xylopyranose, α-D-lyxopyranose, α-D-allopyranose, α-D-altropyranose, α-D-glucopyranose, α-D-mannpyranose, α-D-glucopyranose, α-D-idopyranose, α-D-galactopyranose, α-D-talopyranose, α-D-psicopyranose, α-D-fructopyranose, α-D-sorbopyranose, α-D-tagatopyranose, α-D-ribofuranose, α-D-arabinofuranose, α-D-xylofuranose, α-D-lyxofuranose, α-D-Allofuranose, α-D-Altrofuranose, α-D-Glucofuranose, α-D-Mannofuranose, α-D-gulofuranose, α-D-idofuranose, α-D-galactofuranose, α-D-talofuranose, α-D-psicofuranose, α-D-fructofuranose, α-D-sorbofuranose, α-D-tagatofuranose, α-D-xylulofuranose, α-D-ribulofuranose, α-D-threofuranose, α-D-rhamnopyranose, α-D-erythrofuranose, α-D-glucosamine, α-D-glucopyranuronic acid, β-D-ribopyranose, β-D-arabinopyranose, β-D-xylopyranose, β-D-lyxopyranose, β-D-allopyranose, β-D-altropyranose, β-D-glucopyranose, β-D-mannpyranose, β-D-glucopyranose, β-D-idopyranose, β-D-galactopyranose, β-D-talopyranose, β-D-psicopyranose, β-D-fructopyranose, β-D-sorbopyranose, β-D-tagatopyranose, β-D-ribofuranose, β-D-arabinofuranose, β-D-xylofuranose, β-D-lyxofuranose, β-D-rhamnopyranose, β-D-allofuranose, β-D-altrofuranose, β-D-glucofuranose, β-D-mannofuranose, β-D-gulofuranose, β-D-idofuranose, β-D-galactofuranose, β-D-talofuranose, β-D-psicofuranose, β-D-fructofuranose, β-D-sorbofuranose, β-D-tagatofuranose, β-D-xylulofuranose, β-D-ribulofuranose, β-D-threofuranose, β-D-erythrofuranose, β-D-glucosamine, β-D-glucopyranuronic acid, α-L-ribopyranose, α-L-arabinopyranose, α-L-xylopyranose, α-L-lyxopyranose, α-L-allopyranose, α-L-altropyranose, α-L-glucopyranose, α-L-mannpyranose, α-L-glucopyranose, α-L-idopyranose, α-L-galactopyranose, α-L-talopyranose, α-L-psicopyranose, α-L-fructopyranose, α-L-sorbopyranose, α-L-tagatopyranose, α-L-rhamnopyranose, α-L-ribofuranose, α-L-arabinofuranose, α-L-xylofuranose, α-L-lyxofuranose, α-L-Allofuranose, α-L-Altrofuranose, α-L-Glucofuranose, α-L-Mannofuranose, α-L-gulofuranose, α-L-idofuranose, α-L-galactofuranose, α-L-talofuranose, α-L-psicofuranose, α-L-fructofuranose, α-L-sorbofuranose, α-L-tagatofuranose, α-L-xylulofuranose, α-L-ribulofuranose, α-L-threofuranose, α-L-erythrofuranose, α-L-glucosamine, α-L-glucopyranuronic acid, β-L-ribopyranose, β-L-arabinopyranose, β-L-xylopyranose, β-L-lyxopyranose, β-L-allopyranose, β-L-altropyranose, β-L-glucopyranose, β-L-mannpyranose, β-L-glucopyranose, β-L-idopyranose, β-L-galactopyranose, β-L-talopyranose, β-L-psicopyranose, β-L-fructopyranose, β-L-sorbopyranose, β-L-tagatopyranose, β-L-ribofuranose, β-L-arabinofuranose, β-L-xylofuranose, β-L-lyxofuranose, β-L-allofuranose, β-L-altrofuranose, β-L-glucofuranose, β-L-mannofuranose, β-L-gulofuranose, β-L-idofuranose, β-L-galactofuranose, β-L-talofuranose, β-L-psicofuranose, β-L-fructofuranose, β-L-sorbofuranose, β-L-tagatofuranose, β-L-xylulofuranose, β-L-ribulofuranose, β-L-threofuranose, β-L-erythrofuranose, β-L-glucosamine, β-L-glucopyranuronic acid, and β-L-rhamnopyranose.

The saccharides are further optionally modified to carry amide, carbonate, carbamate, carbonyl, thiocarbonyl, carboxy, thiocarboxy, ester, thioester, ether, epoxy, hydroxyalkyl, alkylenyl, phenylene, alkenyl, imino, imide, isourea, thiocarbamate, thiourea and/or urea moieties.

As used herein the term **"saccharide building block"** or "building block" refers to a saccharide acceptor or saccharide donor, i.e. to a saccharide that is capable of forming a glycosidic bond when being exposed to a glycosylating agent. The saccharide building block can be fully protected (i.e. each hydroxy or amino group is blocked by a protecting group), partially protected (i.e. at least one hydroxy or amino groups is blocked by a protecting group) or unprotected (i.e. none of hydroxy or amino groups is blocked by a protecting group). For forming an amide bond with an anchoring group present on the solid support the saccharide building block may also be modified at the reducing end with a suitable functional group such as a carboxylic acid (e.g. hydroxyacetate), azide, alkyne, thiol or an amine (e.g. aminopropyl or aminopentyl). Any known saccharide building block can be employed in the inventive methods described herein, including saccharides, glycopeptides or glycopeptoids as described in Beilstein J. Org. Chem. 2014, 10, 2453-2460. The building blocks for the coupling reaction may be provided as glycosyl donor or glycosyl acceptor molecules. Glycosyl donor building blocks relate to saccharides having a suitable leaving group at the anomeric position, and may be used as glycosyl donors for the coupling reaction. Glycosyl acceptor building blocks relate to saccharides with at least one unprotected hydroxyl group or amine group, and may be used as glycosyl acceptors for the glycosylation reaction.

**"Alkyl"** refers to saturated hydrocarbon groups of from 1 to 18 carbon atoms, either straight chained or branched, more preferably from 1 to 8 carbon atoms, and most preferably 1 to 6 carbon atoms. An alkyl with a specified number of carbon atoms is denoted as C₁-C₈ alkyl and refers to a linear C₁-C₈ alkyl of -CH₃, -C₂H₅, -C₃H₇, -C₄H₉, -C₅H₁₁, -C₆H₁₃, -C₇H₁₅, -C₈H₁₇, -CH₂-Ph, -CH₂-CH₂-Ph or a branched C₁-C₈ alkyl or preferably branched C₃-C₈ alkyl of -CH(CH₃)₂, -CH₂-CH(CH₃)₂, -CH(CH₃)-C₂H₅, -C(CH₃)₃, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH(CH₃)₂, -C₃H₆-CH(CH₃)-C₂H₅, -C₃H₆-CH(CH₃)-C₂H₅, -C₂H₄-CH(CH₃)-C₃H₇, -CH₂-CH(CH₃)-C₄H₉, -CH(CH₃)-C₅H_{H}, -CH(C₂H₅)-C₄H₉, -C₂H₄-CH(CH₃)-C₃H₇, -CH₂-CH(C₂H₅)-C₃H₇, -CH₂-CH(CH₃)-C₄H₉, -CH₂-CH(CH₃)-CH₂-CH(CH₃)₂, -CH(C₂H₅)-CH₂-CH(CH₃)₂, -CH(CH₃)-C₂H₄-CH(CH₃)₂, -CH(CH₃)-CH₂-CH(CH₃)-C₂H₅, -CH₂-CH(CH₃)-CH(CH₃)-C₂H₅, -CH(CH₃)-CH₂-CH(CH₃)-C₂H₅, -CH(CH₃)-CH(C₂H₅)-C₂H₅, -CH(C₂H₅)-CH(CH₃)-C₂H₅, -CH(CH₃)-CH(CH₃)-C₃H₇, -C₂H₄-CH(CH₃)-CH(CH₃)₂, -CH₂-CH(C₂H₅)-CH(CH₃)₂, -CH₂-CH(CH₃)-CH₂-CH(CH₃)₂, -CH₂-CH(CH₃)-CH(CH₃)-C₂H₅, -C₂H₄-C(CH₃)₂-C₂H₅, -CH₂-C(CH₃)(C₂H₅)₂, -CH₂-C(CH₃)₂-C₃H₇, -CH₂-C(CH₃)₂-C₃H₇, -C(CH₃)(C₂H₅)-C₃H₇, -C(CH₃)₂-C₄H₉, -CH₂-C(CH₃)₂-CH(CH₃)₂, -C(CH₃)(C₂H₅)-CH(CH₃)₂, -C(CH₃)₂-CH₂-CH(CH₃)₂, -C(CH₃)₂-C(CH₃)₃, -C(CH₃)₂-CH(CH₃)-C₂H₅, -C₃H₆-C(CH₃)₃, -C₂H₄-C(CH₃)₂-C₂H₅, -CH₂-CH(CH₃)-C(CH₃)₃, -CH(C₂H₅)-C(CH₃)₃, -CH(CH₃)-CH₂-C(CH₃)₃, -CH(CH₃)-C(CH₃)₂-C₂H₅, -C₅H₁₀-CH(CH₃)₂, -C₄H₈-C(CH₃)₃, -C₄H₈-CH(CH₃)-C₂H₅, -C₄H₈-CH(CH₃)-C₂H₅, -C₃H₆-C(CH₃)₂-C₂H₅, -C₃H₆-CH(C₂H₅)-C₂H₅, -C₃H₆-CH(CH₃)-C₃H₇, -C₂H₄-C(CH₃)₂-C₃H₇, -C₂H₄-CH(C₂H₅)-C₃H₇, -C₂H₄-CH(CH₃)-C₄H₉, -CH₂-C(CH₃)₂-C₄H₉, -CH₂-CH(C₂H₅)-C₄H₉, -CH₂-CH(CH₃)-C₅H₁₁, -C(CH₃)₂-C₅H,₁, -CH(CH₃)-C₆H₁₃, -CH(C₃H₇)-C₄H₉, -CH(C₂H₅)-C₅H₁₁, -CH₂-C(CH₃)(C₂H₅)-C₃H₇, -C₂H₄-CH(CH₃)-CH₂-CH(CH₃)₂, -CH₂-C(CH₃)₂-CH₂-CH(CH₃)₂, -CH₂-CH(C₂H₅)-CH₂-CH(CH₃)₂, -CH₂-CH(CH₃)-C₂H₄-CH(CH₃)₂, -CH₂-CH(CH₃)-CH₂-C(CH₃)₃, -CH₂-CH(CH₃)-CH₂-CH(CH₃)-C₂H₅, -C(CH₃)(C₂H₅)-CH₂-CH(CH₃)₂, -CH(C₃H₇)-CH₂-CH(CH₃)₂, -CH(C₂H₅)-C₂H₄-CH(CH₃)₂, -CH(C₂H₅)-CH₂-C(CH₃)₃, -CH(C₂H₅)-CH₂-CH(CH₃)-C₂H₅, -CH₂-CH(CH₃)-C₂H₄-CH(CH₃)₂, -C(CH₃)₂-C₂H₄-CH(CH₃)₂, -CH(C₂H₅)-C₂H₄-CH(CH₃)₂, -CH(CH₃)-C₃H₆-CH(CH₃)₂, -CH(CH₃)-C₂H₄-C(CH₃)₃, -CH(CH₃)-C₂H₄-CH(CH₃)-C₂H₅, -CH₂-CH(CH₃)-CH₂-CH(CH₃)-C₂H₅, -C(CH₃)₂-CH₂-CH(CH₃)-C₂H₅, -CH(CH₃)-C₂H₄-CH(CH₃)-C₂H₅, -CH(CH₃)-CH₂-C(CH₃)₂-C₂H₅, -CH(CH₃)-CH₂-CH(CH₃)-C₃H₇, -C₂H₄-CH(CH₃)-CH(CH₃)-C₂H₅, -CH₂-C(CH₃)₂-CH(CH₃)-C₂H₅, -CH₂-CH(C₂H₅)-CH(CH₃)-C₂H₅, -CH₂-CH(CH₃)-CH₂-CH(CH₃)-C₂H₅, -CH₂-CH(CH₃)-C(CH₃)₂-C₂H₅, -CH₂-CH(CH₃)-CH(C₂H₅)₂, -C₃H₆-CH(CH₃)-CH(CH₃)₂, -C₂H₄-C(CH₃)₂-CH(CH₃)₂, -C₂H₄-CH(C₂H₅)-CH(CH₃)₂, -C₂H₄-CH(CH₃)-C(CH₃)₃, -C₂H₄-CH(CH₃)-CH(CH₃)-C₂H₅, -C₃H₆-C(CH₃)₂-C₂H₅, -C₂H₄-C(CH₃)₂-C₃H₇, -CH₂-C(CH₃)(C₂H₅)₂, -C₂H₄-C(C₂H₅)₃, -C₂H₄-C(CH₃)₂-C₃H₇, -CH₂-C(CH₃)₂-C₄H₉, -C(C₂H₅)₂-C₃H₇, -C(CH₃)(C₃H₇)-C₃H₇, -C(CH₃)(C₂H₅)-C₄H₉, -C(CH₃)(-C₂H₅)-C₄H₉, -C(CH₃)₂-C₅H₁₁, -C₂H₄-C(CH₃)₂-CH(CH₃)₂, -CH₂-C(CH₃)₂-C(CH₃)₃, -C(C₂H₅)₂-CH(CH₃)₂, -C(CH₃)(C₃H₇)-CH(CH₃)₂, -C(CH₃)(C₂H₅)-C(CH₃)₃, -CH₂-C(CH₃)₂-CH₂-CH(CH₃)₂, -C(CH₃)₂-C₂H₄-CH(CH₃)₂, -C(CH₃)₂-CH₂-C(CH₃)₃, -CH₂-C(CH₃)₂-C(CH₃)₃, -C₄H₈-C(CH₃)₃, -C₃H₆-C(CH₃)₂-C₂H₅, -C₂H₄-C(CH₃)₂-C₃H₇, -C₂H₄-CH(CH₃)-C(CH₃)₃, -CH₂-C(CH₃)₂-C(CH₃)₃.

**"Aryl"** refers to an unsaturated aromatic carbocyclic group of from 6 to 12 carbon atoms inclusively having a single ring (e.g., phenyl) or multiple condensed rings (e.g., naphthyl or anthryl). Exemplary aryls include phenyl, pyridyl, naphthyl and the like.

As used herein, the term "donor" or **"glycosyl donor"** relates to a saccharide having a suitable leaving group at the anomeric position, which may be used as reactant for the coupling reaction. A donor or glycosyl donor refers to a saccharide that forms a glycal, or a saccharide comprising an epoxide or orthoester group or a saccharide that contains a leaving group at the reducing end. Suitable leaving groups include halogen, -O-C(=NH)-CCl₃, -O-C(=NPh)-CF₃, -OAc, -SR⁵, -SO-Ph, -SO₂-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 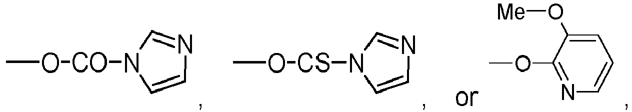 wherein R⁵ represents an alkyl or aryl group.

The term **"glycosyl acceptor"** as used herein relates to a saccharide that contains at least one free hydroxy or amine function and is capable of forming a glycosidic bond with a glycosyl donor under suitable reaction conditions.

The term **"protecting group"** or "protective group" as used herein refers to commonly used groups in organic synthesis, preferably used for protection of amines, hydroxyl groups, thiols, imines, carbonyls, carboxyls or other common functional groups, and particularly preferred for amines and hydroxyl groups. The protecting groups are characterized in that they are stable under reaction conditions applied during the synthesis, i.e. they are not cleaved off or undergo undesired side reactions and prevent any reaction of the protected functional group they are bonded to. Additionally, the protecting groups are selected to not hinder or to not affect the performed reaction steps in terms of yield or stereoselectivity.

Preferred protecting groups for hydroxyl groups are acetyl, phenyl, benzyl, isopropylidene, benzylidene, benzoyl, *p*-methoxybenzyl, *p*-methoxybenzylidene, *p*-methoxyphenyl, *p*-bromobenzylidene, *p*-nitrophenyl, allyl, allyloxycarbonyl, monochloroacetyl, isopropyl, p-bromobenzyl, dimethoxytrityl, trityl, 2-naphthylmethyl, pivaloyl, triisopropylsilyl, *tert*-butyldimethylsilyl, *tert-*butyldiphenylsilyl, *tert*-butylmethoxyphenylsilyl, triethylsilyl, trimethylsilyl, 2-trimethylsilylethoxymethyl, 9-fluorenylmethoxycarbonyl, tert-butyloxycarbonyl, benzyloxymethyl, methyloxymethyl, *tert*-butyloxymethyl, methoxyethyloxymethyl, and levulinoyl.

The protecting groups can be differentiated in "permanent protecting groups" and "temporary protecting groups". Permanent protecting groups are protecting groups that are stable during the entire synthesis and that can be efficiently removed at the late stage of the synthesis. In this case, permanent protecting groups are masking the hydroxyl groups and amino groups, if present, during the entire synthesis. Preferably permanent protecting groups are benzyl, benzoyl, acetyl, allyloxycarbonyl (alloc) and benzyloxycarbonyl group (Cbz).

The term "temporary protecting group" as used herein relates to a protecting group of a protected saccharide which may be selectively removed from the protected saccharide, wherein the remaining further protecting groups or the "permanent protecting groups" are not removed under the same conditions. Preferably, removal of a temporary protecting group allows conversion of the protected saccharide to a saccharide having at least one unprotected hydroxyl group or amine function. Thus, a glycosyl acceptor for use in a subsequent coupling reaction may be provided by removing a temporary protecting group of the protected saccharide.

The temporary protecting groups are generally orthogonal protecting groups that can be selectively removed at different levels of the synthesis to free hydroxyl groups for subsequent introduction of different substituents, including monosaccharides, other protecting groups or other residues present on the molecule. Temporary protecting groups are preferably selected from, but are not restricted to: allyl, p-methoxybenzyl, 2-naphthylmethyl, tri-isopropylsilyl, *tert-*butyldimethylsilyl, tert-butylmethoxyphenylsilyl, triethylsilyl, trimethylsilyl, 2-trimethylsilylethoxymethyl, 9-fluorenylmethoxycarbonyl and levulinoyl.

The ingenious choice of protecting groups allows expedient access to a library of saccharides. It is apparent for a skilled person to choose the protecting groups in such a manner that they can be removed from the saccharide without cleaving the saccharide from the solid support.

The term **"deprotection"** as used herein relates to the removal of one or more protecting groups of a molecule functionalized with one or more protecting groups. The deprotection step in a coupling cycle preferably relates to the removal of a temporary protecting group of a protected saccharide. As a saccharide typically exhibits several hydroxyl groups it is preferred that only one of said hydroxyl groups participates as nucleophile in the coupling reaction. Therefore, orthogonal protecting group strategy is typically applied to provide saccharide having only one free hydroxyl group or amine function, wherein the protecting groups of the remaining hydroxyl groups, the permanent protecting groups, are not removed. Orthogonal protecting group strategies are often the key step of a successful regioselective functionalization of a carbohydrate molecule. Suitable protecting groups and orthogonal protecting group strategies are therefore well known to the skilled person.

The term **"capping"** as used herein relates to blocking or capping of free hydroxyl groups by a highly reactive blocking group to avoid elongation of failure sequences of the oligo- or polysaccharide during the coupling cycles. Deletion sequences missing just one sugar unit (n-1) are the most difficult to separate from the desired product and arise from incomplete coupling steps during any coupling cycle of the sequence. The oligosaccharide chains that fail to couple during one cycle, may be successfully glycosylated during the following elongation steps. Therefore, a severe purification problem may exist at the end of the synthesis. To avoid the elongation of failure sequences, a capping step (i.e., a blocking step) may be included into the coupling cycle. After each completed coupling, a highly reactive blocking group can be used to cap any free hydroxyl acceptors. For example, benzyl trichloroacetimidate can be employed as a capping reagent (activated with TMSOTf) to yield benzyl ethers in positions that were not glycosylated and render them unreactive throughout the synthesis. Using this straightforward capping step, the purification of the finished oligosaccharide products may be greatly simplified, since the presence of (n-1)-deletion sequences will be minimized.

The term **"solid support"** as used herein refers to an insoluble, functionalized, polymeric material to which saccharides or other reagents may be attached or immobilized, directly or via a linker bearing an anchoring group, allowing saccharides to be readily separated (by washing, filtration, centrifugation, etc.) from excess reagents, soluble reaction by-products, or solvents. The solid support has preferably the form of beads or Controlled Pore Glass.

The inventors of the present invention considered the following key issues for the improvement of a method for synthesizing oligo- and polysaccharides: a synthesis strategy with either the reducing or the non-reducing end of the growing saccharide chain attached to a solid support, selection of a polymer and linker that are inert to all reaction conditions during the synthesis but cleaved efficiently when desired, a protecting group strategy consistent with the complexity of the target oligo- or polysaccharide, stereospecific and high yielding glycosylation reactions, and rapid and efficient, high yielding deprotection and capping steps.

Surprisingly, it has been found that a method for synthesizing oligo- and polysaccharides, comprising the steps of providing a solid support with at least one immobilized saccharide; and adding a further saccharide bearing at least one temporary protecting group and a glycosylation reagent to the solid support in order to initiate a coupling reaction of the further saccharide to the saccharide immobilized on the solid support; and performing selective removal of the at least one temporary protecting group of the further saccharide under microwave irradiation resolves the above objective.

Thus, the present invention relates to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation.

According to an aspect of the present invention, the method for synthesizing oligo- and polysaccharides comprises one or more microwave-assisted reaction steps to enable rapid microwave-assisted blocking/deblocking synthesis steps, such as deprotection or capping steps that allow for efficient syntheses in different batch sizes. Use of microwave radiation during part of the coupling cycles can significantly shorten the time to achieve complete conversion, which is important for scale-up.

According to a further aspect of the present invention, the method for synthesizing oligo- or polysaccharides further comprises one or more method steps performed at different temperatures, for example, to allow the addition of reagents at temperatures below 0°C to a reaction mixture at a temperature below 0°C to prevent temperature fluctuations in the reaction mixture, which otherwise may result in loss of sensitive intermediates and reagents. Thus, with the method for synthesizing oligo- and polysaccharides of the present invention unfavorable temperature spikes may be effectively prevented during the transfer of saccharides and glycosylation reagents to the reaction mixture. Furthermore, cooling of the reaction mixture allows for control of the temperature during one or more microwave-assisted synthesis steps to prevent excess thermal energy and overheating of a reaction mixture, which otherwise may result in decomposition of the desired products. Therefore the method for synthesizing oligo- and polysaccharides of the present invention may further comprise one or more steps of adjusting the temperature of a reaction mixture and preferably adjusting the temperature of one or more reagents directly before addition to the reaction mixture. Thus, the method for synthesizing oligo- and polysaccharides of the present invention may further comprise one or more steps of cooling the reaction mixture and preferably cooling of one or more reagents directly before addition to the reaction mixture.

The present invention further relates to an automated method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation.

**Solid supports** for immobilization of carbohydrates or peptides are well-known in the art. Various methods for immobilization of mono- and oligosaccharides to solid supports (e.g. beads, membrane, plates, glass plates, microarrays, etc.) are well known in the art and described in e.g. QSAR Comb. Sci. 25, 2006, No. 11, 1033 - 1038. Thus, any common solid support with acceptor groups or anchor groups suitable for saccharide attachment can be used within the present invention, including commercially available polystyrene beads, amino-PEG cellulose membranes, hydroxyl-modified polypropylene membranes, microarray slides, including but not restricting to Corning® epoxide coated slides or Corning® GAPS™ II coated slides, CodeLink® NHS slides, N-hydroxysuccinimide-activated, epoxy-, amino-, carboxy-, aldehyde-, thiol-, or maleimide-functionalized glass slides, CPG, or aluminium oxide.

The solid support may be provided in form of insoluble resin bead(s) and is preferably a functionalized resin suitable for oligo- and polysaccharide synthesis on a solid support, such as a resin equipped with an appropriate interconnecting molecule or in particular a polystyrene-based resin functionalized with an appropriate interconnecting molecule. Several suitable functionalized solid phase resins for oligo- or polysaccharide synthesis on a solid support have been described in the prior art. Examples of functionalized solid phase resins suitable for use with the device of the present invention are described further below. A solid support or solid phase resin which is stable under microwave conditions or microwave irradiation is particularly preferred with regard to the present invention. Such a microwave-stable solid support or solid phase resin is particularly suitable for performing microwave-assisted reaction steps for synthesizing oligo- and polysaccharides on a solid support. Therefore, it is particularly preferred that no decomposition of the solid phase resin occurs during the microwave-assisted reaction steps of the coupling cycles. In other words a solid support material is preferred which is stable under microwave conditions and also an interconnecting molecule is preferred which is stable under microwave conditions. Thus, a solid phase resin functionalized with an interconnecting molecule which is stable under microwave conditions is particularly preferred, which also means that no decoupling from the solid phase resin occurs during the microwave-assisted reaction steps of the coupling cycles.

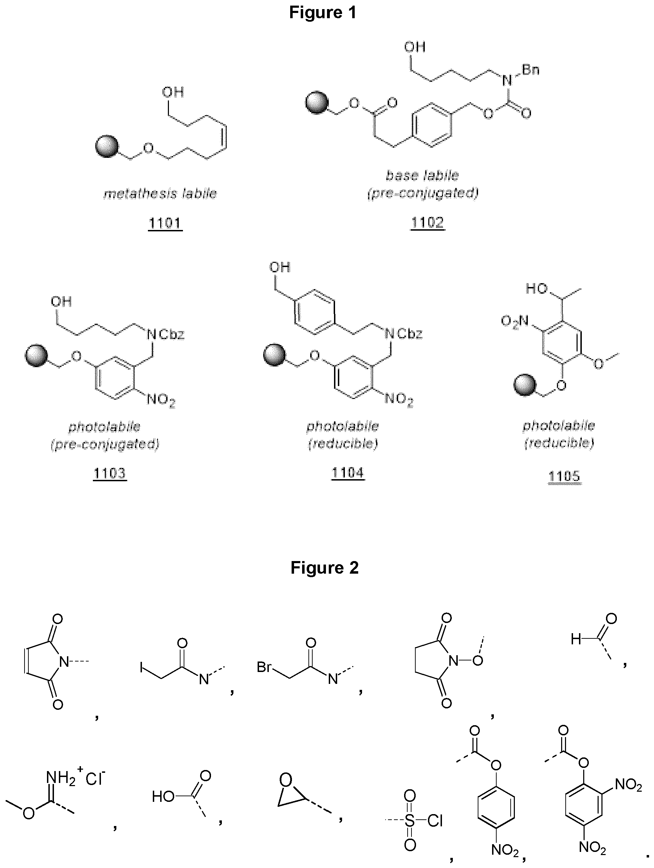

Solid supports present on their surface an **acceptor group** for reacting with a saccharide, i.e. a functionality that is prone to react with a hydroxy group or leaving group of a saccharide employed in the methods described herein, or with a functional group Y of an interconnecting molecule (see Figure 1) to provide modified solid supports, presenting on their surface an acceptor group of the interconnecting molecule that can further react with a hydroxy group or leaving group of a saccharide. The acceptor groups are suitable for immobilizing saccharides on solid supports and are herein synonymously termed as "anchor groups". Suitable acceptor groups on solid supports include but are not restricted to amino, thiol, hydroxy, N-hydroxysuccinimidyl, carboxy, oxime, epoxy or hydrazide groups (see Figure 2).

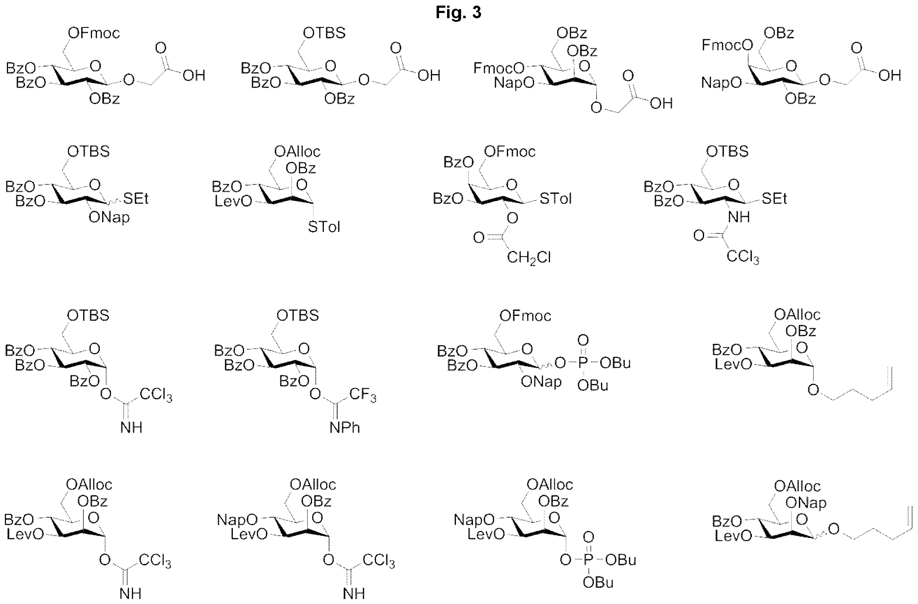

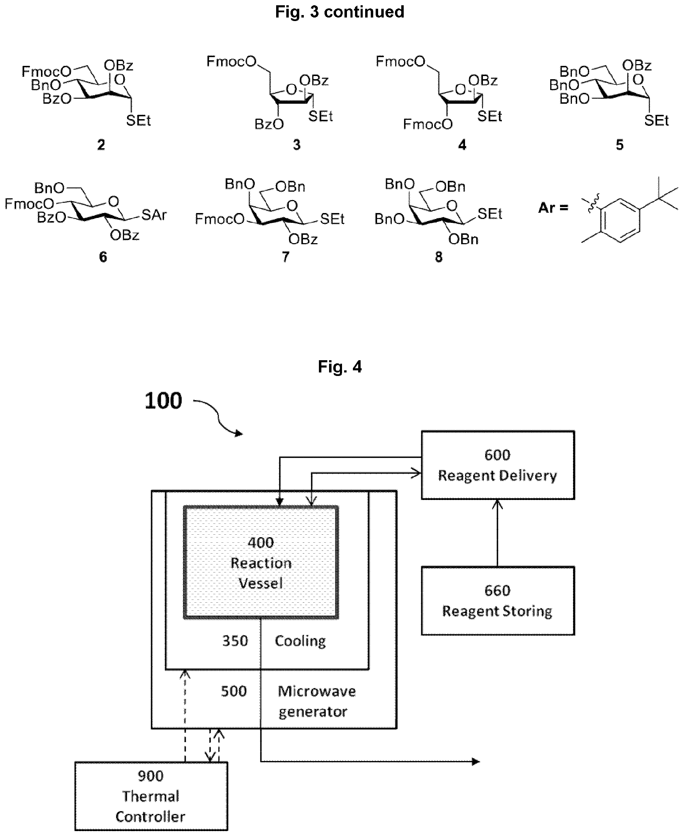

The acceptor group may also be a saccharide, particularly a monosaccharide as listed above, or a disaccharide consisting of monosaccharide units as listed above, bound either directly or via an interconnecting molecule to the surface of the solid support. In this case, the acceptor group is considered to be an immobilized saccharide and the acceptor group may be a glycosyl acceptor comprising such a saccharide. In this case, the glycosyl acceptor forms a part of the saccharide to be synthesized. The glycosyl acceptor contains at least one free hydroxy or amine function and is capable of forming a glycosidic bond with a saccharide under suitable reaction conditions. Suitable acceptor groups are for instance 1-oxyacetates as shown in Figure 3. Thus, it is apparent for a skilled person to choose the glycosyl acceptor depending on the saccharide to be synthesized and in such a manner that the saccharide, after synthesis, can be cleaved from the solid support.

The acceptor group may comprise a glycosyl acceptor of the following formula:

**S-O-L-E**

wherein S represents a monosaccharide selected from 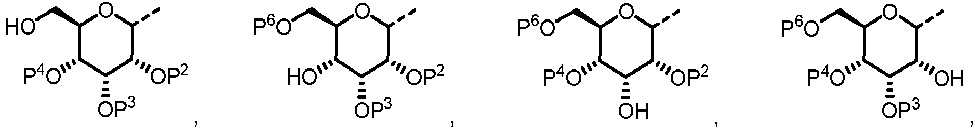 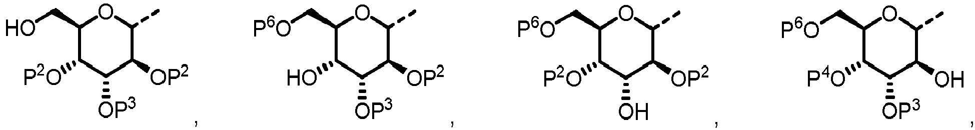 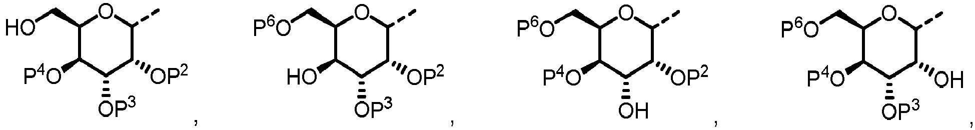 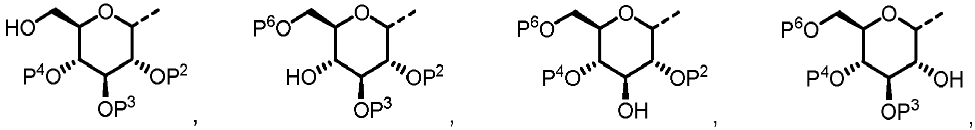 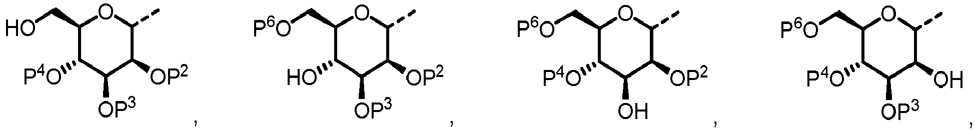 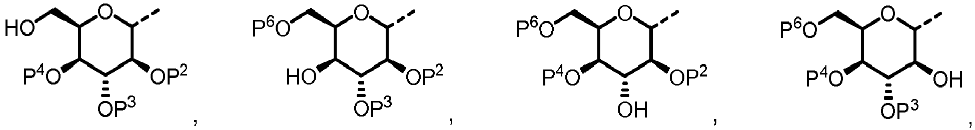 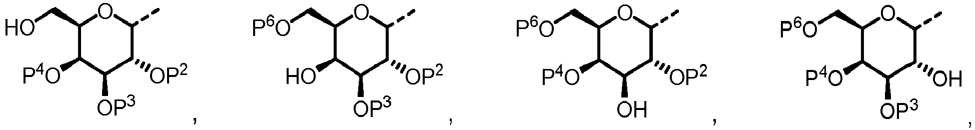 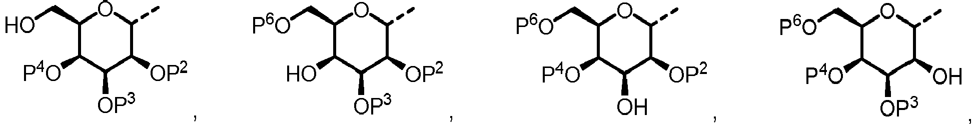 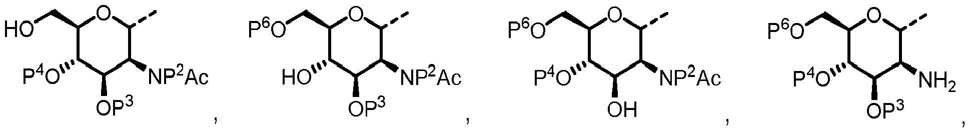 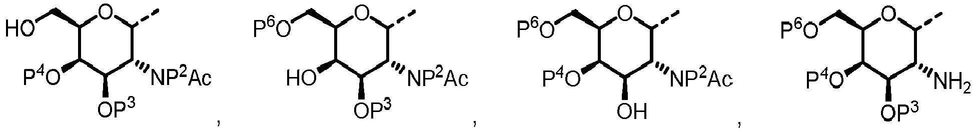
P², P³, P⁴ and P⁶ represent independently of each other protecting groups;
**L** is a linker;
and **E** represents amino, thiol, hydroxy, **N-**hydroxysuccinimidyl**,** carboxylate, carboxylic acid, oxime, epoxy or hydrazide.

Preferably, the linker -L- is selected from: -L^{a}-, -L^{a}-L^{e}-, -L^{a}-L^{b}-L^{e}-, -L^{a}-L^{d}-L^{e}-;
wherein
-L^{a}- is selected from: -(CH₂)ₒ-, -(CH₂-CH₂-O)ₒ-C₂H₄-, -(CH₂-CH₂-O)ₒ-CH₂-;
-L^{b}- represents -O-;
-L^{d}- is selected from -(CH₂)_{q}-, -(CF₂)_{q}-, -(CH₂-CH₂-O)_{q}-C₂H₄-, and -(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-;
and o, q, p1 and p2 are independently of each other an integer selected from 1, 2, 3, 4, 5, and 6.

More preferably, -L- represents -(CH₂)ₒ- and o is an integer selected from 1 2, 3, 4, 5 and 6.

More preferably, -L- represents -(CH₂)ₒ- and o is an integer selected from 1, 2, 3 and 4.

The interconnecting molecule may be chosen in such a manner that it facilitates the identification of synthesized saccharides after cleavage from the solid support via mass spectrometry (MALDI-TOF-MS) by enhancing the molecular weight of the saccharide or via UV/Vis spectroscopy. The interconnecting molecule may also be photocleavable, such as the nitrobenzyl linker shown below or the linkers shown in Figure 1, for obtaining saccharides with a free reducing end after cleavage from the solid support.

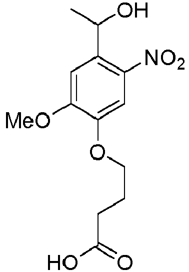

The method of the present invention gives access to saccharides of various lengths, including but not restricting to disaccharide, trisaccharide, tetrasaccharide, pentasaccharide, hexasaccharide, heptasaccharide, octasaccharide, oligosaccharide, glycans, and polysaccharide. Oligosaccharides, glycans, and polysaccharides are particularly preferred with regard to the present invention.

In a preferred embodiment of the present invention, the further saccharide, used in the herein described methods, is a protected glycosyl donor comprising a glycal, epoxide or orthoester group or a protected glycosyl donor having a leaving group at the reducing end. Preferably, the leaving group is selected from: halogen, -O-C(=NH)-CCl₃, -O-C(=NPh)-CF₃, -OAc, -SR⁵, -SO-Ph, -SO₂-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 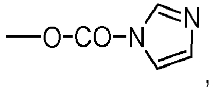 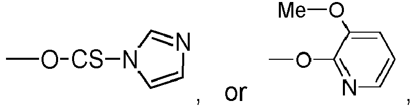 wherein R⁵ represents an alkyl or aryl group.

More preferably, the leaving group is selected from: halogen, -O-C(=NH)-CCl₃, -O-C(=NPh)-CF₃, -O-(CH₂)₃-CH=CH₂, -OAc, and -SR⁵, wherein R⁵ represents an alkyl or aryl group. More preferably, the leaving group is selected from: -O-PO(OR⁵)₂ and -SR⁵, wherein R⁵ represents an alkyl or aryl group.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the further saccharide is a protected glycosyl donor having a leaving group at the reducing end selected from halogen, -O-C(=NH)-CCl₃, -O-C(=NPh)-CF₃, -OAc, -SR⁵, -SO-Ph, -SO₂-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 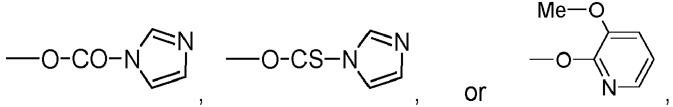 wherein R⁵ represents an alkyl or aryl group.

In a preferred embodiment of the present invention, the glycosylation reagent, used in the herein described methods, is a Lewis acid. Preferably, the glycosylation reagent is selected from: AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST). More preferably, the glycosylation reagent is selected from: trimethylsilyl trifluoromethanesulfonate (TMSOTf), trifluoromethanesulfonic acid (TfOH), trifluoromethanesulfonic anhydride (Tf₂O, triflic anhydride), and NIS/TfOH. Most preferably, the glycosylation reagent is selected from: trimethylsilyl trifluoromethanesulfonate (TMSOTf), trifluoromethanesulfonic acid (TfOH), trifluoromethanesulfonic anhydride (Tf₂O, triflic anhydride), and NIS/TfOH.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the glycosylation reagent is a Lewis acid selected from: AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST).

The coupling reaction in step b) can be carried in out in any solvent suitable for glycosylation reactions known to the person skilled in the art. Preferably, the solvent is an aprotic organic solvent selected from, but not restricted to: methylene chloride, chloroform, acetonitrile, diethyl ether, 1,4-dioxane, methyl tert-butyl ether, tetrahdrofuran, toluene, dimethyl sulfoxide and ethyl acetate.

The further saccharide of step b) of the method of the present invention carries at least one protecting group. Preferably, the at least one protecting group is a temporary protecting group as defined above, which is removed selectively in step c) releasing a free hydroxyl or amino group for the next coupling cycle with a second further saccharide. In a further embodiment the at least one protecting group of the further saccharide is one or more temporary protecting groups as defined above. The temporary protecting groups of a further saccharide carrying or bearing at least two or more temporary groups may be the same temporary protecting groups or different temporary protecting groups which may be cleaved under the same condition, e.g. basis or acidic conditions or may be orthogonal temporary protecting groups which may be cleaved under different conditions, e.g. one under acidic condition and another under basic conditions. Preferably, the temporary protecting group is located at a position of the further saccharide which is intended to be functionalized in a subsequent coupling reaction.

The further saccharide may preferably carry one or more permanent protecting groups as defined above, in addition to the at least one protecting group. Thus, in such an embodiment, the further saccharide may be a further saccharide bearing at least one protecting group and at least one permanent protecting group. The at least one permanent protecting group on the further saccharide is not removed during step c). It is preferred that the at least one protecting group of the further saccharide is a temporary protecting group so that the further saccharide carries at least one temporary protecting group and at least one permanent protecting group. Preferably, the at least one temporary protecting group is located at a position of the saccharide which is intended to be functionalized in a subsequent coupling reaction. Thus, the temporary protecting group preferably masks the acceptor group for the coupling reaction of the next coupling cycle. The remaining functional groups which are not intended to be functionalized in a subsequent coupling reaction step are preferably protected by permanent protecting groups. Thus, in a preferred embodiment, the further saccharide is a fully protected saccharide, i.e. each reactive group (hydroxyl, amino, thio) carries a protecting group. Preferably, one protecting group is a temporary protecting group, whereas the other protecting groups are permanent protecting groups. Also, preferred are fully protected further saccharides having two temporary protecting groups and permanent protecting groups at the remaining reactive groups.

The immobilized saccharide may be an unprotected saccharide or preferably may be a protected saccharide bearing one or more protecting groups, preferably one or more permanent protecting groups and/or one or more temporary protecting groups. It is particularly preferred that the immobilized saccharide of step a) of the method of the present invention has at least one free reactive group (hydroxyl group or amino group or thio group). Particularly preferred is an immobilized saccharide having one reactive group free for glycosylation reaction and the remaining reactive groups blocked by protecting groups. Said protecting groups may be temporary or permanent protecting groups, whereas permanent protecting groups are preferred. In one embodiment, the immobilized saccharide bears one temporary protecting group. In one embodiment, the immobilized saccharide bears one temporary protecting group and at least one permanent protecting group at the reactive groups of the saccharide, wherein one reactive group of the immobilized saccharide remains free for glycosylation reaction with a further saccharide. Thus, it is preferred that the immobilized saccharide is a glycosyl acceptor.

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support; and
c) performing selective removal of the at least one temporary protecting group of the further saccharide under microwave irradiation.

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation,
wherein the further saccharide further carries at least one permanent protecting group.

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation,
wherein the further saccharide further carries at least one temporary protecting group.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing selective removal of the at least one protecting group of the further saccharide under microwave irradiation,
wherein the immobilized saccharide carries at least one permanent protecting group.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing selective removal of the at least one protecting group of the further saccharide under microwave irradiation,
wherein the immobilized saccharide carries at least one temporary protecting group.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing selective removal of the at least one protecting group of the further saccharide under microwave irradiation,
wherein the immobilized saccharide carries at least one permanent protecting group and has at least one free hydroxyl group or free amino group.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing selective removal of the at least one protecting group of the further saccharide under microwave irradiation,
wherein the immobilized saccharide carries at least one temporary protecting group and has at least one free hydroxyl group or free amino group.

Thus, an immobilized saccharide having at least one free hydroxyl group or free amino group is preferred. Preferably the immobilized saccharide is a glycosyl acceptor.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing selective removal of the at least one protecting group of the further saccharide under microwave irradiation,
wherein the immobilized saccharide has one free hydroxyl group.

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation,
wherein the immobilized saccharide carries at least one permanent protecting group and has at least one free hydroxyl group or free amino group, and wherein the further saccharide further carries at least one permanent protecting group.

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation,
wherein the immobilized saccharide has at least one free hydroxyl group or free amino group.

In a preferred embodiment of the present invention, the permanent protecting groups at the immobilized saccharide and the further saccharide may be benzyl, benzoyl, acetyl, allyloxycarbonyl (alloc) and benzyloxycarbonyl group (Cbz).

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation,
wherein the further saccharide and/or immobilized saccharide further carries at least one permanent protecting group, wherein the permanent protecting group is selected from benzyl, benzoyl, acetyl, allyloxycarbonyl (alloc) and benzyloxycarbonyl group (Cbz).

In a preferred embodiment of the present invention, the temporary protecting groups may be allyl, p-methoxybenzyl, 2-naphthylmethyl, tri-isopropylsilyl, *tert-*butyldimethylsilyl, *tert*-butylmethoxyphenylsilyl, triethylsilyl, trimethylsilyl, 2-trimethylsilylethoxymethyl, 9-fluorenylmethoxycarbonyl and levulinoyl.

Thus, the present invention is directed to a method for synthesizing oligo- and polysaccharides, comprising the following steps:
a) providing a solid support with at least one immobilized saccharide;
b) adding a further saccharide bearing at least one temporary protecting group and a glycosylation reagent to the solid support in order to initiate a coupling reaction of the further saccharide to the saccharide immobilized on the solid support;
c) performing selective removal of the at least one temporary protecting group of the further saccharide under microwave irradiation,
wherein the temporary protecting groups are selected from allyl, *p*-methoxybenzyl, 2-naphthylmethyl, tri-isopropylsilyl, *tert*-butyldimethylsilyl, *tert*-butylmethoxyphenyl-silyl, triethylsilyl, trimethylsilyl, 2-trimethylsilylethoxymethyl, 9-fluorenylmethoxycarbonyl and levulinoyl.

According to the invention one or more reaction steps of the method may be performed under microwave irradiation. Preferably at least the selective removal of the at least one temporary protecting group of the further saccharide is performed under microwave irradiation. In preferred embodiments the capping reactions are performed under microwave irradiation.

In a preferred embodiment the solid support obtained in step b) is treated with a capping reagent under microwave irradiation.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
b') treating the solid support obtained in step b) with a capping reagent in order to deactivate or block free hydroxyl groups at the immobilized saccharide; and
c) performing removal of the at least one protecting group of the further saccharide under microwave irradiation.

Microwave-assisted organic chemical synthesis refers to the use of electromagnetic radiation within the microwave frequencies to provide the energy required to initiate, drive or accelerate certain chemical reactions. Several multimode or mono-mode or single-mode microwave generator devices for microwave-assisted organic synthesis are known in the art, for example, in multimode instruments microwaves are reflected by the walls of a relatively large cavity, which may generate pockets of high and low energy as the moving waves either reinforce or cancel out each other leading to a non-homogeneous microwave field in the cavity. To ensure that the field is as homogeneous as possible most multimode instruments are equipped with a mechanical mode stirrer inside the cavity. In the mono-mode cavities a standing wave is created when the electromagnetic irradiation is passed by a waveguide that directs the microwaves directly through a reaction mixture that is positioned in a fixed distance from the radiation source. Multimode microwave devices enable performance of parallel synthesis, whereas mono mode microwave devices provide a high degree of reproducibility due to a very precise heating. The microwave generator device may be one of a magnetron, klystron and solid state devices.

In preferred embodiments of the present invention the reaction in step c) may be performed with a microwave generator device having a cavity, wherein the reaction mixture comprising the saccharide obtained in step b) or b') is placed in the cavity of the microwave generator device. The microwave generator device may be provided as a mono-mode microwave generator device. The microwave generator device may further comprise a waveguide. The microwave generator component preferably comprises a chamber for insertion and fixing of a reaction vessel to provide microwave radiation to the reaction mixture.

In comparison to conductive heating microwave heating (dielectric heating at 2.45 GHz) occurs by disposing the energy directly to the solvent and some reagents, due to interactions of the solvents and reagents with the alternating electric field. Material interacts with the electromagnetic field differently, i.e. materials store and convert the energy to heat to different extents, which have huge impact on their ability to be heated by microwaves. Besides the solvent, several other factors influence the dielectric properties, such as sample volume, vessel material, and the mode of stirring. The application of heat energy (thermal transfer) is one of the most significant factors in increasing the rate of a wide variety of chemical reactions. Microwave-assisted reactions, however, transfer energy to chemical reactions in a different, much faster manner than the conductive devices. Microwave energy directly interacts with polar or ionic molecules. This, effect, known as dipole rotation, is a result of the polar or ionic molecules trying to align themselves with the rapidly changing electric field of the microwaves. The rotational movement of molecules as they try to orient themselves with the electric field creates localized superheating and generates thermal energy as a by-product. Microwave energy transfer is very rapid, producing localized high instantaneous temperatures around pole species while the bulk temperature of the solution or other composition or mixture remains lower.

In one embodiment, the glycosylation reaction of step b) is performed under microwave irradiation. Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support under microwave irradiation; and
c) performing removal of the at least one protecting group of the further saccharide under microwave irradiation.

Preferably, the capping step b') is performed under microwave irradiation.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
b') treating the solid support obtained in step b) with a capping reagent under microwave irradiation in order to deactivate or block free hydroxyl groups at the immobilized saccharide; and
c) performing removal of the at least one protecting group of the further saccharide under microwave irradiation.

In one embodiment the method for synthesizing oligo- and polysaccharides, comprises the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support under microwave irradiation;
b') treating the solid support obtained in step b) with a capping reagent under microwave irradiation in order to deactivate or block free hydroxyl groups at the immobilized saccharide; and
c) performing removal of the at least one protecting group of the further saccharide under microwave irradiation.

In order to avoid excess thermal energy the temperature of the reaction mixture in step b') may be cooled to a temperature range of 0°C to a maximum of 80°C, preferably 25°C to a maximum of 80°C and most preferred to a temperature range of 40°C to a maximum of 80°C. In preferred embodiment the treatment of the solid support with a capping reagent is performed at a reaction temperature of at least 25 °C, more preferred at least 30°C and even more preferred at least 40°C.

In preferred embodiments of the present invention the removal of protecting group(s) in step c) is performed at a reaction temperature of at least 40 °C.

In order to avoid excess thermal energy the temperature of reaction mixture in step c) may be cooled to a temperature range of 0°C to a maximum of 80°C, preferably 25°C to a maximum of 80°C and most preferred to a temperature range of 40°C to a maximum of 80°C. In preferred embodiment the removal of the at least one protecting group is performed at a reaction temperature of at least 25 °C, more preferred at least 30°C and even more preferred at least 40°C.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing selective removal of the at least one protecting group of the further saccharide under microwave irradiation,
wherein the removal of the at least one protecting group is performed at a reaction temperature of at least 40 °C.

Thus, the present invention is directed to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
b') treating the solid support obtained in step b) with a capping reagent under microwave irradiation in order to deactivate or block free hydroxyl groups at the immobilized saccharide; and
c) performing selective removal of the at least one protecting group of the further saccharide under microwave irradiation,
wherein steps b') and c) are performed at a reaction temperature of at least 40 °C.

A solution containing the further saccharide bearing at least one temporary protecting group and a solution of the glycosylation reagent may be added in a continuous flow to the saccharide immobilized on the solid support. A solution of deprotection reagent for selective removal of the at least one temporary group of the further saccharide may be provided in a continuous flow under microwave irradiation.

In further embodiments only specific reaction steps of the coupling cycle may be performed by recirculating the reagents or solutions or reagent solutions in a continuous manner. Preferably the reaction steps under microwave irradiation may be performed in a continuous flow process and the reagents such as the deprotection reagent and/or a capping reagent may be recirculated through the reaction mixture so that a flow may pass the reaction mixture repeated times. Preferably the reaction steps under microwave irradiation may be performed in a continuous flow process and the reagents such as the deprotection reagent and/or a capping reagent may be recirculated through the saccharide immobilized on a solid support so that a flow may pass the saccharide immobilized on a solid support repeated times.

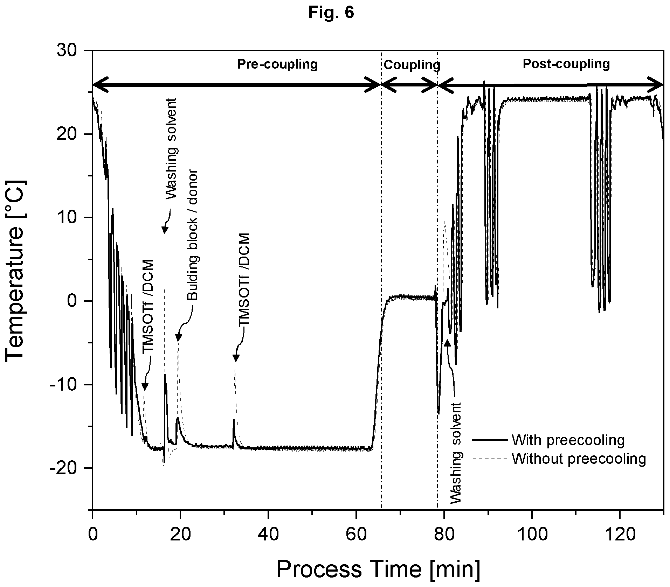

In one embodiment of the inventive methods described herein, the coupling cycle or glycosylation cycle can be divided into three stages. The first stage relates to a solid support with at least one immobilized saccharide and adding a further saccharide bearing at least one protecting group and a glycosylation reagent to the solid support, the second stage relates to the coupling reaction followed by capping and deprotection steps as third stage. Following the deprotection steps and optional capping steps the next coupling cycle may follow to introduce the next building block for building up the desired oligo- or polysaccharide in a step-by-step procedure. Therefore, three stages are present in a coupling cycle, which may be run at different temperatures. Thus, a coupling cycle may be divided into three temperature regimes or stages (see **Figure 6****).**

The first stage relates to the pre-coupling regime in the temperature range T₁ of preferably -40 °C to -10 °C. In this pre-coupling regime a further saccharide bearing at least one protecting group and a glycosylation reagent are allowed to impregnate the solid support (e.g. a resin) and to diffuse through the porous solid. The low temperature in the range of preferably -40°C to -10°C prevents the early decomposition of the intermediate before the actual coupling reaction.

The second stage relates to the coupling regime at a temperature T₂ around 0 °C, i.e. -10 °C and +5 °C. The increase in the temperature to preferably around 0 °C allows the initiation of the coupling reaction by promoting the formation of the intermediates.

The third stage relates to the post-coupling regime at a temperature T₃ around 25°C (room temperature or standard temperature) or above, up to 65 °C. The high reaction temperature T₃ is achieved by microwave irradiation. Thus, the capping and deprotection reactions take place under microwave irradiation and at higher temperatures than the coupling reaction. These reactions complete the coupling cycle. Then, the next coupling cycle or termination of the process may take place. Thus, the present invention relates to a method for synthesizing oligo- and polysaccharides, comprising at least one coupling cycle, wherein each coupling cycle comprises the following steps:
a) providing a solid support with at least one immobilized saccharide;
b1) adding a further saccharide bearing at least one protecting group and a glycosylation reagent to the solid support at temperature T₁,
b2) increasing the reaction temperature to a temperature T₂, with T₂ > T₁ in order to initiate a coupling reaction of the further saccharide to the saccharide immobilized on the solid support; and
c) performing removal of the at least one protecting group of the further saccharide at a reaction temperature of T₃, with T₃ > T₂, wherein the reaction temperature T₃ is achieved by microwave irradiation.

Reworded, the present invention relates to a method for synthesizing oligo- and polysaccharides, comprising at least one coupling cycle, wherein each coupling cycle comprises the following steps:
a) providing a solid support with at least one immobilized saccharide;
b1) adding a further saccharide bearing at least one protecting group and a glycosylation reagent to the solid support at temperature T₁,
b2) increasing the reaction temperature to a temperature T₂, with T₂ > T₁ in order to initiate a coupling reaction of the further saccharide to the saccharide immobilized on the solid support; and
c) performing removal of the at least one protecting group of the further saccharide at a reaction temperature of T₃, with T₃ > T₂ under microwave irradiation;
wherein the reaction temperature T₃ in step c) is reached by adjusting the power of the microwave radiation.

In one embodiment, the method for synthesizing oligo- and polysaccharides comprises at least one coupling cycle, wherein each coupling cycle comprises the following steps:
a) providing a solid support with at least one immobilized saccharide;
b1) adding a further saccharide bearing at least one protecting group and a glycosylation reagent to the solid support at temperature T₁,
b2) increasing the reaction temperature to a temperature T₂, with T₂ > T₁ in order to initiate a coupling reaction of the further saccharide to the saccharide immobilized on the solid support; and
c) performing removal of the at least one protecting group of the further saccharide at a reaction temperature of T₃, with T₃ > T₂, wherein the reaction temperature T₃ is achieved by microwave irradiation,
wherein T₁ is between -40 °C to -10 °C,
T₂ is between -10 °C and +5 °C, and
T₃ is between +20 °C and +70 °C.

In one embodiment, the method for synthesizing oligo- and polysaccharides comprises at least one coupling cycle, wherein each coupling cycle comprises the following steps:
a) providing a solid support with at least one immobilized saccharide;
b1) adding a further saccharide bearing at least one protecting group and a glycosylation reagent to the solid support at temperature T₁,
b2) increasing the reaction temperature to a temperature T₂, with T₂ > T₁ in order to initiate a coupling reaction of the further saccharide to the saccharide immobilized on the solid support; and
c) performing removal of the at least one protecting group of the further saccharide at a reaction temperature of T₃, with T₃ > T₂ under microwave irradiation;
wherein the reaction temperature T₃ in step c) is reached by adjusting the power of the microwave radiation;
wherein T₁ is between -40 °C to -10 °C,
T₂ is between -10 °C and +5 °C, and
T₃ is between +20 °C and +65 °C.

In a preferred embodiment, the method for synthesizing oligo- and polysaccharides comprises at least one coupling cycle, wherein each coupling cycle comprises the following steps:
a) providing a solid support with at least one immobilized saccharide;
b1) adding a further saccharide bearing at least one protecting group and a glycosylation reagent to the solid support at temperature T₁,
b2) increasing the reaction temperature to a temperature T₂, with T₂ > T₁ in order to initiate a coupling reaction of the further saccharide to the saccharide immobilized on the solid support; and
c) performing removal of the at least one protecting group of the further saccharide at a reaction temperature of T₃, with T₃ > T₂ under microwave irradiation;
wherein the reaction temperature T₂ in step b2) and/or the reaction temperature T₃ in step c) are reached by adjusting the power of the microwave radiation;
wherein T₁ is between -40 °C to -10 °C,
T₂ is between -10 °C and +5 °C, and
T₃ is between +20 °C and +65 °C.

In one embodiment of the present invention, the glycosylation reagent and the further saccharide have the same temperature as the solid support before their addition in step b). Thus, preferably all reagents and solutions added to perform the coupling reaction in step b) are pre-cooled close to the temperature of the reaction mixture. Furthermore, the resulting intermediates are often temperature sensitive and may decompose at higher temperatures than the desired temperature of the reaction mixture. It should be clear that already formed temperature sensitive intermediates in the reaction mixture may decompose in case further warmer reagents or solvents are added to the reaction mixture, such as reagents or solvents at room temperature. In such a case it may be suitable to supply these reagents or solvents in a slow and careful manner, for example dropwise manner, by monitoring the temperature of the reaction mixture at the same time. However, in larger batch sizes a larger amount of the reagents to be supplied are added to the reaction mixture. The delayed addition, for example dropwise addition, and the period of time until the reaction mixture is adjusted to the desired and preset temperature may also result in decomposition of the reagents and in particular the sensitive intermediates. The inventors of the present invention have found that on the hand the rapid addition of the reagents to the reaction mixture in the pre-coupling stage and impregnating of the resin to allow these reagents to diffuse through the porous solid for a period of time of 1 minute to 20 minutes, preferably 5 minutes has an advantageous effect on the overall yield of the desired oligo- and polysaccharide. According to a preferred embodiment of the present invention pre-cooled reagents may be added in a faster and rapid manner to the reaction mixture without unfavorable temperature fluctuations in the reaction mixture in the pre-coupling stage at preferably -40°C to -10°C and the added pre-cooled reagents may be allowed to impregnate the resin to diffuse through the porous solid for a period of time of 1 minute to 20 minutes, preferably 5 minutes such that the overall time of the pre-coupling stage including the adding and impregnating of the reagents may be reduced such that decompositions of the reagents and the resulting intermediates may be effectively prevented in comparison to a slow and careful addition of the reagents to be supplied which may result in an overall time of the pre-coupling stage of over 25 minutes or 30 minutes or more.

The rapid addition of the pre-cooled reagents in the pre-coupling stage has been shown to be particularly advantageous with regard to the glycosylation reactions in the method for synthesizing oligo- and polysaccharides of the present invention.

The temperature of the reagents to be supplied preferably corresponds to the temperature of the reaction mixture. For example, if the reaction mixture is actively cooled to a temperature of -20° in the pre-coupling stage of the synthesis cycle the reagents to be supplied may preferably be pre-cooled also to a temperature of -20°C. In such a case the pre-cooled reagents at a temperature of -20°C are added to the reaction mixtures with a temperature of -20°C, wherein temperature fluctuations in the reaction mixture are prevented during addition of the reagents. However, the reagents to be supplied to the reaction vessel may be also added at a slightly higher temperature then the temperature of the reaction mixture in the reaction vessel. Preferably the reagents are pre-cooled to a temperature in the temperature range corresponding to the temperature range of -40°C to -10°C of the pre-coupling stage. In preferred embodiments of the present invention the reagents to be supplied are adjusted at least to a temperature which is not higher than 3°C of the temperature of the reaction. The reagents to be supplied are cooled to a temperature not higher than 3°C of the temperature of the reaction mixture. The reagents to be supplied may be cooled to a range temperature in the reaction mixture from 3°C to -3°C. Thus, it is preferred that the reagents to be supplied are pre-cooled to the corresponding temperature of the reaction mixture with a maximum variance of 3°C or maximum deviation of 3°C. It is preferred that the temperature of the reaction mixture does not exceed a temperature of -10°C in the pre-coupling stage of the coupling cycle.

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the temperature of the reaction mixture in step b) does not exceed a temperature of -10 °C

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the solid support with at least one immobilized saccharide are cooled to a temperature below 0 °C.

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the further saccharide bearing at least one protecting group and the glycosylation reagent are pre-cooled directly before addition to the solid support.

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the solid support with at least one immobilized saccharide is cooled to a temperature below 0 °C and the further saccharide bearing at least one protecting group and the glycosylation reagent are cooled to a temperature below 0°C directly before addition to the solid support in step b).

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the solid support with at least one immobilized saccharide is cooled to a temperature below 0 °C and the further saccharide bearing at least one protecting group and the glycosylation reagent are added at a temperature below 0°C in step b).

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein the solid support with at least one immobilized saccharide is cooled to a temperature below 0°C and wherein the temperature of the further saccharide bearing at least one protecting group and the glycosylation reagent is below 0°C in step b).

The present invention further relates to a method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation,
wherein in step b) the further saccharide and the glycosylation reagent are cooled before addition to the solid support.

In step b) of the inventive methods described herein, the further saccharide and the glycosylation reagent can be added in any order to the solid support, i.e. the further saccharide first, the glycosylation reagent first or both simultaneously. Preferably the further saccharide is added first to the solid support in step b).

The present invention further relates to a method for synthesizing oligo- and polysaccharides, comprising the following steps:
a) providing a solid support with at least one immobilized saccharide;
b) adding a further saccharide bearing at least one protecting group to the solid support followed by adding a glycosylation reagent to the solid support and the further saccharide in order to initiate a coupling reaction of the further saccharide to the saccharide immobilized on the solid support;
c) performing removal of the at least one protecting group of the further saccharide under microwave irradiation.

The present invention further relates to a method for synthesizing oligo- and polysaccharides, comprising the following steps:
a) providing a solid support with at least one immobilized saccharide;
b) adding a further saccharide bearing at least one protecting group to the solid support followed by adding a glycosylation reagent to the solid support and the further saccharide in order to initiate a coupling reaction of the further saccharide to the saccharide immobilized on the solid support;
c) performing removal of the at least one protecting group of the further saccharide under microwave irradiation,
wherein the solid support with at least one immobilized saccharide is cooled to a temperature below 0°C and wherein the temperature of the glycosylation reagent is below 0°C in step b).

The present invention further relates to a method for synthesizing oligo- and polysaccharides, comprising the following steps:
a) providing a solid support with at least one immobilized saccharide;
b) adding a further saccharide bearing at least one protecting group to the solid support followed by adding a glycosylation reagent to the solid support and the further saccharide in order to initiate a coupling reaction of the further saccharide to the saccharide immobilized on the solid support;
c) performing removal of the at least one protecting group of the further saccharide under microwave irradiation,
wherein in step b) the glycosylation reagent is cooled before addition to the further saccharide and the solid support.

Pre-cooling of the reagents prevents and avoids decomposition of the reagents during the glycosylation reaction which otherwise could result in undesired side-products and decomposition products. By adding pre-cooled reagents in step b), conversion and yield could be increased, decomposition of reagents could be decreased and formation of side and decomposition products could effectively be prevented. Additionally, by pre-cooling of the reagents to be supplied the overall period of time of a coupling cycle and consequently the overall period of time for the overall synthesis of the desired product could effectively be reduced.

After the glycan construction on solid phase is completed, i.e. glycosylation cycles are finished, the saccharide obtained in step c) is cleaved from the solid support and the permanent protecting group(s) of the cleaved saccharide, if present, is removed. Afterwards the deprotected saccharide may be purified using standard purification techniques, e.g. by column chromatography.

Alternatively, the cleaved saccharide may be purified first and the protecting group(s) of the cleaved saccharide may be removed after the purification step. After removal of the protecting(s) group a further purification step may follow.

Thus, the method of the present invention may further comprise the steps:
d) cleaving the saccharide obtained in step c) from the solid support;
e) optionally performing removal of the protecting group(s) of the saccharide obtained in step d); and
f) purifying the saccharide obtained in step e).

Thus, the present invention is directed to a method for synthesizing oligo- and polysaccharides, comprising the following steps:
a) providing a solid support with at least one immobilized saccharide having at least one permanent protecting group;
b) adding a further saccharide bearing at least one temporary protecting group and at least one least one permanent protecting group, and a glycosylation reagent to the solid support in order to initiate a coupling reaction of the further saccharide to the saccharide immobilized on the solid support;
c) performing selective removal of the at least one temporary protecting group of the further saccharide under microwave irradiation,
d) cleaving the saccharide obtained in step c) from the solid support;
e) performing complete removal of the permanent protecting groups of the saccharide obtained in step d); and
f) purifying the saccharide obtained in step e).

Thus, the present invention is directed to a method for synthesizing oligo- and polysaccharides, comprising the following steps:
a) providing a solid support with at least one immobilized saccharide having at least one permanent protecting group;
b) adding a further saccharide bearing at least one temporary protecting group and at least one least one permanent protecting group, and a glycosylation reagent to the solid support in order to initiate a coupling reaction of the further saccharide to the saccharide immobilized on the solid support;
c) performing selective removal of the at least one temporary protecting group of the further saccharide under microwave irradiation,
d) cleaving the saccharide obtained in step c) from the solid support;
e') purifying the saccharide obtained in step d); and
f') performing complete removal of the permanent protecting groups of the saccharide obtained in step e').

Thus, the present invention is directed to a method for synthesizing oligo- and polysaccharides, comprising the following steps:
a) providing a solid support with at least one immobilized saccharide having at least one permanent protecting group;
b) adding a further saccharide bearing at least one temporary protecting group and at least one least one permanent protecting group, and a glycosylation reagent to the solid support in order to initiate a coupling reaction of the further saccharide to the saccharide immobilized on the solid support;
c) performing selective removal of the at least one temporary protecting group of the further saccharide under microwave irradiation,
d) cleaving the saccharide obtained in step c) from the solid support;
e') purifying the saccharide obtained in step d);
f') performing complete removal of the permanent protecting groups of the saccharide obtained in step e'), and
g') purifying the saccharide obtained in step f').

Preferably, step b') is carried out between step b) and step c).
b') treating the solid support obtained in step b) with a capping reagent under microwave irradiation in order to deactivate or block free hydroxyl groups at the immobilized saccharide

In one embodiment, the glycosylation reaction of step b) is performed under microwave irradiation. In one embodiment, the glycosylation reaction of step b) is performed without microwave irradiation.

The present invention further relates to a method for synthesizing oligo- and polysaccharides on a solid support, comprising the following steps:
A1) providing a solid support with at least one immobilized saccharide;
A2) washing the solid support to swell the solid support;
B1) adding a further saccharide bearing at least one temporary protecting group and at least one permanent protecting group to the solid support obtained in step A2);
B2) adding a glycosylation reagent to the further saccharide and the solid support;
C) performing a coupling reaction of the further saccharide to the saccharide immobilized on the solid support;
D) optionally treating the solid support obtained in step C) with a capping reagent in order to deactivate or block free hydroxyl groups at the immobilized saccharide;
E) performing selective removal of the at least one temporary protecting group of the further saccharide under microwave irradiation;
F) optionally repeating steps B1), B2), C), D) and E);
G) cleaving the saccharide obtained in step E) from the solid support;
H) performing complete removal of the at least one permanent protecting group of the saccharide obtained in step G); and
I) purifying the saccharide obtained in step H).

In one embodiment, the method for synthesizing oligo- and polysaccharides on a solid support comprises the following steps:
A1) providing a solid support with at least one immobilized saccharide;
A2) washing the solid support to swell the solid support;
B1) adding a further saccharide bearing at least one temporary protecting group to the solid support obtained in step A2);
B2) adding a glycosylation reagent to the further saccharide and the solid support;
C) performing a coupling reaction of the further saccharide to the saccharide immobilized on the solid support;
D) optionally treating the solid support obtained in step C) with a capping reagent in order to deactivate or block free hydroxyl groups at the immobilized saccharide;
E) performing selective removal of the at least one temporary protecting group of the further saccharide under microwave irradiation;
F) optionally repeating steps B1), B2), C), D) and E);
G) cleaving the saccharide obtained in step E) from the solid support; and
I) purifying the saccharide obtained in step G).

Preferably, steps B1) and B2) are carried out in the above listed order. However, steps B1) and B2) may also be carried out in reverse order, i.e. step B2) before step B1). Also both steps may also be performed simultaneously.

Preferably, steps H) and I) are carried out in the above listed order. However, steps H) and I) may also be carried out in reverse order, i.e. step I) before step H).

In preferred embodiment, method for synthesizing oligo- and polysaccharides on a solid support comprises the following steps
A1) providing a solid support with at least one immobilized saccharide having at least one permanent protecting group;
A2) washing the solid support to swell the solid support;
B1) adding a further saccharide bearing at least one temporary protecting group and at least one permanent protecting group to the solid support obtained in step A2);
B2) adding a glycosylation reagent to the further saccharide and the solid support;
C) performing a coupling reaction of the further saccharide to the saccharide immobilized on the solid support;
D) optionally treating the solid support obtained in step C) with a capping reagent in order to deactivate or block free hydroxyl groups at the immobilized saccharide;
E) performing selective removal of the at least one temporary protecting group of the further saccharide under microwave irradiation;
F) optionally repeating steps B1), B2), C), D) and E);
G) cleaving the saccharide obtained in step E) from the solid support;
H) performing complete removal of the at least one permanent protecting groups of the further saccharide and the immobilized saccharide obtained in step G); and
I) purifying the saccharide obtained in step H).

In preferred embodiment, the method for synthesizing oligo- and polysaccharides on a solid support comprises the following steps
A1) providing a solid support with at least one immobilized saccharide having at least one permanent protecting group;
A2) washing the solid support to swell the solid support;
B1) adding a further saccharide bearing at least one temporary protecting group to the solid support obtained in step A2);
B2) adding a glycosylation reagent to the further saccharide and the solid support;
C) performing a coupling reaction of the further saccharide to the saccharide immobilized on the solid support;
D) optionally treating the solid support obtained in step C) with a capping reagent in order to deactivate or block free hydroxyl groups at the immobilized saccharide;
E) performing selective removal of the at least one temporary protecting group of the further saccharide under microwave irradiation;
F) optionally repeating steps B1), B2), C), D) and E);
G) cleaving the saccharide obtained in step E) from the solid support;
H) performing complete removal of the at least one permanent protecting group of the immobilized saccharide obtained in step G); and
I) purifying the saccharide obtained in step H).

In preferred embodiment, the method for synthesizing oligo- and polysaccharides on a solid support comprises the following steps
A1) providing a solid support with at least one immobilized saccharide;
A2) washing the solid support to swell the solid support;
B1) adding a further saccharide bearing at least one temporary protecting group to the solid support obtained in step A2);
B2) adding a glycosylation reagent to the further saccharide and the solid support;
C) performing a coupling reaction of the further saccharide to the saccharide immobilized on the solid support;
D) optionally treating the solid support obtained in step C) with a capping reagent in order to deactivate or block free hydroxyl groups at the immobilized saccharide;
E) performing selective removal of the at least one temporary protecting group of the further saccharide under microwave irradiation;
F) optionally repeating steps B1), B2), C), D) and E);
G) cleaving the saccharide obtained in step E) from the solid support; and
I) purifying the saccharide obtained in step G).

Preferably, step D) is performed under microwave irradiation. In one embodiment. steps C), D) and E) are performed under microwave irradiation. In one embodiment, step C) is performed without microwave irradiation.

Preferably, steps H) and I) are carried out in the above listed order. However, steps H) and I) may also be carried out in reverse order, i.e. step I) before step H).

Preferably, the saccharide immobilized on the solid support is a glycosyl acceptor comprising 1 to 20 monosaccharide units as defined above.

Preferably, the at least one permanent protecting group of the immobilized saccharide is selected from acetyl, phenyl, benzyl, isopropylidene, benzylidene, benzoyl, *p*-methoxybenzyl, *p*-methoxybenzylidene, *p*-methoxyphenyl, *p*-bromobenzylidene, *p*-nitrophenyl, allyl, allyloxycarbonyl, monochloroacetyl, isopropyl, *p*-bromobenzyl, dimethoxytrityl, trityl, 2-naphthylmethyl, pivaloyl, triisopropylsilyl, *tert*-butyldimethylsilyl, *tert*-butyldiphenylsilyl, *tert*-butylmethoxyphenylsilyl, triethylsilyl, trimethylsilyl, 2-trimethylsilylethoxymethyl, 9-fluorenylmethoxycarbonyl, tert-butyloxycarbonyl, benzyloxymethyl, methyloxymethyl, *tert*-butyloxymethyl, methoxyethyloxymethyl, and levulinoyl.

Preferably, the glycosylation reagent is a Lewis acid selected from: AgOTf, BF₃•OEt₂, trimethylsilyl trifluoromethanesulfonate, trifluoromethanesulfonic acid, trifluoromethanesulfonic anhydride, lanthanoid(III) triflates, NIS/AgOTf, NIS/TfOH or dimethyl(methylthio)sulfonium trifluoromethanesulfonate.

Preferably, the further saccharide is a glycosyl donor comprising a glycal, epoxide or orthoester group or having a leaving group at the reducing end selected from halogen, -O-C(=NH)-CCl₃, -O-C(=NPh)-CF₃, -OAc, -SR⁵, -SO-Ph, -SO₂-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 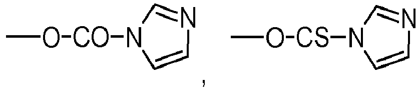 or 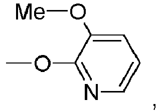 wherein R⁵ represents an alkyl or aryl group. Preferably, the further saccharide is a glycosyl donor having a leaving group at the reducing end selected from -SR⁵ and -O-PO(OR⁵)₂, wherein R⁵ represents an alkyl or aryl group.

Preferably, the temporary protecting group of the further saccharide is selected from allyl, p-methoxybenzyl, 2-naphthylmethyl, tri-isopropylsilyl, *tert*-butyldimethylsilyl, tert-butylmethoxyphenylsilyl, triethylsilyl, trimethylsilyl, 2-trimethylsilylethoxymethyl, 9-fluorenylmethoxycarbonyl and levulinoyl.

Preferably, the at least one permanent protecting group of the further saccharide is selected from acetyl, phenyl, benzyl, isopropylidene, benzylidene, benzoyl, p-methoxybenzyl, *p*-methoxybenzylidene, *p*-methoxyphenyl, *p*-bromobenzylidene, *p*-nitrophenyl, allyl, allyloxycarbonyl, monochloroacetyl, isopropyl, *p*-bromobenzyl, dimethoxytrityl, trityl, 2-naphthylmethyl, pivaloyl, triisopropylsilyl, *tert*-butyldimethylsilyl, *tert*-butyldiphenylsilyl, *tert*-butyl-methoxyphenylsilyl, triethylsilyl, trimethylsilyl, 2-trimethylsilylethoxymethyl, 9-fluorenylmethoxycarbonyl, tert-butyloxycarbonyl, benzyloxymethyl, methyloxymethyl, *tert*-butyloxymethyl, methoxyethyloxymethyl, and levulinoyl.

Preferably, the coupling reaction in step C) is performed at a reaction temperature of less than -10 °C. More preferably, the coupling reaction in step C) is performed at a reaction temperature below 0 °C. Preferably, the further saccharide is cooled to the reaction temperature of the coupling reaction in step C) before addition to the solid support. Preferably, the glycosylation reagent is cooled to the reaction temperature of the coupling reaction in step C) before addition to the solid support and the further saccharide. More preferably, the further saccharide and the glycosylation reagent are cooled to the reaction temperature of the coupling reaction in step C) before addition to the solid support.

Preferably, step D) is performed under microwave irradiation. More preferably, step D) is performed under microwave irradiation and at a temperature of at least 40 °C.

Preferably, step E) is performed at a temperature of at least 40 °C.

A further aspect of the present invention is directed to a method for synthesizing oligo- and polysaccharides on a solid support comprising the following steps
A1) providing a solid support with at least one immobilized saccharide having at least one permanent protecting group;
A2) washing the solid support to swell the solid support;
B1) adding a further saccharide bearing at least one temporary protecting group and at least one permanent protecting group to the solid support obtained in step A2);
B2) adding a glycosylation reagent to the further saccharide and the solid support;
C) performing a coupling reaction of the further saccharide to the saccharide immobilized on the solid support;
D) optionally treating the solid support obtained in step C) with a capping reagent in order to deactivate or block free hydroxyl groups at the immobilized saccharide;
E) performing selective removal of the at least one temporary protecting group of the further saccharide under microwave irradiation;
F) optionally repeating steps B1), B2), C), D) and E);
G) cleaving the saccharide obtained in step E) from the solid support;
H) performing complete removal of the at least one permanent protecting groups of the further saccharide and the immobilized saccharide obtained in step G); and
I) purifying the saccharide obtained in step H).

Preferably, steps H) and I) are carried out in the above listed order. However, steps H) and I) may also be carried out in reverse order, i.e. step I) before step H).

### Synthesizer with thermal controller

Preferably, the method of the present invention may be performed in an automated synthesizer (100) comprising a microwave transparent reaction vessel (400) equipped with a temperature sensor (450), a microwave generator component (500), a reagent storing component (660), a reagent delivery system (600), a cooling device (350) for cooling the microwave transparent reaction vessel and a thermal controller (900) for controlling the temperature inside the microwave transparent reaction vessel, (see **Figure 4****).**

Thus, a further aspect of the present invention is directed to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

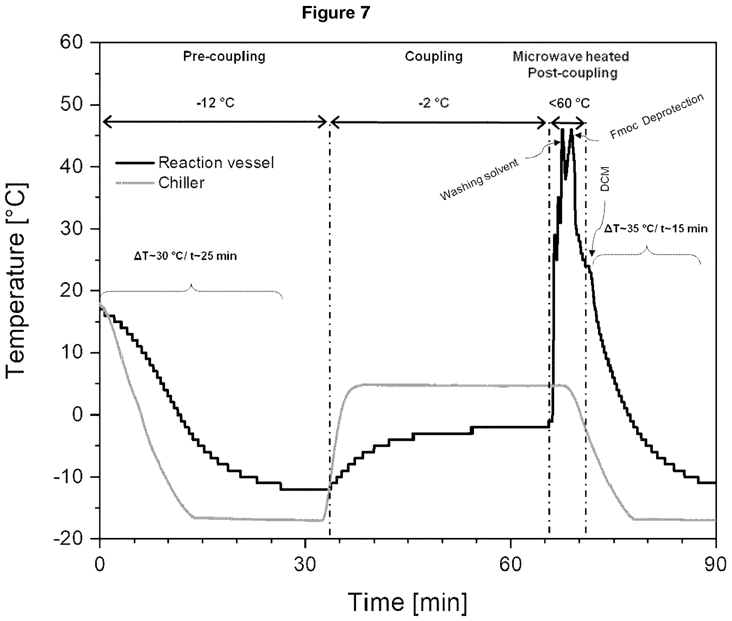

The synthesizer according to the present invention is particularly suitable for automated multi-step synthesis on solid support, wherein each step requires a different temperature regime (see Figure 7). The inventive synthesizer is characterized by a cooling device and a microwave generator component which are regulated or controlled by a thermal controller, thereby allowing rapid adjustment of the reaction temperature and therefore shorter reaction times.

A typical coupling cycle in a glycan synthesis comprises a glycosylation step and several protecting group manipulations ("auxiliary steps") including a capping reaction in order to terminate unreacted saccharides, deprotection of a temporary protecting group and washing of the solid support. These steps of each coupling cycle are conducted in three different three temperature regimes or stages (as described above).

The first stage relates to the pre-coupling regime in the temperature range T₁ of preferably -40 °C to -10 °C. In this pre-coupling regime a further saccharide bearing at least one protecting group and a glycosylation reagent are allowed to impregnate the solid support (e.g. a resin) and to diffuse through the porous solid. The low temperature in the range of preferably -40°C to -10°C prevents the early decomposition of the intermediate before the actual coupling reaction.

The second stage relates to the coupling regime at a temperature T₂ around 0 °C, i.e. -10 °C and +5 °C. The increase in the temperature to preferably around 0 °C allows the initiation of the coupling reaction by promoting the formation of the intermediates. Thus, during the coupling regime, the glycosylation reaction takes place.

The third stage relates to the post-coupling regime at a temperature T₃ around 25 °C (room temperature or standard temperature) or above, up to 70 °C. The high reaction temperature T₃ is achieved by microwave irradiation Thus, the capping and deprotection reactions take place under microwave irradiation and at higher temperatures than the coupling reaction. These reactions complete the coupling cycle. Then, the next coupling cycle or termination of the process may take place.

As can be seen in Figure 7, the temperature must be adjusted between each coupling cycle from high temperature T₃ to the lowest temperature T₁, which requires usually more than 20 minutes (see left part of Figure 7). By using a thermal controller, which is connected to the cooling device, the temperature sensor and the microwave generator component, the reaction temperature can be adjusted more rapidly, so that less time is required between each coupling cycle (see right part of Figure 7), thereby allowing a more rapid automated multistep synthesis.

Particularly, the thermal controller is connected to the temperature sensor in the microwave transparent reaction vessel and the microwave generator component, thereby allowing the reaction mixture to reach the reaction temperature of T₃ in the post-coupling regime. The thermal controller adjusts the power output of the microwave generator component based on the measured temperature inside the reaction vessel in order to maintain the temperature T₃. Also, the thermal controller is connected to the temperature sensor in the microwave transparent reaction vessel and the microwave generator component thereby allowing the reaction mixture to warm up more rapidly from a reaction temperature of T₁ to T₂ during the coupling regime.

The thermal controller is also connected to the cooling device thereby allowing setting a fixed cooling temperature T₁ during pre-coupling regime and/or T₂ during the coupling and post-coupling regime. The microwave generator component is usually turned off by the thermal controller during pre-coupling and coupling regime and only turned on during the post-coupling regime. However, in one embodiment the microwave generator component is already turned on by the thermal controller during coupling regime in order to speed up the warming from T₁ to T₂.

Thus, the thermal controller connected to a cooling device and a microwave generator component allows the use of the microwave generator component as a heater, so that no additional heater is required for performing the auxiliary steps at elevated temperatures. Therefore, due to the thermal controller a simpler and more compact setup of the synthesizer is achieved as no additional cooler or heater are necessary for performing the multi-step reactions at different temperature regimes as set forth above.

According to the present invention the synthesizer comprises a **microwave transparent reaction vessel.** All reactions steps of the multi-step synthesis, including coupling reactions, appropriate deprotection, and optionally capping steps are preferably performed inside of the microwave transparent reaction vessel of the inventive synthesizer. The coupling cycles are repeated as often as necessary to achieve the desired length of the desired oligo-, polysaccharide or peptide.

The microwave transparent reaction vessel may be made of any material known in the art which is chemically and physically compatible with the reagents used and reaction conditions applied in the automated multi-step synthesis of oligo-, polysaccharides and peptides on a solid support. Examples of microwave transparent reaction vessel materials include, but are not limited to, glass, quartz, PTFE (Teflon), polypropylene. In preferred embodiments the reaction vessel is interchangeable. In preferred embodiments the reaction vessel is made of fluoropolymers such as PFA for improved chemical resistance and ease of swapping reactions vessels of different sizes. In preferred embodiments the reaction vessel is made of glass.

The microwave transparent reaction vessel of the synthesizer according to the present invention is equipped with a temperature sensor. The temperature sensor can be any type of temperature sensor commonly known in the art that is suitable for probing the temperature range from T₁ to T₃ and that is stable in presence of the reaction mixture, building blocks and reagents used in the multi-step synthesis. Suitable temperature sensors are for example, but not limited to, negative temperature coefficient (NTC) thermistor, resistance temperature detector, thermocouple or fiber optic temperature probe. Preferably, the temperature sensor is a fiber optic temperature probe.

The reaction vessel of the present invention is adapted for holding a reaction mixture. The reaction vessel therefore comprises an inner cavity or effective loading space for performing one or more synthesis steps of the synthesis cycles. The reaction vessel is adapted to enable performance of one or more synthesis steps or even all of the synthesis steps of the synthesis cycles in an isolated, anhydrous and inert atmosphere. These steps may include, but are not limited to, coupling steps, washing steps, deprotection steps, capping steps and decoupling from the solid phase resin. The solid support or solid phase resin may be initially loaded into the reaction vessel. With other words the solid support or solid phase resin may be loaded into the inner cavity or effective loading space of the reaction vessel. The loading of the reaction vessel with the solid phase resin may be performed as part of a pre-automation process but may also be part of the automation process. Thus, the loading with the solid support may be also performed in an automated manner. The solid support may be provided in form of insoluble resin bead(s) and is preferably a functionalized resin suitable for oligo- and polysaccharide synthesis on a solid support, such as a resin equipped with an appropriate linker or in particular a polystyrene-based resin functionalized with an appropriate linker. Several suitable functionalized solid phase resins for oligo- or polysaccharide synthesis on a solid support have been described in the prior art. Examples of functionalized solid phase resins suitable for use with the device of the present invention are described further below.

Thus, it is preferred that the microwave transparent reaction vessel is adapted for receiving a solid support. Therefore, the reaction vessel preferably comprises an inner chamber or effective loading space for performing one or more synthesis steps of the synthesis of oligo- and polysaccharides on a solid support, preferably in an isolated, anhydrous and inert atmosphere. It is preferred that the inner cavity or effective loading space holds between 1 mL and 100 mL solvent, more preferably 5 - 20 mL solvent. It is preferred that the inner cavity or the effective loading space is sized to accommodate the solid support, reagents and solvent.

Therefore the present invention further relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
wherein the microwave transparent reaction vessel is adapted for receiving the solid support.

The reaction vessel therefore comprises an inner cavity or effective loading space for performing one or more synthesis steps of the coupling cycles to obtain the desired oligo- or polysaccharide. The reaction vessel may be one of a batch reaction vessel or semi-batch reaction vessel or a flow reactor. The term flow reactor as used herein refers preferably to a continuous flow reactor for performing the synthesis in a continuous manner.

For performing the coupling reactions appropriate building blocks and activators are supplied or added to the microwave transparent reaction vessel. Preferably, the building blocks and activators are supplied or added to the reaction vessel already containing the solid support or solid phase resin. The coupling reactions preferably take place inside the microwave transparent reaction vessel followed by appropriate deprotection, capping and/or washing steps. All of these synthesis steps and treatments in the synthesis cycles are preferably performed inside of the reaction vessel of the device of the present invention. The synthesis cycles are repeated as often as necessary to achieve the desired length of the desired oligo- or polysaccharide. The synthesis cycle is preferably repeated at least three times, more preferably at least four times or more preferably more than four times for obtaining an oligo- or polysaccharide.

For supplying liquids, solutions and/or gases the microwave transparent reaction vessel of the inventive synthesizer may further comprise one or more inlets, for example, for supplying washing solvents or washing solutions, building blocks or building block solutions, activators or activator solutions, capping solutions, deprotection solutions and inert gas, and may further comprise one or more outlets, for example, for discharging the liquids or solutions after each chemical reaction and/or also ventilation exits for inert gas or other gases from the reaction mixture.

In preferred embodiments of the present invention the one or more inlets may also function as one or more outlets, that means one or more inlets for supplying reagents, solvents, reaction mixtures, inert gas and the like can also be used to remove a solution, inert gas, reaction components and the like from the device for automated synthesis. In such embodiments one or more technical means such as one or more valves, valve assemblies, one or more vents, one or more manifolds and/or similar technical means may be located and mounted upstream to the one or more inlets of the reaction vessel. For example, by implementing a valve assembly or one or more valves in the device of the present invention the delivery of liquids, solutions and/or gases and also the discharging of liquids, solutions and/or gases after or during each chemical reaction or treatment may be realized through one inlet of the reaction vessel only or through two, three or more inlets of the reaction vessel. By implementation of technical means such as valves, valve assemblies, vents, manifolds or similar technical means, the total number of in- and outlets of the reaction vessel may be reduced.

Furthermore, technical means such as valves, valve assemblies, vents and manifolds and similar technical means may be electronically coupled to a **computing device** comprising at least one processor and a computer-readable storage medium comprising computer readable instructions thereon that may be executed by the processor, therefore the technical means may be under control of such a computing device. The processor may be configured to control the temperature setting of the pre-cooling device, may be configured to control the temperature setting of the cooling device, may be configured to set the time and power output of the microwave generator component, may be configured to control the amount and speed of delivering fluid from the reagent delivery component. The synthesizer preferably comprises one or more or a plurality of technical means electronically coupled to the computing device comprising at least one processor to allow the processor to control the one or more or plurality of technical means based on computer readable instructions stored on a computer readable medium which may be executed by the processor to enable the automation of the method for synthesizing oligo-, polysaccharides or peptides with the inventive synthesizer. The one or more technical means or plurality of technical means may be wired or wirelessly connected to the computing device. The one or more technical means or plurality of technical means may comprise one or more valves, valve assemblies, one or more vents and similar technical means of the one or more components and systems of the synthesizer of the present invention. Thus, the delivery of the liquids, solutions and/or gases and also the discharging of liquids and solutions may be under control of the computing device comprising the at least one processor and a computer-readable storage medium comprising computer readable instructions thereon. The computing device comprising the at least one processor and a computer-readable storage medium comprising computer readable instructions thereon may be configured to control said technical means in order to deliver liquids, solutions and/or gases to the microwave transparent reaction vessel and further to discharge liquids and solutions from the microwave transparent reaction vessel in a controlled and automated manner to allow automation of the synthesis on a solid support with the synthesizer of the present invention.

The microwave transparent reaction vessel may further comprise one or more openings or apertures for insertion of various other components or technical means. For example, the reaction vessel may further comprise an opening or aperture for insertion of a temperature probe or a temperature sensor for monitoring and measuring the temperature inside of the reaction vessel. Monitoring and measuring the temperature inside of the reaction vessel thereby also means monitoring and measuring the temperature of the reaction mixture during one or more or even all synthesis steps of the synthesis cycles. Thus, it is preferred that the reaction vessel further comprises at least one opening or aperture for insertion of a temperature sensor for monitoring and measuring the temperature inside of the reaction vessel.

The one or more inlets of the microwave transparent reaction vessel may be located at various positions of the reaction vessel body. In preferred embodiments of the present invention the microwave transparent reaction vessel may comprise one or more inlets at the top of the reaction vessel, for example, for delivery of washing solvents or washing solutions, building blocks or building block solutions, and activators or activator solutions and may further comprise one or more outlets at the top of the reaction vessel, for example, for exits for inert gas or other gases and may further comprise one or more inlets at the bottom of the reaction vessel, for example, for delivery of capping solutions, deprotection solutions and inert gas and may further comprise one or more outlets for discharging the liquids or solutions after each chemical reaction or after each washing step. As mentioned above, by implementing further technical means such as one or more valves, valve assemblies or similar technical means, these one or more inlets may also function as outlets for the liquids, solution and/or gases.

Thus, in preferred embodiments of the present invention the microwave transparent reaction vessel comprises one or more inlets at the top of the reaction vessel and one or more inlets at the bottom of the reaction vessel to enable two delivery methods for solvents and reagents, one from the top and another from the bottom of the reaction vessel. The usage of two different ports allows for the separation of incompatible reagents such as acids and bases, which may produce salts when combined. Over time these salts may build up and block tubing that may corrode components of the device of the present invention, which may lead to costly repairs. It is therefore preferred that the solutions to remove the temporary protecting groups (for example protecting groups such as Fmoc, Lev, ClAc, and NAP) and acetyl capping solutions are delivered via the one or more bottom inlets or bottom port of the reaction vessel. These reactions are desired to be performed rapidly and solutions are provided in excess, so these solutions will be pushed into the reaction vessel for example by pressurized inert gas such as argon. The inert gas inlet may serve for a dual purpose to not only deliver the reagents, but to efficiently mix the reaction. In addition, the one or more bottom inlets may serve to remove all the reagents and solutions from the reaction vessel. Oppositely, the activators or activator solutions and the building blocks or building block solutions are preferably delivered through the one or more top inlets or top port of the reaction vessel. These reagents are more valuable and are required to be delivered in a controlled stoichiometry, so these are delivered preferably dropwise, for example, via a syringe pump system.

In preferred embodiments of the present invention the microwave transparent reaction vessel may comprise only one inlet at the bottom of the reaction vessel, for example, for delivery of capping solutions, deprotection solutions and inert gas and for discharging the liquids or solutions after each chemical reaction or after each washing step and one or more inlets at the top of the reaction vessel, for example, for delivery of washing solvents or washing solutions, building blocks or building block solutions, and activators or activator solutions. The reaction vessel may further comprise openings or apertures at the top of the reaction vessel for insertion of, for example, a temperature probe or a temperature sensor. The reaction may further comprise an exhaust gases exit at the top of the reaction vessel.

In preferred embodiments of the present invention the microwave transparent reaction vessel may comprise at least three inlets/outlets, preferably four inlets/outlets (601, 602, 645, 655) at the top of the reaction vessel, for example, one inlet for delivery of washing solvents or washing solutions, one inlet for delivery of building blocks or building block solutions and one inlet for delivery of activators or activator solutions and preferably a further outlet for the exhaust of gases and/or mixing gas (655). The separate and distinct delivery of the building blocks and activators to the microwave transparent reaction vessel is advantageous for preventing a contacting of the building blocks and activators before supplying the building blocks and activators to the reaction mixture inside of the reaction vessel. It is particular preferred that the building blocks or building block solutions and the activators or activator solutions are not mixed or merged with each other before delivery to the reaction vessel. It is further advantageous that the washing solvents or washing solutions are separately supplied to the reaction vessel to allow rapid delivery of washing solvents or washing solution in a larger amount and to ensure well distribution of the washing solvents or washing solutions inside of the reaction vessel. For example, a channel for supplying the washing solvents or washing solution may be used to supply the washing solvents or washing solution to the reaction vessel. Such a channel may be adapted for spraying the washing solvents or washing solutions by splitting the liquid among a series of holes at the tip of the channel. The channel may also comprise a conical end to assure complete dropping of the washing solvents or washing solutions. Such a channel for delivery of washing solvents or washing solution allows to effectively wash-off other reagent solutions, reagents or adhered solid support from the inner walls of the reaction vessel. In contrary, the building blocks and activators are preferably supplied directly into the reaction mixture in a dropwise manner.

In embodiments of the present invention wherein the microwave transparent reaction vessel comprises one or more inlets at the bottom of the reaction vessel or only one inlet at the bottom of the reaction vessel it is preferred that the effective loading space or inner chamber of the reaction vessel is fenced by the bottom inlet. The bottom compartment may prevent canalization of fluid through a frit. A frit allows for dispersion of inert gas bubbling for mixing purposes. The frit may also ensure that the solid support remains inside of the reaction vessel. Thus, in preferred embodiments of the present invention the microwave transparent reaction vessel may further comprise a frit. It is preferred that the frit is located in the bottom compartment of the reaction vessel. Thus, to prevent solid support from being drawn from the bottom inlet, the end in the reaction vessel may be fitted with a frit or a filter.

Preferably the microwave transparent reaction vessel is made of a material such as glass, quartz, PTFE (Teflon), polypropylene or fluoropolymers. A reaction vessel made of glass may be provided in form of a triple-jacketed glass reaction vessel. The reaction vessel may be provided as a glass reaction vessel surrounded by a second cavity that allows for circulation of microwave transparent coolant fluid to control the temperature of the reaction mixture. Thus, the reaction vessel may be provided with a cooling jacket for cooling the microwave transparent reaction vessel.

In preferred embodiments of the present invention the microwave transparent reaction vessel may be provided as interchangeable reaction vessel. In such embodiments it is preferred that the reaction vessel may be removed from a cooling means such as a cooling jacket without interrupting the flow of a circulating coolant fluid.

In such embodiments the microwave transparent reaction vessel may be removed for example for loading with solid support or for discharging the solid support. Furthermore such an interchangeable reaction vessel allows use of various reaction vessels of different sizes for different batch sizes.

Therefore the present invention relates to a synthesizer for microwave-assisted automated multistep synthesis on a solid support comprising:
(a) an interchangeable, microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

In a further embodiment, the synthesizer for microwave-assisted automated multistep synthesis on a solid support comprises:
(a) an interchangeable, microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.
wherein the microwave transparent reaction vessel is adapted for receiving the solid support.

The inventors have surprisingly found that a microwave transparent reaction vessel made of fluoropolymers such as polytetrafluoroethylene (PTFE), perfluoroalkoxy alkanes (PFA), fluorinated ethylene propylene (FEP), ethylene tetrafluoroethylene (ETFE) or a reaction vessel comprising a coating of polytetrafluoroethylene (PTFE), perfluoroalkoxy alkanes (PFA), fluorinated ethylene propylene (FEP), ethylene tetrafluoroethylene (ETFE) is particularly suitable for the device for the synthesis of oligo- and polysaccharides on a solid support of the present invention.

The inventors of the present invention have found that the change in the manufacturing of the microwave transparent reaction vessels from silicate glass to fluoropolymer allows one the one hand integration of a microwave device into the synthesis cycle, and on the other hand improves chemical resistance, as well as eases of swapping reaction vessels of different sizes. Thus, it is advantageous to switch from a glass reaction vessel to one made of fluoropolymers such as perfluoroalkoxy alkanes (PFA). Microwave transparency, improved flow, minimal resin adherence, thermal resistance, and chemical resistance are among the many properties that PFA excels over glass. Typical silicate glass contains hydroxyl groups, which creates a hydrophilic surface. During the synthesis cycles, the resin often adheres to glass walls above the reaction solution causing deletion sequences and mixtures in the final product. To prevent adhesion problems, regular silanization of the glass reactor is necessary. On the other hand, PFA reactors have better hydrophobic properties and are non-adherent to the resin. This allows for reaction vessels made from PFA materials to tolerate a wider range of chemical reactions that are incompatible with silane coating on glass. An additional advantage is the physical durability of the PFA reactor compared to glass. Typically, AGA synthesizers are utilized for high throughput synthesis and are in constant use, so durable components are preferred.

In preferred embodiments of the present invention the microwave transparent reaction vessel is made of perfluoroalkoxy alkanes (PFA). In further preferred embodiments of the present invention the microwave transparent reaction vessel comprises a coating of perfluoroalkoxy alkanes (PFA). Perfluoroalkoxy alkanes are copolymers of tetrafluoroethylene (C₂F₄) and perfluoroethers (C₂F₃OR^{f}, where R^{f} is a perfluorinated group such as trifluoromethyl (CF₃).

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
wherein the microwave transparent reaction vessel is made of perfluoroalkoxy alkanes (PFA).

In one embodiment, the synthesizer for microwave-assisted automated multistep synthesis on a solid support comprises:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
wherein the microwave transparent reaction vessel is made of glass.

In preferred embodiments of the present invention the microwave transparent reaction vessel may further comprise a cap adapted for closing the reactor vessel for protecting the inner chamber or effective loading space from the external atmosphere. Thus, a cap may be provided for closing the reaction vessel in order to provide an isolated, anhydrous and inert atmosphere inside of the reaction vessel.

According to the present invention the synthesizer comprises a **reagent storing component.** The synthesizer of the present invention may comprise one or more reagent storing components. The synthesizer may comprise at least one reagent storing component. The reagent storing component or the one or more reagent storing components are preferably adapted for storing the building blocks and/or building block solutions, activators and/or activator solutions, washing solvents and/or washing solutions, deprotection solutions and/or capping solutions. The reagent storing component or the one or more reagent storing components may comprise one or more reagent containers or may comprise a plurality of reagent containers. The reagent storing component or the one or more reagent storing components may comprise one or more reagent containers for storing building blocks, one or more reagent containers for storing building block solutions, one or more reagent containers for storing activators, one or more reagent containers for storing activator solutions, one or more reagent containers for storing washing solvents and/or washing solutions, one or more reagent containers for storing deprotection solutions and one or more reagent containers for storing capping solutions. The reagent storing component or the one or more reagent storing components are preferably adapted for storing reagents and solvents in an anhydrous and inert atmosphere. In preferred embodiments of the present invention the reagents and solvents are stored under argon pressure. In preferred embodiments the reagent storing component or the one or more reagent storing components are connected to an inert gas delivery system. The gas delivery system may provide inert gas, such as argon, to the reagent storing component or the one or more reagent storing components. The inert gas delivery system or may provide inert gas, such as argon, to each of the containers of the reagent storing component or the one or more reagent storing components. Thus, in preferred embodiments of the present invention each of the containers of the reagent storing component or the one or more reagent storing components may be connected to an inert gas delivery system.

The containers may be made of any suitable chemical resistant material known in the art which is suitable for storing chemical reagents and/or solutions and furthermore suitable for storing the building blocks and/or building block solutions, activators and/or activator solutions, washing solvents or washing solutions, deprotection solutions and also capping solutions. The containers may preferably be adapted for storing the reagents in an anhydrous and inert atmosphere, for example, under argon pressure. For example, synthesis-ready building blocks may be pre-weighed and either dissolved in the appropriate anhydrous solvent or kept as solids and placed in sealed vials, for example, on a 64-position carousel or a 4-position means (e.g. test tube type containers) where a solution of building block (e.g. up to 8 mL of the appropriate concentration) is coupled to the system by multiport valves or similar technical means. Preferably a cap seals the microwave transparent reaction vessel which has an inlet for inert gas, such as argon, providing an anhydrous atmosphere and an outlet for the building block solution extraction. The extraction tube may reach the bottom container and may be allowed to take out the required volume of building block solutions. The building blocks are preferably those that can be synthesized in large quantities, are stable upon storage for extended periods of time, and upon activation result in very high coupling yields with excellent stereo-control. Solvents and reagents may for example be placed in various sizes of glass bottles and kept under inert gas, for example, under argon pressure.

Preferably the synthesizer comprises a reagent storing component for storing building blocks and/or building block solutions, activators and/or activator solutions, washing solutions and/or washing solvents and/or deprotection solutions. Preferably the synthesizer comprises a reagent storing component for storing one or more reagents and/or reagent solution and/or solvents. The reagents and/or reagent solutions preferably comprise building blocks, building block solutions, activator, activators solutions, washing solutions, washing solvents and deprotection solutions. Preferably the synthesizer comprises a reagent storing component for storing building blocks or building block solutions, activators or activator solutions, washing solutions or washing solvents, deprotection solutions and capping solutions.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component for storing building blocks and/or building block solutions, activators and/or activator solutions, washing solutions and/or washing solvents and deprotection solutions,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

The device of the present invention may comprise one or more liquid, solution or inert gas **delivery systems** for driving the flow of liquids or gases to the reaction vessel.

According to the present invention the synthesizer comprises a **reagent delivery system.** The inventive synthesizer may comprise at least one reagent delivery system. Preferably the synthesizer of the present invention comprises a reagent delivery system adapted for delivery of building blocks or building block solutions, activators or activator solutions, washing solvents or washing solutions, deprotection solutions, capping solutions and inert gas to the microwave transparent reaction vessel. Preferably the inventive synthesizer comprises a reagent delivery system adapted for receiving building blocks or building block solutions, activators or activator solutions, washing solvents or washing solutions, deprotection solutions and capping solutions from the reagent storing component. The device of the present invention may also comprise one or more reagent delivery systems. The reagent delivery system may comprise one or more reagent delivery sub-systems or reagent delivery sub-components for delivery of one or more of the one or more reagents, solutions and/or reagent solution to the reaction vessel.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system for delivering building blocks and/or building block solutions, activators and/or activator solutions, washing solutions and/or washing solvents and deprotection solutions,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

In one embodiment, the synthesizer for microwave-assisted automated multistep synthesis on a solid support comprises:
(a) a microwave transparent reaction vessel equipped with a temperature sensor,
(b) a microwave generator component,
(c) a reagent storing component,
(d) a reagent delivery system for delivering building block solutions, activator solutions, washing solvents and/or washing solutions and deprotection solutions,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

In one embodiment, the synthesizer for microwave-assisted automated multistep synthesis on a solid support comprises:
(a) a microwave transparent reaction vessel equipped with a temperature sensor,
(b) a microwave generator component,
(c) a reagent storing component for storing building blocks and/or building block solutions, activators and/or activator solutions, washing solutions and/or washing solvents and deprotection solutions,
(d) a reagent delivery system for delivering building block and/or building block solutions, activator solutions, washing solvents and/or washing solutions and deprotection solutions,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

Preferably the reagent delivery system connects the reagent storing component with the microwave transparent reaction vessel. Preferably the reagent delivery system is connected to the reagent storing component and is further connected to the microwave transparent reaction vessel. It is preferred that the reagent storing component and the microwave transparent reaction vessel are not directly connected to each other, in other words it is preferred that the reagent storing component and the microwave transparent reaction vessel are not in direct fluid communication with each other. Preferably the reagent delivery system is interposed between the reaction vessel and the reagent storing component. Preferably the reagent delivery system is in fluid communication with the reagent storing component and is further in fluid communication with the microwave transparent reaction vessel. It is preferred that the reagent delivery system receives the reagents from the reagent storing component and after receiving the reagents from the reagent storing component the reagents delivery system may deliver the reagents to the microwave transparent reaction vessel in order supply the reagents to the reaction mixture inside the microwave transparent reaction vessel.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
wherein the reagent delivery system connects the reagent storing component with the microwave transparent reaction vessel.

The reagent delivery system may be connected to the microwave transparent reaction vessel through one or more inlets of the reaction vessel for delivery of the reagents and/or reagents solution and/or solvents to the reaction vessel through the one or more inlets of the reaction vessel. The reagent delivery system may be connected to the microwave transparent reaction vessel through one or more reagent delivery lines. Thus, the reagent delivery system may be adapted to deliver reagents and/or reagent solutions and/or solutions and/or solvents to the reaction vessel and may be further adapted to supply the reagents and/or reagent solution and/or solutions or solvents through the one or more inlets of the reaction vessel. Preferably the reagent delivery system may be adapted to deliver one or more reagents and/or one or more reagent solutions and/or one or more solutions in different amounts, at different points in time and in a specific sequence or specific order, and further through specific inlets of the one or more inlets of the reaction vessel during the synthesis cycles on a solid support. It is therefore preferred that the reagent delivery system comprises suitable technical means, preferably suitable technical means electronically coupled to a computing device comprising at least one processor, for delivery of one or more reagents and/or one or more reagent solutions and/or one or more solutions in different amounts, at different points in time and in a specific sequence or specific order during the synthesis cycles. The reagent delivery system may comprise a pump system or one or more pumps such as syringe pumps, peristaltic pumps or other suitable pumps and may further comprise one or more valves or one or more valve assemblies, one or more manifolds, one or more distributing components and similar suitable technical means. It is preferred that the pump system or the one or more pumps, the one or more valves or the one or more valve assemblies, the one or more manifolds and/or the one or more distributing components and similar technical means are under control of a computing device comprising at least one processor.

The synthesizer of the present invention may therefore comprise one or more valves or valve assemblies for regulating, directing or controlling the flow of fluids like gases or liquids. Valves or valve assemblies may be adapted for opening, closing or partially obstructing various passageways. Valves may be operated in gradual change between two or more positions, such as in two-port, three-port, four-port or in multiport valves. The inventive synthesizer may comprise one or more fluidic valves operably connected to one or more components of the synthesizer. Each fluidic valve of the synthesizer of the present invention may be a rotary valve, solenoid valve block or other multi-port valve or valve system or other suitable valves known to those skilled in the art. The synthesizer of the present invention may further comprise one or more pumps such as syringe pumps, peristaltic pumps or other pumps known to those skilled in the art which may be operably connected to one or more fluidic valves or valve assemblies of the synthesizer of the present invention.

In preferred embodiments the reagent delivery system of the synthesizer according to the present invention may comprise one or more valves, preferably one or more fluidic valves, more preferably one or more rotary valves, and even more preferably one or more rotary valves with 3, 4, 5, 6, 7, 8, 9 or 10 ports. Preferably the reagent delivery system may comprise one or more valves such as one or more rotary valves, one or more solenoid valve blocks or other multiport valves. The reagent delivery system may further comprise one or more pumps or a pump system. Preferably, the reagent delivery system comprises at least one syringe pump. Preferably, the reagents may be delivered via a syringe pump system. Preferably the reagents may be delivered via a syringe pump system as a component of the reagent delivery system. Preferably, the syringe pump system may be a component of the reagent delivery system.

In preferred embodiments of the present invention a syringe pump may drive the fluids within the reagent delivery system. The syringe pump and the reagent delivery system may be connected through one or more loop lines. Loop lines allow for careful delivery of sensitive and/or corrosive chemical solutions. The resting volume of the loops is defined as a function of the tubing length. A driving solvent may be taken to fill up the loops ahead the withdrawing of chemical solution from the reagent delivery system. This avoids the direct contact of the reagents with the syringe pump, which prevents pump deterioration and cross-contamination. The loops may be made from an inert material such as, for example, Teflon, poly-(tetrafluoroethylene) (PTFE) and the like. The size of the loops may be varied. The exact size will depend on the capacity of the syringe pump and the amount of reagent to be delivered to the reaction vessel. The size of each loop will also depend on the nature of the reagent to which it is associated. For example, if the reaction vessel is 20 mL, then a loop sized from about 1 to 5 mL may be used, preferably from about 2 to 4 mL. Each loop may be sized the same or different. For example, loops attached to building blocks may be smaller than those attached to basic reagents such as the deprotection or capping reagents as the quantity of the former used during any synthesis step is relatively small compared to the amount of such basic reagents.

As already described above in preferred embodiments of the present invention the microwave transparent reaction vessel may comprise one or more inlets at the top of the reaction vessel and may comprise one or more inlets at the bottom of the reaction vessel. In further preferred embodiments the microwave transparent reaction vessel may comprise one or more inlets at the top of the reaction vessel and may comprise only one inlet at the bottom of the reaction vessel. The reagent delivery system may be connected to the microwave transparent reaction vessel through the one or more inlets at the top of the reaction vessel and/or through the one or more inlets at the bottom of the reaction vessel. In preferred embodiments of the present invention the reagent delivery system may be adapted to deliver building blocks or building block solutions, activators or activator solutions, and washing solvents or washing solutions through the one or more inlets at the top of the reaction vessel. The reagent delivery system may be further adapted to deliver deprotection solutions and capping solutions through the one or more inlets at the bottom of the reaction vessel. The reagent delivery system may be further adapted to deliver inert gas, such as argon, to the reaction vessel.

The reagent delivery system may comprise one or more reagent distribution components. The reagent delivery system may comprise one or more reagent distribution components for delivery of reagents and/or solutions and/or reagent solution to the reaction vessel. Each of the reagent distribution components may be connected to the reagent storing component or one or more reagent storing components. In preferred embodiments of the present invention the reagent delivery system comprises a first and a second reagent distribution component for driving the flow of fluids or gases to the reaction vessel. In preferred embodiments of the present invention the synthesizer comprises a first reagent distribution component and a second reagent distribution component.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system comprising a first reagent distribution component and a second reagent distribution component,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

The first reagent distribution component may be connected to the microwave transparent reaction vessel through the one or more inlets on the top of the reaction vessel. Thus, the first distribution component or the top distribution component may be in fluid communication with the microwave transparent reaction vessel through the one or more inlets at the top of the reaction vessel. The second distribution component may be connected to the one or more inlets at the bottom of the reaction vessel. Thus, the second distribution component or bottom distribution component may be in fluid communication with the reaction vessel through the one or more inlets at the bottom of the reaction vessel.

Preferably the first distribution component or the top distribution component supplies the washing solvents, building blocks or building block solutions and activators or activator solutions to the microwave transparent reaction vessel. The top distribution component may be also adapted to provide a ventilation exit for gases.

Preferably the second distribution component or the bottom distribution component supplies the deprotection solutions, capping solutions and supplies inert gas (bubbling gas) to the microwave transparent reaction vessel. The inert gas may be provided as bubbling gas which may be used for mixing and creating an inert and anhydrous atmosphere inside the reaction vessel. In preferred embodiments the bottom distribution component is also adapted for discharging the liquids or solution after one or more synthesis or washing steps.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system comprising a first reagent distribution component for delivery of washing solvents, building blocks and/or building block solutions and activators and/or activator solutions and a second reagent distribution component for delivery of deprotection solutions,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

In preferred embodiments of the present invention the reagent delivery system or the one or more reagent distribution components may further comprise one or more reagent distribution sub-components. In preferred embodiments of the present invention the reagent delivery system may comprise a building block distribution component for delivery of the building blocks and/or building block solutions, an activator distribution component for delivery of the activators and/or the activator solutions and/or a washing solvent distribution component for delivery of washing solvents or washing solution, a deprotection distribution component for delivery of deprotection solution and may further comprise a capping distribution component for delivery of capping solutions to the reaction vessel.

In preferred embodiments of the present invention the **top distribution component** may comprise a building block distribution component, an activator distribution component and a washing solvents or washing solutions distribution component. The top distribution component may further comprise a syringe pump for driving the fluids within the top distribution component. The lines between the syringe pump and the top distribution component may be provided in form of loop lines to allow for careful delivery of sensitive and/or corrosive chemical solutions. The resting volume of the loops is defined as a function of the tubing length. The top distribution component may further comprise a washing solvents component, wherein a driving solvent may be taken to fill up the loops ahead the withdrawing of chemical solutions from the building blocks distribution component and the activator distribution component. This avoids the direct contact of the reagents with the syringe pump, which prevents the pump deterioration and cross-contamination.

The **building block distribution component** may comprise one or more valves or a valve assembly or similar technical means, for example, a rotary valve that distributes the fluids within the building block distribution component. The building block distribution component is connected to the reagent storing component, preferably to a building blocks storing component which may comprise one or more building block containers or building block solution containers. Each of the building block containers or building block solution containers may be connected to the one or more valves or valve assembly or similar technical means. The building block containers of the building block storing component may be connected to a gas delivery system to provide inert gas to each of the building block containers. Thus, each of the building block containers may be stored under anhydrous and inert atmosphere. Each of the building block containers or building block solutions containers may be in fluid communication with the building block distribution component through the one or more valves or valve assembly or similar technical means. The one or more valves or the valve assembly may be further connected to the reaction vessel. Thus, the microwave transparent reaction vessel may be in fluid communication with the building block distribution component through the one or more valves or valve assembly or similar technical means. The reaction vessel may be in fluid communication with the building block distribution component through, for example, one or more valves or valve assembly and may be further connected through a building blocks delivery line. The building block distribution component may be also connected to a waste container and/or gas delivery system for receiving inert gas through one or more further delivery lines. The one or more valves or valve assembly or other suitable technical means for distribution of the building blocks within the building block distribution component may be under control of a processor of a computing device configured to control the distribution of the building blocks and preferably configured to control the one or more valves or valve assembly such as a rotary valve distributor. The building block distribution component may be connected to a syringe pump system, for example, through one or more valves or valve assembly and may be further connected to the syringe pump via a loop line.

The **activator distribution component** may comprise one or more valves or a valve assembly or similar technical means for example a rotary valve that distributes the fluids within the activator distribution component. The activator distribution component is connected to a reagent storing component, preferably an activator or activator solution storing component which may comprise one or more activator containers or activator solution containers. The activator containers of the activator storing component may be connected to a gas delivery system to provide inert gas to each of the activator containers. Thus, each of the activator containers may be stored under anhydrous and inert atmosphere. Each of the activator containers or activator solution containers may be connected to the one or more valves or valve assembly or similar technical means of the activator distribution component. Thus, each of the activator containers or activator solutions container may be in fluid communication with the one or more valves or valve assembly of the activator distribution component. The activator distribution component may be connected to the reaction vessel through the one or more valves or valve assembly or similar technical means. Thus, the microwave transparent reaction vessel may be in fluid communication with the activator distribution component through the one or more valves or valve assembly or similar technical means. The microwave transparent reaction vessel may be in fluid communication with the activator distribution component through one or more valves or valve assembly or similar technical means and may be further connected through an activator delivery line. The activator distribution component may be also connected to a waste container and/or to a gas delivery system for receiving inert gas through one or more delivery lines. The one or more valves or valve assembly or similar technical means may be under control of a processor of a computing device configured to control the distribution of the activators within the activator distribution component and preferably configured to control the one or more valves or valve assembly and similar technical means such as a rotary valve distributor. The activator distribution component may be connected to a syringe pump system, for example, through one or more valves or valve assembly and may be further connected to the syringe pump via a loop line.

The **washing solvent distribution** component may provide the reaction vessel with one or more washing solvents or washing solutions. Exemplary washing solvents suitable for automated synthesis of oligo-, polysaccharides and peptides on a solid support include, but are not limited to dichloromethane (DCM), tetrahydrofuran (THF) and dimethylformamide (DMF). The washing solvent distribution component is connected to a reagent storing component, preferably a washing solvent storing component. Each of the washing solvents may be stored in a respective solvent container of the washing solvent storing component. Thus, the washing solvent distribution component may be connected to a washing solvent storing component comprising one or more solvent containers. The washing solvent distribution component may be further connected to a syringe pump. One or more of the solvent containers may be connected to the syringe pump through the washing solvent distribution component. The washing solvent distribution component may comprise one or more valves or valve assembly or similar technical means which may be connected to each of the solvent containers of the washing solvents storing component. As an example, the washing solvent distribution component may comprise a multi-port valve, such as a four way magnetic valve. The washing solvents may be delivered to the microwave transparent reaction vessel by the washing solvent distribution component. The washing solvents may be delivered to the reaction vessel through a washing solvent delivery line. The washing solvent containers of the washing solvent storing component may be connected to a gas delivery system to provide inert gas to the each washing solvent container. Thus, each of the washing solvent containers may be stored under anhydrous and inert atmosphere, such as under argon atmosphere. The magnetic valve may be further connected to the gas delivery system to receive inert gas and to provide and deliver inert gas to the microwave transparent reaction vessel. The one or more valves or valve assembly or similar technical means suitable for distribution of the washing solvents within the washing solvent distribution component may be under control of a processor of a computing device configured to control the distribution of the washing solvents and washing solution and preferably configured to control the one or more valves or valve assembly and similar technical means such as a magnetic valve distributor.

The **bottom distribution component** may comprise one or more valves such as one or more multi-port valves, or valve assemblies, or similar technical means. The bottom distribution component may comprise a deprotection distribution component for delivery of deprotection solutions to the microwave transparent reaction vessel and may further comprise a capping distribution component for delivery of capping solution to the microwave transparent reaction vessel. The bottom distribution component may be further connected to a waste container. The bottom distribution component may be further connected with a gas delivery component for delivery of inert gas, such as argon, to the reaction vessel. One or more of the components of the bottom distribution component may be connected to the microwave transparent reaction vessel through a loop line which allows for delivery of chemical solutions from the deprotection distribution component, the capping distribution component and delivery of inert gas from the gas delivery component. The loop lines may be also adapted for discharge of waste liquids from the reaction vessel. The bottom distribution component may comprise for example a multiple ways magnetic valve which may be electronically coupled to a computing device comprising at least one processor configured to control the delivery of deprotection solutions, capping solutions and inert gas to the reaction vessel and further configured to control the discharging of liquids and solutions of the reaction vessel after each reaction or washing step. The deprotection distribution component is connected to a reagent storing component, preferably a deprotection solution storing component. The deprotection storing component may comprise one or more containers for storing deprotection reagents. The deprotection storing component may be connected to a gas delivery system to provide inert gas to the each deprotection reagent container. Thus, each of the deprotection reagent containers may be stored under anhydrous and inert atmosphere, such as under argon atmosphere. The capping distribution component is connected to a reagent storing component, preferably a capping solution storing component. The capping storing component may comprise one or more containers for storing capping reagents. The capping storing component may be connected to a gas delivery system to provide inert gas to the each capping reagent container. Thus, each of the capping reagent containers may be stored under anhydrous and inert atmosphere, such as under argon pressure.

The synthesizer of the present invention may further comprise an **inert gas delivery system** to provide anhydrous and inert gas to one or more components of the synthesizer of the present invention for mixing, atmosphere conditioning and driving fluid purposes. Preferably the synthesizer is adapted for performing reactions under anhydrous and inert atmosphere. The gas delivery system comprises at least one gas container, such as an argon container, to provide inert gas to the one or more components of the inventive synthesizer. The gas delivery system may comprise one or more valves, valve assemblies, one or more vents and/or one or more manifolds or similar technical means to allow the distribution and delivery of inert gas to the one or more components of the synthesizer of the present invention. The one or more valves, valve assemblies, one or more vents and/or one or more manifolds or similar technical means allow control of the pressure within the one or more lines which connect the one or more components of the synthesizer. The one or more valves, valve assemblies, one or more vents and/or one or more manifolds or similar technical means may be under control of a computing device comprising at least one processor. The processor may be configured to control the delivery and distribution of the inert gas within the inventive synthesizer and is configured to control gas pressure within the one or more lines and one or more components of the synthesizer of the present invention.

In one embodiment of the present invention, the inventive synthesizer is equipped with an inert gas delivery system comprising a gas container and a gas valve manifold establishing a separate fluid connection between each of the one or more reagent containers and the gas container, wherein at least two fluid communications are separate of each other. Preferably, in such embodiments the reagent delivery system is configured to establish a fluid communication between each of the one or more reagent containers and the microwave transparent reaction vessel, wherein at least two fluid communications are separate of each other. The usage of at least two separate fluid communications between the gas delivery system and the reagent storing component as well as between the reagent storing component and the reagent delivery system allows for the separation of incompatible reagents such as acids and bases, which may produce salts when combined. Over time these salts may build up ubiquitously in the synthesizer and block tubing and valves that may corrode sensitive components of the inventive synthesizer, which may lead to costly repairs. It is therefore preferred that the solutions to remove the temporary protecting groups (for example protecting groups such as Fmoc, Lev, ClAc, and Nap) and acetyl capping solutions are delivered via separate fluid communications to the microwave transparent reaction vessel. To establish at least two separate fluid communications between the reagent storing component and the microwave transparent reaction vessel, the reaction vessel is preferably equipped with at least two inlets, wherein the at least two inlets are located at different compartments of the reaction vessel in order to avoid premature mixing of incompatible reagents. Thus, the microwave transparent reaction vessel is preferably equipped with at least one top inlet and at least one bottom inlet.

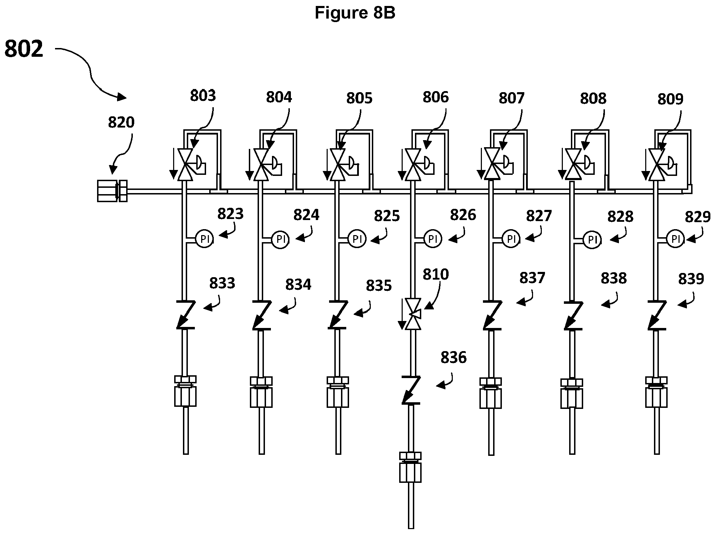

**Fig. 8B** shows in detail the gas manifold of the inner gas system. Each line counts at least with: a pressure regulator valve (**803-809**), a pressure sensor (**823-829**), and a check valve (**833-839**). In addition, a flow control valve **810** allows regulating the flow rate of inert gas serving as bubbling gas for mixing the reagent. The inlet and outlets tubing of manifold are provided with tubing connectors (e.g. **820**) to prevent leakages In absent of pressure difference (standby mode); the check valve will close isolating the atmospheres from the different blocks; services (bubbling, reactor discharging gas), reagents (building block/donor, activators, deprotection and capping) and solvents. Such arrangement prevents the undesirable contamination by volatile and incompatible species used during the glycosylation

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system configured to establish a fluid communication between each of the one or more reagent containers and the microwave transparent reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a separate fluid connection between each of the one or more reagent containers and the gas container, wherein at least two fluid communications are separate of each other.

In one embodiment, the synthesizer for microwave-assisted automated multistep synthesis on a solid support comprises:
(a) a microwave transparent reaction vessel equipped with a temperature sensor comprising at least one inlet at the top of the reaction vessel and at least one inlet at the bottom of the reaction vessel,
(b) a microwave generator component,
(c) a reagent storing component,
(d) a reagent delivery system configured to establish a fluid communication between each of the one or more reagent containers and the microwave transparent reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a separate fluid connection between each of the one or more reagent containers and the gas container, wherein at least two fluid communications are separate of each other.

The synthesizer according to the present invention comprises **a cooling device** for cooling the reaction vessel. The cooling device therefore provides active cooling to the microwave transparent reaction vessel and thus provides active cooling of the reaction mixture inside of the reaction vessel. The cooling device is therefore preferably in thermal communication with the microwave transparent reaction vessel. The cooling device is preferably adapted for cooling the reaction vessel to temperatures of -80°C to +60 °C, preferably -40°C to +40°C, more preferably -40°C to +25°C, and more preferably -40°C to +20°C. Thus, the synthesizer of the present invention may comprise a cooling device for regulating the temperature of the reaction vessel. The cooling device may be under control of a thermal controller as a part of a computing device comprising at least one processor. The cooling device may be further adapted to maintain the temperature within the reaction vessel. The cooling device may be adapted to adjust the temperature within +1°C and -1°C of the reaction temperature. The cooling device may be equipped with a temperature sensor or thermometer, wherein the cooling device temperature may be adjusted either manually or preferably by a computing device, more preferably by a thermal controller. For example, the cooling device could be an external refrigerated circulator or a cooling block, or a chiller. The cooling block may be made of any heat transfer material. The block may have channels running through to pass microwave transparent coolant fluid through. The microwave transparent reaction vessel may be placed in a cavity of the cooling block. In a preferred embodiment of the present invention the microwave transparent coolant fluid may be circulated around the microwave transparent reaction vessel via a sleeve surrounding the reaction vessel. In preferred embodiments of the present invention the cooling device may comprise a cooling jacket. The cooling jacket may be in form of a cooling coil surrounding the microwave transparent reaction vessel. Preferably, the cooling jacket is in thermal communication with the microwave transparent reaction vessel. The cooling coil or cooling jacket may be further provided with a thermal isolation cover to increase the heat exchange efficiency of the cooling jacket or cooling coil.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel, wherein the cooling device comprises a cooling jacket in thermal communication with the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

In one embodiment, the synthesizer for microwave-assisted automated multistep synthesis on a solid support comprises:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel, comprising a cooling coil in thermal communication with the reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

The microwave transparent reaction vessel may be constructed as a double-wall structure which forms two cavities, wherein the first cavity accommodates the multi-step synthesis of oligo-, polysaccharides or peptides and wherein the second cavity accommodates a microwave transparent coolant fluid. The double-wall structure of the microwave transparent reaction vessel may be made of glass. Thus, the microwave transparent reaction vessel may be provided with a cooling jacket for cooling the reaction vessel. Preferably, the cooling jacket may be made of a microwave transparent material such as glass, quartz, PTFE (Teflon), polypropylene or fluoropolymers. In preferred embodiments of the present invention the reaction vessel may be provided as an interchangeable microwave transparent reaction vessel. In such embodiments it is preferred that the microwave transparent reaction vessel may be removed from a cooling means such as a cooling jacket without interrupting the flow of a circulating microwave transparent coolant fluid. In such embodiments the reaction vessel may be removed for example for loading with solid support or for discharging the solid support. Furthermore such an interchangeable reaction vessel allows use of various reaction vessels with different sizes for different batch sizes.

The cooling device may be connected to a coolant fluid reservoir. The cooling device may be connected to a cooling circuit pump. In preferred embodiments of the invention the cooling jacket surrounding the reaction vessel may be connected to a coolant fluid reservoir and a coolant circuit pump. The coolant fluid reservoir, the coolant circuit pump and the cooling jacket surrounding the reaction vessel may form a closed cooling circuit. The cooling jacket may be provided in form of a cooling coil surrounding the reaction vessel. The coolant circuit pump may be under control of a computer comprising at least one processor. The cooling device may comprise a cooling unit configured to control the temperature of the cooling device. The cooling unit may be electronically coupled to a computing device comprising at least one processor. The cooling circuit pump may be electronically coupled to a computing device comprising at least one processor to control the flow of the microwave transparent coolant fluid through the cooling circuit.

In one embodiment of the present invention the synthesizer comprises at least one **cooling device** for cooling the microwave transparent reaction vessel or more precisely for cooling the content of the reaction vessel and at least one **pre-cooling device** for pre-cooling the reagents to be supplied to the reaction vessel. The cooling device provides at least active cooling of the reaction vessel. The pre-cooling device provides at least active cooling of one or more of the reagents and/or washing solutions to be supplied to the reaction vessel.

Therefore the present invention relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system comprising a first reagent distribution component for delivery of washing solvents, building blocks and/or building block solutions and activators and/or activator solutions and a second reagent distribution component for delivery of deprotection solutions,
(e1) a cooling device for cooling the microwave transparent reaction vessel,
(e2) a pre-cooling device for pre-cooling the reagents to be supplied,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

In one embodiment the synthesizer for microwave-assisted automated multistep synthesis on a solid support comprises:
(a) a microwave transparent reaction vessel equipped with a temperature sensor,
(b) a microwave generator component,
(c) a reagent storing component,
(d) a reagent delivery system comprising a first reagent distribution component for delivery of washing solvents, building blocks and/or building block solutions and activators and/or activator solutions and a second reagent distribution component for delivery of deprotection solutions,
(e1) a cooling device for cooling the microwave transparent reaction vessel,
(e2) a pre-cooling device for pre-cooling the reagents and washing solutions to be supplied,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

Current synthesizers of the prior art require expensive and large cooling systems, thereby allowing, in principle, active cooling of the reaction vessel during the coupling reactions. However, the synthesizers known in the art still suffer from low to moderate yields, decomposition of reagents and formation of side and decomposition products. These deficiencies could neither be remedied by cooling the reaction vessel to even lower temperatures or by a very slow addition and careful delivery of reagents to the reaction mixture. The first attempt did not result in the desired improvement of yield and reduction of decomposition and decrease of formation of side and decomposition products, and the latter approach led to an impractical increase of the total reaction time, while simultaneously also the side and decomposition products increased without the desired enhancement of the yield. In addition, up-scaling of this way running the reaction was almost impossible. Thus, the inventors have found that pre-cooling of the reagents prevents and avoids decomposition of the reagents during the synthesis cycles which otherwise could result in undesired side-products and decomposition products.

As an example, Fig. 6 shows a chart comparing temperature readings inside the reaction vessel, taken during a synthesis cycle, in the presence and absence of the pre-cooling device. The three thermal stages are identified. It can be clearly recognized that temperature spikes appear when a liquid is dispensed in the reaction vessel. The dashed curve shows the temperature profile for the device without pre-cooling of the reagents to be supplied. The thermal spikes are remarkable at the pre-coupling regime (subzero temperatures). The solid line depicts the temperature profile with active pre-cooling. The incoming solutions of reagents are effectively pre-cooled and this suppresses the undesired temperature spikes.

The inventive synthesizer comprising an improved cooling system comprising a cooling device and a pre-cooling device allows the prevention of fluctuations in temperature of a reaction mixture which may otherwise result in loss of sensitive intermediates and reagents, in particular in the coupling steps of the synthesis cycles. Thus, with the cooling system of the synthesizer comprising a cooling device and a pre-cooling device unfavorable temperature spikes may be effectively prevented during the transfer of building blocks and activators to the microwave transparent reaction vessel. Furthermore, the cooling system of the present invention allows for advantageous temperature adjustments of the microwave transparent reaction vessel in the pre-coupling regime and further of the reaction mixture to prevent decomposition of the reactants, sensitive intermediates and desired products at different thermal stages of the synthesis cycles. Thus, the improved cooling system of the inventive synthesizer consists of a cooling device and a pre-cooling device. Therefore the inventive synthesizer comprises a cooling device for cooling the microwave transparent reaction vessel and a pre-cooling device for pre-cooling the reagents to be supplied for control of the temperature of one or more reagents and/or the reaction mixture during one or more synthesis steps of the synthesis cycles to prevent decomposition of the sensitive intermediates and reagents. Of course, the cooling device for cooling the microwave transparent reaction vessel and the pre-cooling device for pre-cooling the reagents can be combined to a single cooling device for cooling both, the reaction vessel and the reagents to be supplied. Most preferably the one cooling device for both is a Peltier cooling element.

As used herein, the term >> **pre-cooling the reagents** << refers to the cooling of the reagent storing component and/or the cooling of the reagent delivery system to the reaction vessel and preferably to the cooling of the reagent delivery system and more preferably to the cooling of the reagent storing component and the cooling of the reagent delivery system.

Preferably the pre-cooling device is in thermal communication with the reagent delivery system. The pre-cooling device is preferably interposed between the microwave transparent reaction vessel and the reagent delivery device. The pre-cooling device is preferable positioned in close proximity to the one or more inlets of the microwave transparent reaction vessel. Thus, the pre-cooling device may be adapted to function as a pre-cooling inlet for pre-cooling the reagents to be supplied. The pre-cooling component may be adapted for active cooling of the reagents to be supplied. In other words the incoming solutions at room temperature from the reagent delivery system are actively cooled by the pre-cooling device to a temperature around the preset temperature of the reaction mixture in the reaction vessel. By pre-cooling the at room temperature incoming solutions to around the temperature of the reaction vessel the reagents to be supplied are added to the reaction mixture at a temperature that preferably corresponds to the temperature of the reaction mixture in the microwave transparent reaction vessel.

In one embodiment, all reagents and solvents, including, building blocks, activators and washing solvents, are pre-cooled before addition to the reaction mixture in the microwave transparent reaction vessel. In one embodiment, only the activators or activator solutions are pre-cooled before addition to the reaction mixture in the reaction vessel. Preferably at least the building blocks and activators (or a solution thereof) are pre-cooled before addition to the reaction mixture in the reaction vessel and preferably all reagents and solutions added to perform the coupling reaction are pre-cooled close to the temperature of the reaction mixture in the reaction vessel. Furthermore, the resulting intermediates are often temperature sensitive and may decompose at higher temperatures then the desired temperature of the reaction mixture. It should be clear that already formed temperature sensitive intermediates in the reaction mixture may decompose in case further warmer reagents or solvents are added to the reaction mixture, such as reagents or solvents at room temperature. In such a case it may be suitable to supply these reagents or solvents in a slow and careful manner, for example dropwise manner, by monitoring the temperature of the reaction mixture at the same time. However, in larger batch sizes a larger amount of the reagents to be supplied are added to the reaction mixture. The delayed addition, for example dropwise addition, and the period of time until the reaction mixture is adjusted to the desired and preset temperature may also result in decomposition of the reagents and in particular the sensitive intermediates. The inventors of the present invention have found that on the one hand the rapid addition of the reagents to the reaction mixture in the pre-coupling stage and impregnating of the resin to allow these reagents to diffuse through the porous solid for a period of time of 1 minute to 20 minutes, preferably 5 minutes has an advantageous effect on the overall yield of the desired oligo- and polysaccharide.

On the other hand the inventors have found that the monitoring of the reaction mixture temperature during the addition of reagents and solvents and the careful and slow addition of these reagents such that the reaction mixtures maintains the desired reaction mixture has also an advantageous effect on the overall yield of the desired oligo-, polysaccharide or peptide. The inventive synthesizer comprising a pre-cooling device for pre-cooling the reagents to be supplied has the advantage that the pre-cooled reagents to be supplied may be added in a faster and rapid manner to the reaction mixture without unfavorable temperature fluctuations in the reaction mixture in the pre-coupling stage at preferably -40°C to -10°C and has the advantages that the added pre-cooled reagents may be allowed to impregnate the resin to diffuse through the porous solid for a period of time of 1 minute to 20 minutes, preferably 5 minutes such that the overall time of the pre-coupling stage including the adding and impregnating of the reagents may be reduced such that decompositions of the reagents and the resulting intermediates may be effectively prevented in comparison to a slow and careful addition of the reagents to be supplied which may result in an overall time of the pre-coupling stage of over 25 minutes or 30 minutes or more.

The rapid addition of the pre-cooled reagents in the pre-coupling stage to allow effective impregnation of the resin and the prevention of the temperature fluctuation during the addition of the pre-cooled reagents has been shown to be particularly advantageous with regard to the glycosylation reactions in the synthesis of oligo- and polysaccharides.

The pre-cooling device is preferably adapted for pre-cooling of the reagents to be supplied to the corresponding temperature of the reaction mixture in the microwave transparent reaction vessel. For example, if the microwave transparent reaction vessel is actively cooled to a temperature of -30° in the pre-coupling stage of a synthesis cycle the reagents to be supplied may preferably be pre-cooled also to a temperature of -30°C by the pre-cooling device or preferably to -31 °C or -32°C or -33°C or to a lower temperature. In such a case the pre-cooled reagents at a temperature of -30°C are added to the reaction mixture having a temperature of -30°C, wherein temperature fluctuations in the reaction mixture are prevented during addition of the reagents. However, the reagents to be supplied to the reaction vessel may be also added at a slightly higher temperature then the temperature of the reaction mixture in the reaction vessel.

Preferably the reagents are pre-cooled by the pre-cooling device to a temperature in the temperature range corresponding to the temperature range of -40°C to -8°C, preferably of -30°C to -9°C, and more preferably to the temperature range of -20°C to -10°C of the pre-coupling stage. In preferred embodiments of the present invention the pre-cooling device may be adapted for pre-cooling the reagents to be supplied at least to a temperature which is not higher than -3°C, preferably -6°C, more preferably -8°C or -10°C of the temperature of the reaction mixture in the transparent reaction vessel so that the temperature of the supplied reagents is not higher than 3°C, preferably 6°C, more preferably 8°C or 10°C below the temperature of the reaction mixture in the reaction vessel. Preferably the pre-cooling device may be adapted to cool the reagents to be supplied to a temperature in the range of 3°C to 6°C below the temperature of the reaction mixture in the reaction vessel. Thus, it is preferred that the reagents to be supplied are pre-cooled to the corresponding temperature of the reaction vessel with a maximum variance of 0°C to 10°C below the temperature of the reaction mixture in the reaction vessel. Most preferably the temperature of the reaction mixture in the reaction vessel does not exceed a temperature of -10°C in the pre-coupling stage of the synthesis cycle.

Thus, it is preferred that the reagents to be supplied are pre-cooled to the corresponding temperature of the reaction vessel with a maximum variance of 10°C or maximum deviation of 10°C, preferably with a maximum variance of 5°C or maximum deviation of 5°C, more preferably with a maximum variance of 3°C or maximum deviation of 3°C. In preferred embodiments it is preferred that the temperature of the reaction mixture in the reaction vessel does not exceed a temperature of -10°C in the pre-coupling stage of the synthesis cycle.

Most preferably, the temperature of the reagents to be supplied to the reaction vessel is in the range of -18°C to -8°C, preferably in the range of -16°C to -9°C, more preferably in the range of -14°C to -10°C and still more preferably in the range of -12°C to -10°C.

In preferred embodiments of the present invention various liquid lines such as washing solvents lines, building block lines, and activator solution lines may be pre-cooled before feeding the reaction vessel. In other words the pre-cooling device may be located upstream to the microwave transparent reaction vessel. The pre-cooling device may be interposed between the microwave transparent reaction vessel and the reagent delivery system.

Thus, the microwave transparent reaction vessel may be connected to a pre-cooling device which allows active pre-cooling of various liquid lines such as washing solvents, building blocks, activator solution before said washing solvents, building blocks, activator solution are delivered and supplied to the reaction vessel. In such embodiments the flow of liquid to the microwave transparent reaction vessel from the reagent delivery system is not interrupted as the reagents to be supplied to the reaction vessel are pre-cooled during the delivery to the reaction vessel. Preferably the flow speed of the liquids through the various liquid lines are taken into account as by lower flow speeds the reagents may be pre-cooled for a longer period of time to enable effective cooling to the desired temperatures. In one embodiment, all liquid lines between the reagent delivery system and the microwave transparent reaction vessel are pre-cooled by the pre-cooling device located between the reagent delivery system and the reaction vessel. In one embodiment, only one liquid line between the reagent delivery system and the reaction vessel is pre-cooled by the pre-cooling device located between the reagent delivery system and the reaction vessel. In a further embodiment, at least one liquid line between the reagent delivery system and the reaction vessel is pre-cooled by the pre-cooling device located between the reagent delivery system and the microwave transparent reaction vessel.

In preferred embodiments the pre-cooling device may comprise a metal cooling surface made of a metal heat exchange material. In such embodiment the building block delivery line and activator line may contact the metal cooling surface at a preset temperature. Thus, the metal cooling surface may be cooled to a predefined temperature. Preferably the predefined temperature corresponds to the temperature of the reaction mixture in the reaction vessel, thus preferably corresponds to the desired reaction temperature. The metal cooling surface may be made of any known metal heat exchange material. In further preferred embodiments the washing solvent delivery line may also contact the metal cooling surface at a preset temperature.

In preferred embodiments the pre-cooling device may comprise a thermoelectric contact-cooling device such as a Peltier cooler. The cooling effect provided by a Peltier cooler is based on the well-known Peltier effect. The Peltier cooler is adapted to provide the means to reach the preset temperature. Thus, the Peltier cooler may be in thermal communication with the metal cooling surface.

In preferred embodiments the thermoelectric contact-cooling device is assisted by a line of coolant fluid, such as for example cooling water, preferably at a temperature of 15°C to cool down the warm side of the thermoelectric contact-cooling device. The cooling water line could be provided by a central cooling system or a compact commercial cooling unit.

In preferred embodiments the pre-cooling device may also provide active cooling to the reaction vessel. In other words the pre-cooling device may be a part or component of the cooling device. In such embodiments the pre-cooling device may provide active cooling to the cooling jacket surrounding the reaction vessel. Thus, the pre-cooling device may be adapted to provide active cooling to the cooling circuit comprising the cooling jacket, cooling circuit pump and the coolant fluid reservoir. In such embodiments the cooling device for cooling the reaction vessel and the pre-cooling device for pre-cooling the reagents to be supplied are adjusted simultaneously to the same preset temperature. Thus, based on the preset temperature for cooling of the reaction vessel the reagents to be supplied will be pre-cooled to the temperature of the reaction vessel.

In embodiments of the inventive synthesizer comprising a pre-cooling device, the reagent delivery system may be connected to the pre-cooling device. With other words, the reagent delivery system may be connected to a pre-cooling device for pre-cooling the reagents to be supplied. The reagent delivery system device may be adapted for receiving reagents from the reagent storing device and may be further adapted for delivery of the received reagents to a pre-cooling device for pre-cooling the reagents to be supplied and may be further adapted for delivery of the pre-cooled reagents to the microwave transparent reaction vessel. Thus, in preferred embodiments the reagent delivery system may be adapted for receiving one or more reagents such as building blocks and activators and/or one or more reagent solutions such as building block solutions and activator solutions and/or one or more washing solutions and/or one or more deprotection solutions from a reagent storing component or one or more reagent storing components and may be further adapted for delivery of the received one or more reagents and/or one or more reagent solutions and/or one or more washing solution and/or one or more deprotection solutions to a pre-cooling device for pre-cooling the reagents to be supplied and may be further adapted to deliver the pre-cooled one or more reagents and/or one or more reagent solutions and/or one or more washing solutions and/or one or more deprotection solutions to the reaction vessel.

In embodiments of the inventive synthesizer comprising a pre-cooling device, reagent delivery system may be adapted for delivery of one or more of the reagents and/or reagents solutions and/or washing solutions and/or deprotection solution to the pre-cooling device for pre-cooling the reagents to be supplied. In such embodiments it is particularly preferred that at least the building blocks and the activators are delivered to the pre-cooling device for pre-cooling the reagents to be supplied. In further preferred embodiments the reagent delivery system may also be adapted for delivery of the washing solutions or washing solvents to the pre-cooling device for pre-cooling the reagents to be supplied.

In embodiments of the inventive synthesizer comprising a pre-cooling device, the reagent delivery system may be in thermal communication with a pre-cooling device for pre-cooling the reagents to be supplied. In further preferred embodiments of the present invention one or more delivery lines connecting the reagent delivery system and the reaction vessel may be in thermal communication with a pre-cooling device for pre-cooling the reagents to be supplied. Thus, in such embodiments the reagent delivery system may be adapted for delivery of one or more reagents to the reaction vessel, the reagent delivery system in thermal communication with a pre-cooling device for pre-cooling the reagents to be supplied, wherein the one or more reagents are pre-cooled by the pre-cooling device before entering the reaction vessel, and preferably wherein the one or more reagents are pre-cooled through contact cooling by the pre-cooling device for pre-cooling the reagents to be supplied.

In some embodiments of the inventive synthesizer comprising a pre-cooling device, the reagent delivery system may be interposed between a pre-cooling device and the reagent storing component. In such embodiments the reagent delivery system is connected to the reagent storing component and is further connected to a pre-cooling device. It is particularly preferred that the reagent storing component and the pre-cooling device are not directly connected to each other. In other words it is preferred that the reagent storing component and the pre-cooling device are connected through the reagent delivery system. It is further preferred that the pre-cooling device for pre-cooling the reagents to be supplied connects the reagent delivery system with the microwave transparent reaction vessel. Thus, in some embodiments the microwave transparent reaction vessel, the pre-cooling device, the reagent delivery system and the reagent storing device are successively connected in the following sequence: reaction vessel - pre-cooling device - reagent delivery system - reagent storing device.

In some embodiments of the inventive synthesizer comprising a pre-cooling device and a reagent delivery system comprising a first reagent distribution component and a second reagent distribution component, it is particularly preferred that the first distribution component or the top distribution component is connected to the pre-cooling device for pre-cooling the reagents to be supplied for delivery of pre-cooled building blocks or building block solutions and activators or activator solutions to the microwave transparent reaction vessel. In such embodiments the first distribution component or the top distribution component may also deliver the washing solvents or washing solution to the pre-cooling device and thereafter may supply pre-cooled washing solvents or washing solution to the microwave transparent reaction vessel. The top distribution component may be also adapted to provide a ventilation exit for gases.

In some embodiments of the inventive synthesizer comprising a pre-cooling device, the components of the reagent delivery system, particularly, building block distribution component, activator distribution component and the washing solvent distribution component are adapted to deliver the respective building blocks, activators and washing solvents to the pre-cooling device for pre-cooling the reagents to be supplied and are further adapted to deliver the respective pre-cooled building blocks, pre-cooled activators and pre-cooled washing solvents to the microwave transparent reaction vessel.

Therefore the present invention further relates to a synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
wherein the microwave transparent reaction vessel is a flow reactor.

In one embodiment, the synthesizer for microwave-assisted automated multistep synthesis on a solid support comprises:
(a) a microwave transparent reaction vessel equipped with a temperature sensor,
(b) a microwave generator component,
(c) a reagent storing component,
(d) a reagent delivery system,
(e1) a cooling device for cooling the microwave transparent reaction vessel,
(e2) a pre-cooling device for pre-cooling the reagents and/or washing solutions to be supplied,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
wherein the microwave transparent reaction vessel is a flow reactor.

The reagent delivery system may be connected to the microwave transparent flow reaction vessel through one or more inlets of the microwave transparent flow reaction vessel for delivery of the reagents and/or reagents solution and/or solvents in a continuous flow through the microwave transparent flow reaction vessel. The reagent delivery system may be connected to the microwave transparent flow reaction vessel through one or more reagent delivery lines. Thus, the reagent delivery system may be adapted to deliver reagents and/or reagent solutions and/or solutions and/or solvents in a continuous flow through the flow reaction vessel and may be further adapted to supply the reagents and/or reagent solution and/or solutions or solvents through the one or more inlets of the flow reaction vessel. Preferably the reagent delivery system may be adapted to deliver one or more reagents and/or one or more reagent solutions and/or one or more solutions in different amounts, at different points in time and in a specific sequence or specific order, and at a specific flow rate or different flow rates and further through specific inlets of the one or more inlets of the microwave transparent flow reaction vessel during the synthesis cycles of the automated synthesis of oligo-and polysaccharides on a solid support. It is therefore preferred that the reagent delivery system comprises suitable technical means, preferably suitable technical means electronically coupled to a computing device comprising at least one processor, for delivery of one or more reagents and/or one or more reagent solutions and/or one or more solutions in different amounts, at different points in time and in a specific sequence or specific order and at a specific flow rate or at different flow rates during the synthesis cycles of the automated synthesis of oligo-and polysaccharides on solid support. The reagent delivery system may comprise a pump system or one or more pumps such as syringe pumps, peristaltic pumps or other suitable pumps and may further comprise one or more valves or one or more valve assemblies, one or more manifolds, one or more distributing components and similar suitable technical means. It is preferred that the pump system or the one or more pumps, the one or more valves or the one or more valve assemblies, the one or more manifolds and/or the one or more distributing components and similar technical means are under control of a computing device comprising at least one processor.

### Synthesizer with gas valve manifold

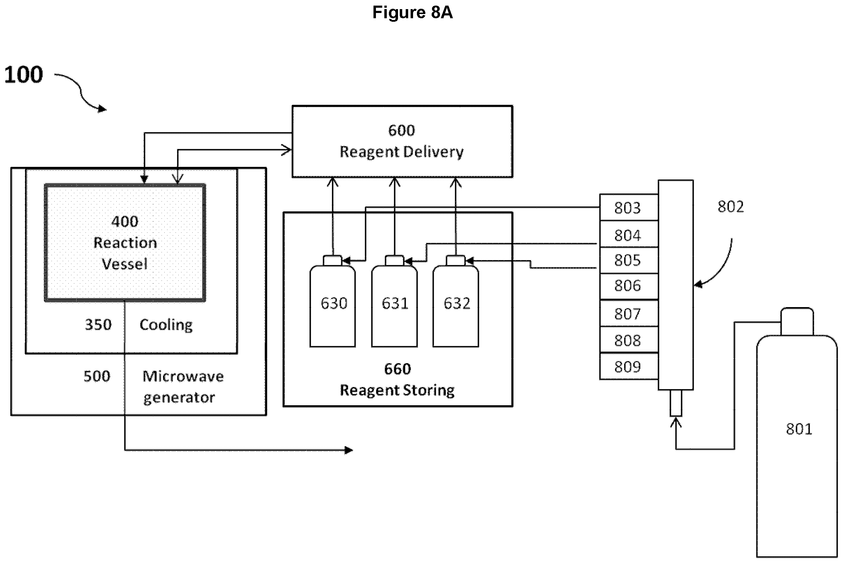

Preferably, the method of the present invention may be performed in an automated synthesizer (100) comprising a reaction vessel (400), a reagent storing component (660) comprising one or more reagent containers, a reagent delivery system (600), a cooling device (350) for cooling the reaction vessel and an inert gas delivery system (800) comprising a gas container and a gas valve manifold, (see **Figure 8**).

Thus, a further aspect of the present invention is directed to a synthesizer for automated multistep synthesis on a solid support comprising:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

The synthesizer according to the present invention is particularly suitable for automated multi-step synthesis on solid support, wherein multiple incompatible reagents are used that should not get into contact with each other within the synthesizer, except in the reaction vessel. Thus, the inventive synthesizer enables separation of incompatible reagents such as acids and bases, which may produce salts when combined. Over time these salts may build up ubiquitously in the synthesizer and block tubings and valves that may corrode sensitive components of the synthesizer, which may lead to costly repairs. It is therefore necessary that the solutions to remove the temporary protecting groups (for example protecting groups such as Fmoc, Lev, ClAc, and Nap) and acetyl capping solutions are delivered via separate fluid communications to the reaction vessel. A separate fluid communication is achieved by using an inert gas delivery system for driving the reagents to the reaction vessel comprising a gas container and a gas valve manifold, wherein the gas valve manifold establishes fluid communication between the gas container and each reaction container in such a way that at least two fluid communications are separate. Using separate fluid communications between the inert gas delivery system and the reagent containers as well as between the reagent containers and the reaction vessel allows delivery or transfer of the reagents to the reaction vessel without mixing of vapor phases of volatile incompatible substances, such as piperidine and organic solvents which often contain acidic impurities such as chloroform or methylene chloride.

In a preferred embodiment of the invention, the gas valve manifold is in a separate fluid communication with each of the one or more reagent containers and each of the one or more reagent containers is in a separate fluid communication with reaction vessel. Thus, in one embodiment the synthesizer for automated multistep synthesis on a solid support comprises:
(a) a reaction vessel,
(c) a reagent storing component comprising one or more reagent containers,
(d) a reagent delivery system configured to establish a separate fluid communication between each of the one or more reagent containers and the reaction vessel,
(e) a cooling device for cooling the reaction vessel,
(g) an inert gas delivery system comprising a gas container and a gas valve manifold establishing a separate fluid communication between each of the one or more reagent containers and the gas container.

The synthesizer of the present invention comprises an **inert gas delivery system** (see **Figure 8A**) to provide anhydrous and inert gas to one or more components of the synthesizer of the present invention for mixing, atmosphere conditioning and driving fluid purposes. Preferably the synthesizer is adapted for performing reactions under anhydrous and inert atmosphere. The gas delivery system **800** comprises at least one gas container, such as an argon container **801,** and a gas valve manifold **802** to provide inert gas to the one or more components of the inventive synthesizer. The gas delivery system may further comprise one or more valves, valve assemblies, one or more vents and/or one or more additional manifolds or similar technical means (**803-809**) to allow the distribution and delivery of inert gas to the one or more components of the synthesizer of the present invention. The gas valve manifold, one or more valves, valve assemblies, one or more vents and/or one or more additional manifolds or similar technical means allow control of the pressure within the one or more lines which connect the one or more components of the synthesizer. The gas valve manifold, one or more valves, valve assemblies, one or more vents and/or one or more additional manifolds or similar technical means may be under control of a computing device comprising at least one processor. The processor may be configured to control the delivery and distribution of the inert gas within the inventive synthesizer and is configured to control gas pressure within the one or more lines and one or more components of the synthesizer of the present invention.

According to the present invention the synthesizer comprises a **reaction vessel.** All reactions steps of the multi-step synthesis, including coupling reactions, appropriate deprotection, and optionally capping steps are preferably performed inside of the reaction vessel of the inventive synthesizer. The coupling cycles are repeated as often as necessary to achieve the desired length of the desired oligo-, polysaccharide or peptide.

The reaction vessel may be made of any material known in the art which is chemically and physically compatible with the reagents used and reaction conditions applied in the automated multi-step synthesis of oligo-, polysaccharides and peptides on a solid support. Examples of reaction vessel materials include, but are not limited to, glass, quartz, PTFE (Teflon), polypropylene. In preferred embodiments the reaction vessel is microwave transparent. In preferred embodiments the reaction vessel is interchangeable. In preferred embodiments the reaction vessel is made of fluoropolymers such as PFA for improved chemical resistance and ease of swapping reactions vessels of different sizes. In preferred embodiments the reaction vessel is made of glass.

The reaction vessel of the synthesizer according to the present invention can be equipped with a temperature sensor. The temperature sensor can be any type of temperature sensor commonly known in the art that is suitable for probing the temperature range from T₁ to T₃ and that is stable in presence of the reaction mixture, building blocks and reagents used in the multi-step synthesis. Suitable temperature sensors are for example, but not limited to, negative temperature coefficient (NTC) thermistor, resistance temperature detector, thermocouple, infrared sensor or fiber optic temperature probe. Preferably, the temperature sensor is a fiber optic temperature probe.

The reaction vessel of the present invention is adapted for holding a reaction mixture. The reaction vessel therefore comprises an inner cavity or effective loading space for performing one or more synthesis steps of the synthesis cycles. The reaction vessel is adapted to enable performance of one or more synthesis steps or even all of the synthesis steps of the synthesis cycles in an isolated, anhydrous and inert atmosphere. These steps may include, but are not limited to, coupling steps, washing steps, deprotection steps, capping steps and decoupling from the solid phase resin. The solid support or solid phase resin may be initially loaded into the reaction vessel. With other words the solid support or solid phase resin may be loaded into the inner cavity or effective loading space of the reaction vessel. The loading of the reaction vessel with the solid phase resin may be performed as part of a pre-automation process but may also be part of the automation process. Thus, the loading with the solid support may be also performed in an automated manner. The solid support may be provided in form of insoluble resin bead(s) and is preferably a functionalized resin suitable for oligo-and polysaccharide synthesis on a solid support, such as a resin equipped with an appropriate linker or in particular a polystyrene-based resin functionalized with an appropriate linker. Several suitable functionalized solid phase resins for oligo- or polysaccharide synthesis on a solid support have been described in the prior art. Examples of functionalized solid phase resins suitable for use with the synthesizer of the present invention are described further below.

Thus, it is preferred that the reaction vessel is adapted for receiving a solid support. Therefore, the reaction vessel preferably comprises an inner chamber or effective loading space for performing one or more synthesis steps of the synthesis of oligo-, polysaccharides or peptides on a solid support, preferably in an isolated, anhydrous and inert atmosphere. It is preferred that the inner cavity or effective loading space holds between 1 mL and 100 mL solvent, more preferably 5 - 20 mL solvent. It is preferred that the inner cavity or the effective loading space is sized to accommodate the solid support, reagents and solvent.

Therefore the present invention further relates to a synthesizer for automated multistep synthesis on a solid support comprising:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other,
wherein the reaction vessel is adapted for receiving the solid support.

The reaction vessel therefore comprises an inner cavity or effective loading space for performing one or more synthesis steps of the coupling cycles to obtain the desired oligo- or polysaccharide. The reaction vessel may be one of a batch reaction vessel or semi-batch reaction vessel or a flow reactor. The term flow reactor as used herein refers preferably to a continuous flow reactor for performing the synthesis in a continuous manner.

For performing the coupling reactions appropriate building blocks and activators are supplied or added to the reaction vessel. Preferably, the building blocks and activators are supplied or added to the reaction vessel already containing the solid support or solid phase resin. The coupling reactions preferably take place inside the reaction vessel followed by appropriate deprotection, capping and/or washing steps. All of these synthesis steps and treatments in the synthesis cycles are preferably performed inside of the reaction vessel of the device of the present invention. The synthesis cycles are repeated as often as necessary to achieve the desired length of the desired oligo- or polysaccharide. The synthesis cycle is preferably repeated at least three times, more preferably at least four times or more preferably more than four times for obtaining an oligo- or polysaccharide.

For supplying liquids, solutions and/or gases the reaction vessel of the inventive synthesizer may further comprise two or more inlets, for example, for supplying washing solvents or washing solutions, building blocks or building block solutions, activators or activator solutions, capping solutions, deprotection solutions and inert gas, and may further comprise one or more outlets, for example, for discharging the liquids or solutions after each chemical reaction and/or also ventilation exits for inert gas or other gases from the reaction mixture.

In preferred embodiments of the present invention the two or more inlets may also function as one or more outlets, that means one or more inlets for supplying reagents, solvents, reaction mixtures, inert gas and the like can also be used to remove a solution, inert gas, reaction components and the like from the device for automated synthesis. In such embodiments one or more technical means such as one or more valves, valve assemblies, one or more vents, one or more manifolds and/or similar technical means may be located and mounted upstream to the one or more inlets of the reaction vessel. For example, by implementing a valve assembly or one or more valves in the device of the present invention the delivery of liquids, solutions and/or gases and also the discharging of liquids, solutions and/or gases after or during each chemical reaction or treatment may be realized through one inlet of the reaction vessel only or through two, three or more inlets of the reaction vessel. By implementation of technical means such as valves, valve assemblies, vents, manifolds or similar technical means, the total number of in-and outlets of the reaction vessel may be reduced.

Therefore the present invention further relates to a synthesizer for automated multistep synthesis on a solid support comprising:
(a) a reaction vessel comprising at least one inlet at the top of the reaction vessel and at least one inlet at the bottom of the reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

In one embodiment, the inventive synthesizer for automated multistep synthesis on a solid support comprises:
(a) a reaction vessel comprising at least one inlet at the top of the reaction vessel and at least one inlet at the bottom of the reaction vessel,
(c) a reagent storing component comprising one or more reagent containers,
(d) a reagent delivery system configured to establish a separate fluid communication between each of the one or more reagent containers and the reaction vessel,
(e) a cooling device for cooling the reaction vessel,
(g) an inert gas delivery system comprising a gas container and a gas valve manifold establishing a separate fluid communication between each of the one or more reagent containers and the gas container.

The inventive synthesizer may further comprise a **microwave generator component.** Microwave-assisted organic chemical synthesis refers to the use of electromagnetic radiation within the microwave frequencies to provide the energy required to initiate, drive or accelerate certain chemical reactions. Several multimode or mono-mode / single-mode microwave generator components for microwave-assisted organic synthesis are known in the art, for example, in multimode instruments microwaves are reflected by the walls of a relatively large cavity, which may generate pockets of high and low energy as the moving waves either reinforce (hot spots) or cancel out each other (cold spots) leading to a nonhomogeneous microwave field in the cavity. In order to provide a homogeneous field, multimode instruments may be equipped with a mechanical mode stirrer inside the cavity. In mono-mode cavities a standing wave may be created when the electromagnetic irradiation is passed by a waveguide that directs the microwaves directly through a reaction vessel that is positioned in a fixed distance from the radiation source or by generating travelling waves where the microwaves make a single pass through the reaction vessel, are reflected off the cavity wall and pass through the reaction vessel a second time. Multimode microwave components enable performance of parallel synthesis, whereas mono-mode microwave components provide a high degree of reproducibility due to a very precise heating. The microwave generator component may be one of a magnetron, klystron and solid state devices. A solid-state microwave generator component uses a semiconductor transistor which generates single, defined microwave frequencies in a controlled manner.

Thus, in preferred embodiments of the present invention the synthesizer may further comprise a microwave generator component, preferably a mono-mode / single-mode microwave generator component. The microwave generator component may further comprise a waveguide. The microwave generator component preferably comprises a chamber for insertion and fixing of the reaction vessel to provide microwave radiation to the reaction vessel.

Therefore the present invention further relates to a synthesizer for automated multistep synthesis on a solid support comprising:
(a) a microwave transparent reaction vessel,
(b) a microwave generator component,
(c) a reagent storing component comprising one or more reagent containers,
(d) a reagent delivery system configured to establish a fluid communication between each of the one or more reagent containers and the microwave transparent reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

The microwave transparent reaction vessel may be constructed as a double-wall structure which forms two cavities, wherein the first cavity accommodates the multi-step synthesis of oligo-, polysaccharides or peptides and wherein the second cavity accommodates a microwave transparent coolant fluid. The double-wall structure of the microwave transparent reaction vessel may be made of glass. Thus, the microwave transparent reaction vessel may be provided with a cooling jacket for cooling the reaction vessel. Preferably, the cooling jacket may be made of a microwave transparent material such as glass, quartz, PTFE (Teflon), polypropylene or fluoropolymers. In preferred embodiments of the present invention the reaction vessel may be provided as an interchangeable microwave transparent reaction vessel. In such embodiments it is preferred that the microwave transparent reaction vessel may be removed from a cooling means such as a cooling jacket without interrupting the flow of a circulating microwave transparent coolant fluid. In such embodiments the reaction vessel may be removed for example for loading with solid support or for discharging the solid support. Furthermore such an interchangeable reaction vessel allows use of various reaction vessels with different sizes for different batch sizes.

In comparison to conductive heating microwave heating (dielectric heating at 2.45 GHz) occurs by disposing the energy directly to the solvent and some reagents, due to interactions of the solvents and reagents with the alternating electric field. Material interacts with the electromagnetic field differently, i.e. materials store and convert the energy to heat to different extents, which have huge impact on their ability to be heated by microwaves. Besides the solvent, several other factors influence the dielectric properties, such as sample volume, vessel material, and the mode of stirring. The application of heat energy (thermal transfer) is one of the most significant factors in increasing the rate of a wide variety of chemical reactions. Microwave-assisted reactions, however, transfer energy to chemical reactions in a different, much faster manner than the conductive devices. Microwave energy directly interacts with polar or ionic molecules. This, effect, known as dipole rotation, is a result of the polar or ionic molecules trying to align themselves with the rapidly changing electric field of the microwaves. The rotational movement of molecules as they try to orient themselves with the electric field creates localized superheating and generates thermal energy as a by-product.

Thermal energy may be raised beyond a critical point for a given reaction, resulting in excess thermal energy. Excess thermal energy increases the temperature of a reaction mixture. This excess thermal energy may have detrimental effects on heat-sensitive reactions or compositions. Excess thermal energy can drive side reactions that degrade the reactants, catalysts and desired product of the desired reaction. Furthermore, some products may be unstable at room temperature. Therefore it is advantageous to maintain a low bulk reaction mixture temperature. The beneficial effects of simultaneous cooling during microwave irradiation have been described in the art to be useful for chemical synthesis with solid phase supports.

The inventors of the present invention have found that the utilization of a microwave generator component is not only instrumental for hastening the cooling to heating process, microwave-assisted synthesis has also been shown to drastically reduce chemical reaction time. During a typical AGA cycle there is the glycosylation coupling step as well as several auxiliary steps (acetyl capping and temporary group deprotection). These auxiliary steps increase the overall yield of the final glycan by terminating unglycosylated nucleophiles as well as they remove temporary protecting groups that allows the next coupling to occur. These auxiliary steps have been a bottleneck in the overall time required for one AGA cycle and the use of microwave radiation drastically reduces the reaction time. Under these rapid microwave-assisted conditions, the steps remained orthogonal and few side reactions were observed. With shortened reaction times for these auxiliary steps, the overall duration of a standard AGA cycle could be successfully reduced from 100 -130 minutes to below 70 minutes and even to 45 minutes. Thus, the auxiliary steps carried out in the automated saccharide synthesis benefit from the use of microwave radiation.

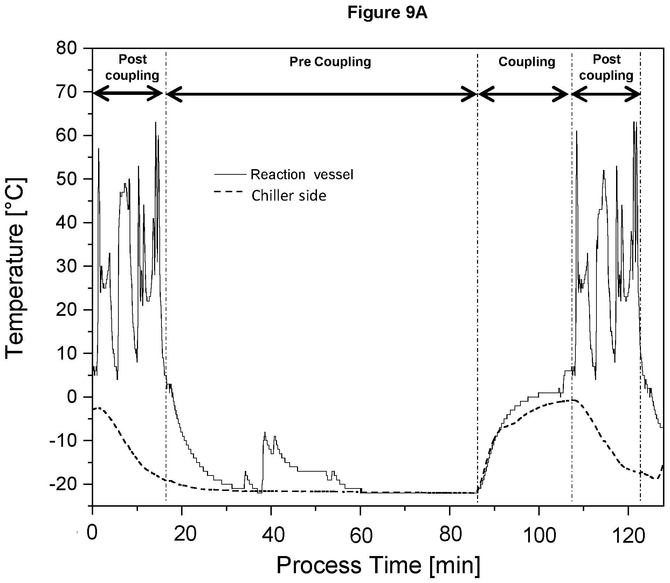

**FIG. 9A** shows the temperature profiles (for detail see Example 3) of the reaction vessel (in solid line) and the chiller (in segmented line; measured at the tubing side) for a complete cycle (Pre-coupling, coupling and post-coupling) during the synthesis of mannose tetrameter via phosphate donor. In addition, the post-coupling step from the previous cycle is shown, too. The experiment was performed without precooling. In this implementation the chiller provides the active cooling to the reaction vessel by fluid communication with a jacket. The chiller adjusts **T1** (pre-coupling temperature) and **T2** (coupling temperature). During the post coupling the microwave irradiation heats up the reagent and washing solvents during the capping a deprotection modules. Simultaneously the chiller adjusts the temperature for the next coupling.

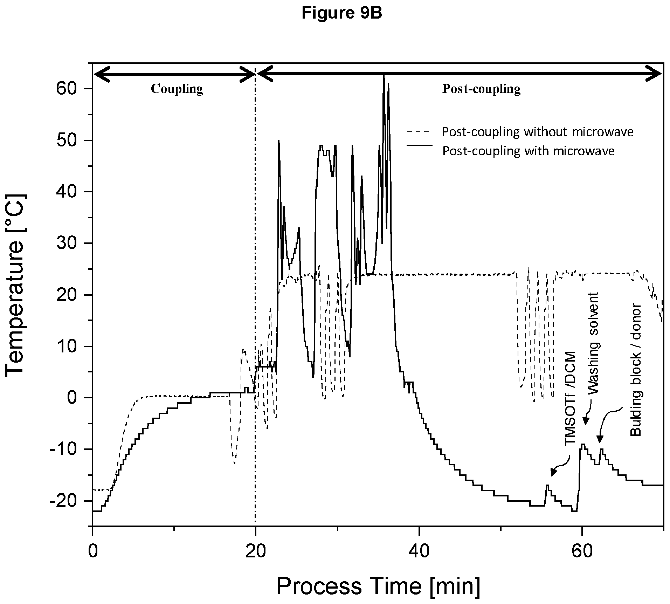

**FIG. 9B** compares the temperature profiles during post-coupling reactions assisted by microwave radiation (in solid line, Example 3, **FIG. 9A**). In segmented line the temperature profiles during post-coupling with conventional temperature regulation by heat exchanger with the jacket/coil surrounding the reaction vessel (**FIG 6**). A dramatic time reduction is shown, the conventional post-coupling reaction (capping and deprotection) takes from 50 min, while the microwave assisted post coupling reaction modules is completed in 15.

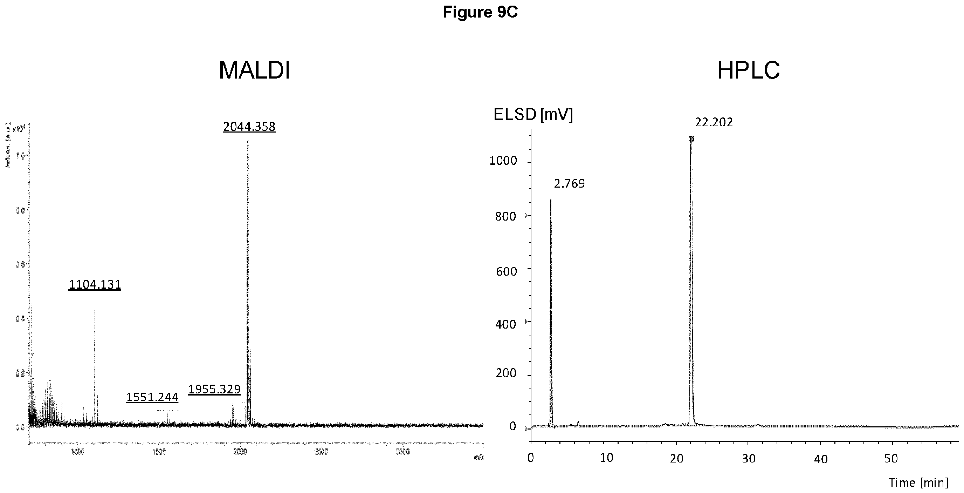

**FIG. 9C** shows the analytical results of the experiments described in **FIG. 9A****.** Mass spectra (MALDI) and HPLC chromatogram are provided. The experiment performed under simultaneous microwave heating and active cooling during the post-coupling step shows mainly the desired product by HPLC. Barely traces of deletion sequences have been observed.

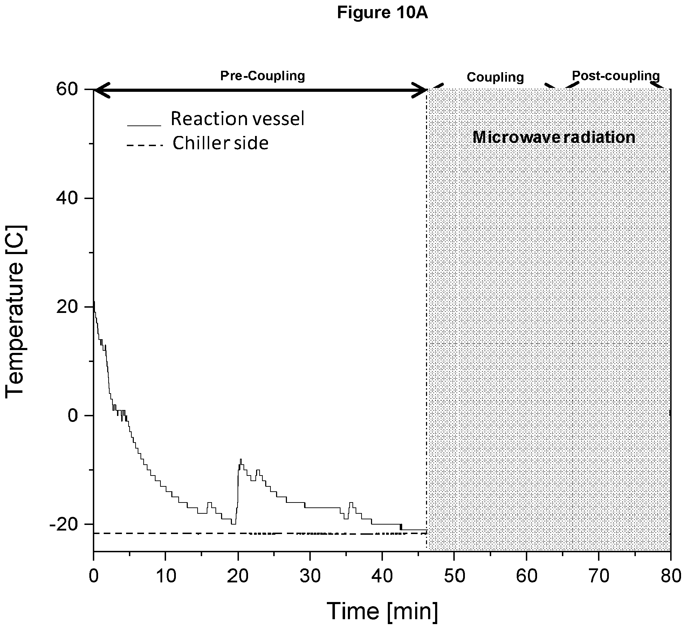

**FIG. 10A** shows the temperature profiles (Example 4) of the reaction vessel (in solid line) and the chiller (in segmented line; measured at the tubing side) for a complete cycle (pre-coupling, coupling and post-coupling) during the synthesis of mannose tetrameter via phosphate donor. The experiment was performed without precooling. In this implementation the chiller provides the active cooling to the reaction vessel temperature in fluid communication with a jacket. The chiller provides constant cooling action, working at constant temperature **T1** (pre-coupling temperature). The coupling temperature **T2** is adjusted by microwave irradiation. Later, the post coupling temperature **T3** is reached by microwave irradiation too. The microwave radiation heats up the reagent and washing solvents during the capping a deprotection modules. The microwave irradiation occurs while the chiller provides constant cooling (**T1**). The inventor shows the improvement within cycle duration by combining constant cooling simultaneously with microwave radiation to adjust the temperature in the reaction vessel (**T2** and **T3**). The conventional cycle (**FIG 6**) takes 130 min, while the cycle with temperature control assisted by microwave takes less than 80 min.

By combining active cooling and microwave heating the temperature adjustment rate is improved in both direction of the thermal gradient (from lower to higher temperatures and vice versa).

With simultaneous active cooling and microwave heating the temperature the energy consumption of the chiller is highly optimized. As a consequence; a less powerful chiller is required in this setup since the chiller needs to absorb less heat to adjust and/or maintain the temperature.

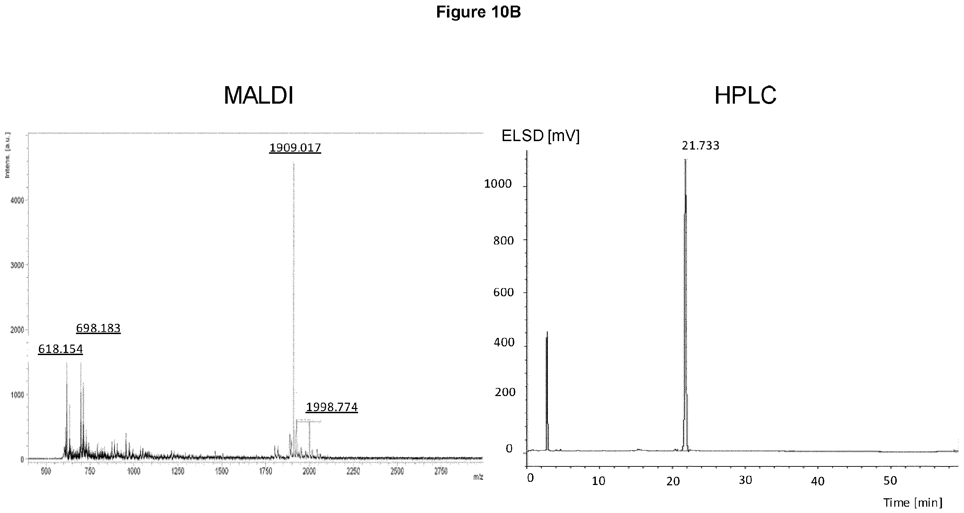

**FIG. 10B** shows the analytical results of the experiments described in **FIG. 10A****.** Mass spectrometry (MALDI) and HPLC chromatogram are provided. The experiment performed under simultaneous microwave heating and active cooling during the coupling and post-coupling steps show as result: mainly the desired product was obtained.

In one embodiment of the inventive synthesizer having a microwave generator component, the synthesizer further comprises a **thermal controller** for controlling the temperature inside the reaction vessel. Thus, the present invention is also directed to a synthesizer for automated multistep synthesis on a solid support comprising:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

A typical coupling cycle in a glycan synthesis comprises a glycosylation step and several protecting group manipulations ("auxiliary steps") including a capping reaction in order to terminate unreacted saccharides, deprotection of a temporary protecting group and washing of the solid support. These steps of each coupling cycle are conducted in three different three temperature regimes or stages (as described above).

The first stage relates to the pre-coupling regime in the temperature range T₁ of preferably -40 °C to -10 °C. In this pre-coupling regime a further saccharide bearing at least one protecting group and a glycosylation reagent are allowed to impregnate the solid support (e.g. a resin) and to diffuse through the porous solid. The low temperature in the range of preferably -40°C to -10°C prevents the early decomposition of the intermediate before the actual coupling reaction.

The second stage relates to the coupling regime at a temperature T₂ around 0 °C, i.e. -10 °C and +5 °C. The increase in the temperature to preferably around 0 °C allows the initiation of the coupling reaction by promoting the formation of the intermediates. Thus, during the coupling regime, the glycosylation reaction takes place.

The third stage relates to the post-coupling regime at a temperature T₃ around 25 °C (room temperature or standard temperature) or above, up to 65 °C. The high reaction temperature T₃ is achieved by microwave irradiation. Thus, the capping and deprotection reactions take place under microwave irradiation and at higher temperatures than the coupling reaction. These reactions complete the coupling cycle. Then, the next coupling cycle or termination of the process may take place.

As can be seen in Figure 7, the temperature must be adjusted between each coupling cycle from high temperature T₃ to the lowest temperature T₁, which requires usually more than 20 minutes (see left part of Figure 7). By using a thermal controller, which is connected to the cooling device, the temperature sensor and the microwave generator component, the reaction temperature can be adjusted more rapidly, so that less time is required between each coupling cycle (see right part of Figure 7), thereby allowing a more rapid automated multistep synthesis.

Particularly, the thermal controller is connected to the temperature sensor in the microwave transparent reaction vessel and the microwave generator component thereby allowing the reaction mixture to reach the reaction temperature of T₃ in the post-coupling regime. The thermal controller adjusts the power output of the microwave generator component based on the measured temperature inside the reaction vessel in order to maintain the temperature T₃. Also, the thermal controller is connected to the temperature sensor in the microwave transparent reaction vessel and the microwave generator component thereby allowing the reaction mixture to warm up more rapidly from a reaction temperature of T₁ to T₂ during the coupling regime.

The thermal controller is also connected to the cooling device thereby allowing setting a fixed cooling temperature T₁ during pre-coupling regime and/or T₂ during the coupling and post-coupling regime. The microwave generator component is usually turned off by the thermal controller during pre-coupling and coupling regime and only turned on during the post-coupling regime. However, in one embodiment the microwave generator component is already turned on by the thermal controller during coupling regime in order to speed up the warming from T₁ to T₂.

Thus, the thermal controller connected to a cooling device and a microwave generator component allows the use of the microwave generator component as a heater, so that no additional heater is required for performing the auxiliary steps at elevated temperatures. Therefore, due to the thermal controller a simpler and more compact setup of the synthesizer is achieved as no additional cooler or heater are necessary for performing the multi-step reactions at different temperature regimes as set forth above.

Furthermore, technical means such as valves, valve assemblies, vents and manifolds and similar technical means may be electronically coupled to a **computing device** comprising at least one processor and a computer-readable storage medium comprising computer readable instructions thereon that may be executed by the processor, therefore the technical means may be under control of such a computing device. The processor may be configured to control the temperature setting of the pre-cooling device, may be configured to control the temperature setting of the cooling device, may be configured to set the time and power output of the microwave generator component, may be configured to control the amount and speed of delivering fluid from the reagent delivery component. The synthesizer preferably comprises one or more or a plurality of technical means electronically coupled to the computing device comprising at least one processor to allow the processor to control the one or more or plurality of technical means based on computer readable instructions stored on a computer readable medium which may be executed by the processor to enable the automation of the method for synthesizing oligo-, polysaccharides or peptides with the inventive synthesizer. The one or more technical means or plurality of technical means may be wired or wirelessly connected to the computing device. The one or more technical means or plurality of technical means may comprise one or more valves, valve assemblies, one or more vents and similar technical means of the one or more components and systems of the synthesizer of the present invention. Thus, the delivery of the liquids, solutions and/or gases and also the discharging of liquids and solutions may be under control of the computing device comprising the at least one processor and a computer-readable storage medium comprising computer readable instructions thereon. The computing device comprising the at least one processor and a computer-readable storage medium comprising computer readable instructions thereon may be configured to control said technical means in order to deliver liquids, solutions and/or gases to the reaction vessel and further to discharge liquids and solutions from the reaction vessel in a controlled and automated manner to allow automation of the synthesis on a solid support with the synthesizer of the present invention.

The reaction vessel may further comprise one or more openings or apertures for insertion of various other components or technical means. For example, the reaction vessel may further comprise an opening or aperture for insertion of a temperature probe or a temperature sensor for monitoring and measuring the temperature inside of the reaction vessel. Monitoring and measuring the temperature inside of the reaction vessel thereby also means monitoring and measuring the temperature of the reaction mixture during one or more or even all synthesis steps of the synthesis cycles. Thus, it is preferred that the reaction vessel further comprises at least one opening or aperture for insertion of a temperature sensor for monitoring and measuring the temperature inside of the reaction vessel.

The one or more inlets of the reaction vessel may be located at various positions of the reaction vessel body. In preferred embodiments of the present invention the reaction vessel may comprise one or more inlets at the top of the reaction vessel, for example, for delivery of washing solvents or washing solutions, building blocks or building block solutions, and activators or activator solutions and may further comprise one or more outlets at the top of the reaction vessel, for example, for exits for inert gas or other gases and may further comprise one or more inlets at the bottom of the reaction vessel, for example, for delivery of capping solutions, deprotection solutions and inert gas and may further comprise one or more outlets for discharging the liquids or solutions after each chemical reaction or after each washing step. As mentioned above, by implementing further technical means such as one or more valves, valve assemblies or similar technical means, these one or more inlets may also function as outlets for the liquids, solution and/or gases.

Thus, the reaction vessel of the inventive synthesizer comprises at least two inlets, wherein the at least two inlets are located at different compartments of the reaction vessel in order to avoid premature mixing of incompatible reagents. Preferably the reaction vessel comprises one or more inlets at the top of the reaction vessel and one or more inlets at the bottom of the reaction vessel to enable at least two separate delivery methods for solvents and reagents, one from the top and another from the bottom of the reaction vessel. The usage of two different ports allows for the separation of incompatible reagents such as acids and bases, which may produce salts when combined. Over time these salts may build up and block tubing that may corrode components of the device of the present invention, which may lead to costly repairs. It is therefore preferred that the solutions to remove the temporary protecting groups (for example protecting groups such as Fmoc, Lev, ClAc, and NAP) and acetyl capping solutions are delivered via the one or more bottom inlets or bottom port of the reaction vessel. These reactions are desired to be performed rapidly and solutions are provided in excess, so these solutions will be pushed into the reaction vessel for example by pressurized inert gas such as argon. The inert gas inlet may serve for a dual purpose to not only deliver the reagents, but to efficiently mix the reaction. In addition, the one or more bottom inlets may serve to remove all the reagents and solutions from the reaction vessel. Oppositely, the activators or activator solutions and the building blocks or building block solutions are preferably delivered through the one or more top inlets or top port of the reaction vessel. These reagents are more valuable and are required to be delivered in a controlled stoichiometry, so these are delivered preferably dropwise, for example, via a syringe pump system.

In preferred embodiments of the present invention the reaction vessel may comprise only one inlet at the bottom of the reaction vessel, for example, for delivery of capping solutions, deprotection solutions and inert gas and for discharging the liquids or solutions after each chemical reaction or after each washing step and one or more inlets at the top of the reaction vessel, for example, for delivery of washing solvents or washing solutions, building blocks or building block solutions, and activators or activator solutions. The reaction vessel may further comprise openings or apertures at the top of the reaction vessel for insertion of, for example, a temperature probe or a temperature sensor. The reaction may further comprise an exhaust gases exit at the top of the reaction vessel.

In preferred embodiments of the present invention the reaction vessel may comprise at least three inlets/outlets, preferably four inlets/outlets (601, 602, 645, 655) at the top of the reaction vessel, for example, one inlet for delivery of washing solvents or washing solutions, one inlet for delivery of building blocks or building block solutions and one inlet for delivery of activators or activator solutions and preferably a further outlet for the exhaust of gases and/or mixing gas (655). The separate and distinct delivery of the building blocks and activators to the reaction vessel is advantageous for preventing a contacting of the building blocks and activators before supplying the building blocks and activators to the reaction mixture inside of the reaction vessel. It is particular preferred that the building blocks or building block solutions and the activators or activator solutions are not mixed or merged with each other before delivery to the reaction vessel. It is further advantageous that the washing solvents or washing solutions are separately supplied to the reaction vessel to allow rapid delivery of washing solvents or washing solution in a larger amount and to ensure well distribution of the washing solvents or washing solutions inside of the reaction vessel. For example, a channel for supplying the washing solvents or washing solution may be used to supply the washing solvents or washing solution to the reaction vessel. Such a channel may be adapted for spraying the washing solvents or washing solutions by splitting the liquid among a series of holes at the tip of the channel. The channel may also comprise a conical end to assure complete dropping of the washing solvents or washing solutions. Such a channel for delivery of washing solvents or washing solution allows to effectively wash-off other reagent solutions, reagents or adhered solid support from the inner walls of the reaction vessel. In contrary, the building blocks and activators are preferably supplied directly into the reaction mixture in a dropwise manner.

In embodiments of the present invention wherein the reaction vessel comprises one or more inlets at the bottom of the reaction vessel or only one inlet at the bottom of the reaction vessel it is preferred that the effective loading space or inner chamber of the reaction vessel is fenced by the bottom inlet. The bottom compartment may prevent canalization of fluid through a frit. A frit allows for dispersion of inert gas bubbling for mixing purposes. The frit may also ensure that the solid support remains inside of the reaction vessel. Thus, in preferred embodiments of the present invention the reaction vessel may further comprise a frit. It is preferred that the frit is located in the bottom compartment of the reaction vessel. Thus, to prevent solid support from being drawn from the bottom inlet, the end in the reaction vessel may be fitted with a frit or a filter.

Preferably the reaction vessel is made of a material such as glass, quartz, PTFE (Teflon), polypropylene or fluoropolymers. A reaction vessel made of glass may be provided in form of a triple-jacketed glass reaction vessel. The reaction vessel may be provided as a glass reaction vessel surrounded by a second cavity that allows for circulation of microwave transparent coolant fluid to control the temperature of the reaction mixture. Thus, the reaction vessel may be provided with a cooling jacket for cooling the reaction vessel.

In preferred embodiments of the present invention the reaction vessel may be provided as interchangeable reaction vessel. In such embodiments it is preferred that the reaction vessel may be removed from a cooling means such as a cooling jacket without interrupting the flow of a circulating microwave transparent coolant fluid.

In such embodiments the reaction vessel may be removed for example for loading with solid support or for discharging the solid support. Furthermore such an interchangeable reaction vessel allows use of various reaction vessels of different sizes for different batch sizes.

Therefore the present invention relates to a synthesizer for automated multistep synthesis on a solid support comprising:
(a) an interchangeable reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other,
wherein the reaction vessel is adapted for receiving the solid support.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other,
wherein the reaction vessel is adapted for receiving the solid support.

The inventors have surprisingly found that a reaction vessel made of fluoropolymers such as polytetrafluoroethylene (PTFE), perfluoroalkoxy alkanes (PFA), fluorinated ethylene propylene (FEP), ethylene tetrafluoroethylene (ETFE) or a reaction vessel comprising a coating of polytetrafluoroethylene (PTFE), perfluoroalkoxy alkanes (PFA), fluorinated ethylene propylene (FEP), ethylene tetrafluoroethylene (ETFE) is particularly suitable for the synthesizer of the present invention.

The inventors of the present invention have found that the change in the manufacturing of the reaction vessels from silicate glass to fluoropolymer improves chemical resistance, as well as eases of swapping reaction vessels of different sizes. Thus, it is advantageous to switch from a glass reaction vessel to one made of fluoropolymers such as perfluoroalkoxy alkanes (PFA). Improved flow, minimal resin adherence, thermal resistance, and chemical resistance are among the many properties that PFA excels over glass. Typical silicate glass contains hydroxyl groups, which creates a hydrophilic surface. During the synthesis cycles, the resin often adheres to glass walls above the reaction solution causing deletion sequences and mixtures in the final product. To prevent adhesion problems, regular silanization of the glass reactor is necessary. On the other hand, PFA reactors have better hydrophobic properties and are non-adherent to the resin. This allows for reaction vessels made from PFA materials to tolerate a wider range of chemical reactions that are incompatible with silane coating on glass. An additional advantage is the physical durability of the PFA reactor compared to glass. Typically, AGA synthesizers are utilized for high throughput synthesis and are in constant use, so durable components are preferred.

In preferred embodiments of the present invention the reaction vessel is made of perfluoroalkoxy alkanes (PFA). In further preferred embodiments of the present invention the reaction vessel comprises a coating of perfluoroalkoxy alkanes (PFA). Perfluoroalkoxy alkanes are copolymers of tetrafluoroethylene (C₂F₄) and perfluoroethers (C₂F₃OR^{f}, where R^{f} is a perfluorinated group such as trifluoromethyl (CF₃).

Therefore the present invention relates to a synthesizer for automated multistep synthesis on a solid support comprising:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other,
wherein the reaction vessel is made of perfluoroalkoxy alkanes (PFA).

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other,
wherein the reaction vessel is made of perfluoroalkoxy alkanes (PFA).

In one embodiment, the synthesizer for automated multistep synthesis on a solid support comprises:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other,
wherein the reaction vessel is made of glass.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other,
wherein the reaction vessel is made of glass.

In preferred embodiments of the present invention the reaction vessel may further comprise a cap adapted for closing the reactor vessel for protecting the inner chamber or effective loading space from the external atmosphere. Thus, a cap may be provided for closing the reaction vessel in order to provide an isolated, anhydrous and inert atmosphere inside of the reaction vessel.

According to the present invention the synthesizer comprises a **reagent storing component.** The synthesizer of the present invention may comprise one or more reagent storing components. The synthesizer may comprise at least one reagent storing component. The reagent storing component or the one or more reagent storing components are preferably adapted for storing the building blocks and/or building block solutions, activators and/or activator solutions, washing solvents and/or washing solutions, deprotection solutions and/or capping solutions. The reagent storing component or the one or more reagent storing components may comprise one or more reagent containers or may comprise a plurality of reagent containers. The reagent storing component or the one or more reagent storing components may comprise one or more reagent containers for storing building blocks, one or more reagent containers for storing building block solutions, one or more reagent containers for storing activators, one or more reagent containers for storing activator solutions, one or more reagent containers for storing washing solvents and/or washing solutions, one or more reagent containers for storing deprotection solutions and one or more reagent containers for storing capping solutions. The reagent storing component or the one or more reagent storing components are preferably adapted for storing reagents and solvents in an anhydrous and inert atmosphere. In preferred embodiments of the present invention the reagents and solvents are stored under argon pressure. In preferred embodiments the reagent storing component or the one or more reagent storing components are connected to an inert gas delivery system. The gas delivery system may provide inert gas, such as argon, to the reagent storing component or the one or more reagent storing components. The inert gas delivery system may provide inert gas, such as argon, to each of the containers of the reagent storing component or the one or more reagent storing components. Thus, in preferred embodiments of the present invention each of the containers of the reagent storing component or the one or more reagent storing components may be connected to an inert gas delivery system.

The containers may be made of any suitable chemical resistant material known in the art which is suitable for storing chemical reagents and/or solutions and furthermore suitable for storing the building blocks and/or building block solutions, activators and/or activator solutions, washing solvents or washing solutions, deprotection solutions and also capping solutions. The containers may preferably be adapted for storing the reagents in an anhydrous and inert atmosphere, for example, under argon pressure. For example, synthesis-ready building blocks may be pre-weighed and either dissolved in the appropriate anhydrous solvent or kept as solids and placed in sealed vials, for example, on a 64-position carousel or a 4-position means (e.g. test tube type containers) where a solution of building block (e.g. up to 8 mL of the appropriate concentration) is coupled to the system by multiport valves or similar technical means. Preferably a cap seals the reaction vessel which has an inlet for inert gas, such as argon, providing an anhydrous atmosphere and an outlet for the building block solution extraction. The extraction tube may reach the bottom container and may be allowed to take out the required volume of building block solutions. The building blocks are preferably those that can be synthesized in large quantities, are stable upon storage for extended periods of time, and upon activation result in very high coupling yields with excellent stereo-control. Solvents and reagents may for example be placed in various sizes of glass bottles and kept under inert gas, for example, under argon pressure.

Preferably the synthesizer comprises a reagent storing component for storing building blocks and/or building block solutions, activators and/or activator solutions, washing solutions and/or washing solvents and/or deprotection solutions. Preferably the synthesizer comprises a reagent storing component for storing one or more reagents and/or reagent solution and/or solvents. The reagents and/or reagent solutions preferably comprise building blocks, building block solutions, activator, activators solutions, washing solutions, washing solvents and deprotection solutions. Preferably the synthesizer comprises a reagent storing component for storing building blocks or building block solutions, activators or activator solutions, washing solutions or washing solvents, deprotection solutions and capping solutions.

Therefore the present invention relates to a synthesizer for automated multistep synthesis on a solid support comprising:
(a) a reaction vessel,
(c) a reagent storing component for storing building blocks and/or building block solutions, activators and/or activator solutions, washing solutions and/or washing solvents and deprotection solutions, the reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component for storing building blocks and/or building block solutions, activators and/or activator solutions, washing solutions and/or washing solvents and deprotection solutions, the reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

According to the present invention the synthesizer comprises a **reagent delivery system.** The inventive synthesizer may comprise at least one reagent delivery system. Preferably the synthesizer of the present invention comprises a reagent delivery system adapted for delivery of building blocks or building block solutions, activators or activator solutions, washing solvents or washing solutions, deprotection solutions, capping solutions and inert gas to the reaction vessel. Preferably the inventive synthesizer comprises a reagent delivery system adapted for receiving building blocks or building block solutions, activators or activator solutions, washing solvents or washing solutions, deprotection solutions and capping solutions from the reagent storing component. The device of the present invention may also comprise one or more reagent delivery systems. The reagent delivery system may comprise one or more reagent delivery sub-systems or reagent delivery sub-components for delivery of one or more of the one or more reagents, solutions and/or reagent solution to the reaction vessel.

Therefore the present invention relates to a synthesizer for automated multistep synthesis on a solid support comprising:
(a) a reaction vessel,
(c) a reagent storing component comprising one or more reagent containers,
(d) a reagent delivery system for delivering building blocks and/or building block solutions, activators and/or activator solutions, washing solutions and/or washing solvents and deprotection solutions, wherein the reagent delivery system is configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system for delivering building blocks and/or building block solutions, activators and/or activator solutions, washing solutions and/or washing solvents and deprotection solutions, wherein the reagent delivery system is configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

In one embodiment, the synthesizer for automated multistep synthesis on a solid support comprises:
(a) a reaction vessel,
(c) a reagent storing component comprising one or more reagent containers,
(d) a reagent delivery system for delivering building block solutions, activator solutions, washing solvents and/or washing solutions and deprotection solutions, wherein the reagent delivery system is configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system for delivering building block solutions, activator solutions, washing solvents and/or washing solutions and deprotection solutions, wherein the reagent delivery system is configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

In one embodiment, the synthesizer for automated multistep synthesis on a solid support comprises:
(a) a reaction vessel,
(c) a reagent storing component for storing building blocks and/or building block solutions, activators and/or activator solutions, washing solutions and/or washing solvents and deprotection solutions, the reagent storing component comprising one or more reagent containers,
(d) a reagent delivery system for delivering building blocks and/or building block solutions, activators and/or activator solutions, washing solutions and/or washing solvents and deprotection solutions, wherein the reagent delivery system is configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component for storing building blocks and/or building block solutions, activators and/or activator solutions, washing solutions and/or washing solvents and deprotection solutions, the reagent storing component comprising one or more reagent containers,
(d) a reagent delivery system for delivering building blocks and/or building block solutions, activators and/or activator solutions, washing solutions and/or washing solvents and deprotection solutions, wherein the reagent delivery system is configured to establish a fluid communication between each of the one or more reagent containers and the reaction vessel, wherein at least two fluid communications are separate of each other,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

Preferably the reagent delivery system connects the reagent storing component with the reaction vessel. Preferably the reagent delivery system is connected to the reagent storing component and is further connected to the reaction vessel. It is preferred that the reagent storing component and the reaction vessel are not directly connected to each other, in other words it is preferred that the reagent storing component and the reaction vessel are not in direct fluid communication with each other. Preferably the reagent delivery system is interposed between the reaction vessel and the reagent storing component. Preferably the reagent delivery system is in fluid communication with the reagent storing component and is further in fluid communication with the reaction vessel. It is preferred that the reagent delivery system receives the reagents from the reagent storing component and after receiving the reagents from the reagent storing component the reagents delivery system may deliver the reagents to the reaction vessel in order supply the reagents to the reaction mixture inside the reaction vessel.

The reagent delivery system may be connected to the reaction vessel for delivery of the reagents and/or reagents solution and/or solvents to the reaction vessel through the two or more inlets of the reaction vessel. The reagent delivery system may be connected to the reaction vessel through two or more reagent delivery lines. Thus, the reagent delivery system may be adapted to deliver reagents and/or reagent solutions and/or solutions and/or solvents to the reaction vessel and may be further adapted to supply the reagents and/or reagent solution and/or solutions or solvents through the one or more inlets of the reaction vessel. Preferably the reagent delivery system may be adapted to deliver one or more reagents and/or one or more reagent solutions and/or one or more solutions in different amounts, at different points in time and in a specific sequence or specific order, and further through specific inlets of the one or more inlets of the reaction vessel during the synthesis cycles on a solid support. It is therefore preferred that the reagent delivery system comprises suitable technical means, preferably suitable technical means electronically coupled to a computing device, for delivery of one or more reagents and/or one or more reagent solutions and/or one or more solutions in different amounts, at different points in time and in a specific sequence or specific order during the synthesis cycles. The reagent delivery system may comprise a pump system or one or more pumps such as syringe pumps, peristaltic pumps or other suitable pumps and may further comprise one or more valves or one or more valve assemblies, one or more manifolds, one or more distributing components and similar suitable technical means. It is preferred that the pump system or the one or more pumps, the one or more valves or the one or more valve assemblies, the one or more manifolds and/or the one or more distributing components and similar technical means are under control of a computing device comprising at least one processor.

The synthesizer of the present invention may therefore comprise one or more valves or valve assemblies for regulating, directing or controlling the flow of fluids like gases or liquids. Valves or valve assemblies may be adapted for opening, closing or partially obstructing various passageways. Valves may be operated in gradual change between two or more positions, such as in two-port, three-port, four-port or in multiport valves. The inventive synthesizer may comprise one or more fluidic valves operably connected to one or more components of the synthesizer. Each fluidic valve of the synthesizer of the present invention may be a rotary valve, solenoid valve block or other multi-port valve or valve system or other suitable valves known to those skilled in the art. The synthesizer of the present invention may further comprise one or more pumps such as syringe pumps, peristaltic pumps or other pumps known to those skilled in the art which may be operably connected to one or more fluidic valves or valve assemblies of the synthesizer of the present invention.

In preferred embodiments the reagent delivery system of the synthesizer according to the present invention may comprise one or more valves, preferably one or more fluidic valves, more preferably one or more rotary valves, and even more preferably one or more rotary valves with 3, 4, 5, 6, 7, 8, 9 or 10 ports. Preferably the reagent delivery system may comprise one or more valves such as one or more rotary valves, one or more solenoid valve blocks or other multiport valves. The reagent delivery system may further comprise one or more pumps or a pump system. Preferably, the reagent delivery system comprises at least one syringe pump. Preferably, the reagents may be delivered via a syringe pump system. Preferably the reagents may be delivered via a syringe pump system as a component of the reagent delivery system. Preferably, the syringe pump system may be a component of the reagent delivery system.

In preferred embodiments of the present invention a syringe pump may drive the fluids within the reagent delivery system. The syringe pump and the reagent delivery system may be connected through one or more loop lines. Loop lines allow for careful delivery of sensitive and/or corrosive chemical solutions. The resting volume of the loops is defined as a function of the tubing length. A driving solvent may be taken to fill up the loops ahead the withdrawing of chemical solution from the reagent delivery system. This avoids the direct contact of the reagents with the syringe pump, which prevents pump deterioration and cross-contamination. The loops may be made from an inert material such as, for example, Teflon, poly-(tetrafluoroethylene) (PTFE) and the like. The size of the loops may be varied. The exact size will depend on the capacity of the syringe pump and the amount of reagent to be delivered to the reaction vessel. The size of each loop will also depend on the nature of the reagent to which it is associated. For example, if the reaction vessel is 20 mL, then a loop sized from about 1 to 5 mL may be used, preferably from about 2 to 4 mL. Each loop may be sized the same or different. For example, loops attached to building blocks may be smaller than those attached to basic reagents such as the deprotection or capping reagents as the quantity of the former used during any synthesis step is relatively small compared to the amount of such basic reagents.

As already described above in preferred embodiments of the present invention the reaction vessel may comprise one or more inlets at the top of the reaction vessel and may comprise one or more inlets at the bottom of the reaction vessel. In further preferred embodiments the reaction vessel may comprise one or more inlets at the top of the reaction vessel and may comprise only one inlet at the bottom of the reaction vessel. The reagent delivery system may be connected to the reaction vessel through the one or more inlets at the top of the reaction vessel and through the one or more inlets at the bottom of the reaction vessel. In preferred embodiments of the present invention the reagent delivery system may be adapted to deliver building blocks or building block solutions, activators or activator solutions, and washing solvents or washing solutions through the one or more inlets at the top of the reaction vessel. The reagent delivery system may be further adapted to deliver deprotection solutions and capping solutions through the one or more inlets at the bottom of the reaction vessel. The reagent delivery system may be further adapted to deliver inert gas, such as argon, to the reaction vessel.

The reagent delivery system may comprise one or more reagent distribution components. The reagent delivery system may comprise one or more reagent distribution components for delivery of reagents and/or solutions and/or reagent solution to the reaction vessel. Each of the reagent distribution components may be connected to the reagent storing component or one or more reagent storing components. In preferred embodiments of the present invention the reagent delivery system comprises a first and a second reagent distribution component for driving the flow of fluids or gases to the reaction vessel. In preferred embodiments of the present invention the synthesizer comprises a first reagent distribution component and a second reagent distribution component.

Therefore the present invention relates to a synthesizer for automated multistep synthesis on a solid support comprising:
(a) a reaction vessel,
(c) a reagent storing component comprising one or more reagent containers,
(d) a reagent delivery system comprising a first reagent distribution component and a second reagent distribution component, wherein the first reagent distribution component is configured to establish a separate fluid communication between the one or more reagent containers and the reaction vessel and wherein the second reagent distribution component is configured to establish a separate fluid communication between the one or more reagent containers and the reaction vessel,
(e) a cooling device for cooling the reaction vessel,
(g) an inert gas delivery system comprising a gas container and a gas valve manifold establishing a separate fluid communication between each of the one or more reagent containers and the gas container.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system comprising a first reagent distribution component and a second reagent distribution component, wherein the first reagent distribution component is configured to establish a separate fluid communication between the one or more reagent containers and the reaction vessel and wherein the second reagent distribution component is configured to establish a separate fluid communication between the one or more reagent containers and the reaction vessel,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

The first reagent distribution component may be connected to the reaction vessel through the one or more inlets on the top of the reaction vessel. Thus, the first distribution component or the top distribution component may be in fluid communication with the reaction vessel through the one or more inlets at the top of the reaction vessel. The second distribution component may be connected to the one or more inlets at the bottom of the reaction vessel. Thus, the second distribution component or bottom distribution component may be in fluid communication with the reaction vessel through the one or more inlets at the bottom of the reaction vessel.

Preferably the first distribution component or the top distribution component supplies the washing solvents, building blocks or building block solutions and activators or activator solutions to the reaction vessel. The top distribution component may be also adapted to provide a ventilation exit for gases.

Preferably the second distribution component or the bottom distribution component supplies the deprotection solutions, capping solutions and supplies inert gas (bubbling gas) to the reaction vessel. The inert gas may be provided as bubbling gas which may be used for mixing and creating an inert and anhydrous atmosphere inside the reaction vessel. In preferred embodiments the bottom distribution component is also adapted for discharging the liquids or solution after one or more synthesis or washing steps.

Therefore the present invention relates to a synthesizer for automated multistep synthesis on a solid support comprising:
(a) a reaction vessel,
(c) a reagent storing component comprising one or more reagent containers,
(d) a reagent delivery system comprising a first reagent distribution component for delivery of washing solvents, building blocks and/or building block solutions and activators and/or activator solutions and a second reagent distribution component for delivery of deprotection solutions, wherein the first reagent distribution component is configured to establish a separate fluid communication between the one or more reagent containers and the reaction vessel and wherein the second reagent distribution component is configured to establish a separate fluid communication between the one or more reagent containers and the reaction vessel,
(e) a cooling device for cooling the reaction vessel,
(g) an inert gas delivery system comprising a gas container and a gas valve manifold establishing a separate fluid communication between each of the one or more reagent containers and the gas container.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system comprising a first reagent distribution component for delivery of washing solvents, building blocks and/or building block solutions and activators and/or activator solutions and a second reagent distribution component for delivery of deprotection solutions, wherein the first reagent distribution component is configured to establish a separate fluid communication between the one or more reagent containers and the reaction vessel and wherein the second reagent distribution component is configured to establish a separate fluid communication between the one or more reagent containers and the reaction vessel,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

In preferred embodiments of the present invention the reagent delivery system or the one or more reagent distribution components may further comprise one or more reagent distribution sub-components. In preferred embodiments of the present invention the reagent delivery system may comprise a building block distribution component for delivery of the building blocks and/or building block solutions, an activator distribution component for delivery of the activators and/or the activator solutions and/or a washing solvent distribution component for delivery of washing solvents or washing solution, a deprotection distribution component for delivery of deprotection solution and may further comprise a capping distribution component for delivery of capping solutions to the reaction vessel.

In preferred embodiments of the present invention the **top distribution component** may comprise a building block distribution component, an activator distribution component and a washing solvents or washing solutions distribution component. The top distribution component may further comprise a syringe pump for driving the fluids within the top distribution component. The lines between the syringe pump and the top distribution component may be provided in form of loop lines to allow for careful delivery of sensitive and/or corrosive chemical solutions. The resting volume of the loops is defined as a function of the tubing length. The top distribution component may further comprise a washing solvents component, wherein a driving solvent may be taken to fill up the loops ahead the withdrawing of chemical solutions from the building blocks distribution component and the activator distribution component. This avoids the direct contact of the reagents with the syringe pump, which prevents the pump deterioration and cross-contamination.

The **building block distribution component** may comprise one or more valves or a valve assembly or similar technical means, for example, a rotary valve that distributes the fluids within the building block distribution component. The building block distribution component is connected to the reagent storing component, preferably to a building blocks storing component which may comprise one or more building block containers or building block solution containers. Each of the building block containers or building block solution containers may be connected to the one or more valves or valve assembly or similar technical means. The building block containers of the building block storing component may be connected to a gas delivery system to provide inert gas to each of the building block containers. Thus, each of the building block containers may be stored under anhydrous and inert atmosphere. Each of the building block containers or building block solutions containers may be in fluid communication with the building block distribution component through the one or more valves or valve assembly or similar technical means. The one or more valves or the valve assembly may be further connected to the reaction vessel. Thus, the reaction vessel may be in fluid communication with the building block distribution component through the one or more valves or valve assembly or similar technical means. The reaction vessel may be in fluid communication with the building block distribution component through, for example, one or more valves or valve assembly and may be further connected through a building blocks delivery line. The building block distribution component may be also connected to a waste container and/or gas delivery system for receiving inert gas through one or more further delivery lines. The one or more valves or valve assembly or other suitable technical means for distribution of the building blocks within the building block distribution component may be under control of a processor of a computing device configured to control the distribution of the building blocks and preferably configured to control the one or more valves or valve assembly such as a rotary valve distributor. The building block distribution component may be connected to a syringe pump system, for example, through one or more valves or valve assembly and may be further connected to the syringe pump via a loop line.

The **activator distribution component** may comprise one or more valves or a valve assembly or similar technical means for example a rotary valve that distributes the fluids within the activator distribution component. The activator distribution component is connected to a reagent storing component, preferably an activator or activator solution storing component which may comprise one or more activator containers or activator solution containers. The activator containers of the activator storing component may be connected to a gas delivery system to provide inert gas to each of the activator containers. Thus, each of the activator containers may be stored under anhydrous and inert atmosphere. Each of the activator containers or activator solution containers may be connected to the one or more valves or valve assembly or similar technical means of the activator distribution component. Thus, each of the activator containers or activator solutions container may be in fluid communication with the one or more valves or valve assembly of the activator distribution component. The activator distribution component may be connected to the reaction vessel through the one or more valves or valve assembly or similar technical means. Thus, the reaction vessel may be in fluid communication with the activator distribution component through the one or more valves or valve assembly or similar technical means. The reaction vessel may be in fluid communication with the activator distribution component through one or more valves or valve assembly or similar technical means and may be further connected through an activator delivery line. The activator distribution component may be also connected to a waste container and/or to a gas delivery system for receiving inert gas through one or more delivery lines. The one or more valves or valve assembly or similar technical means may be under control of a processor of a computing device configured to control the distribution of the activators within the activator distribution component and preferably configured to control the one or more valves or valve assembly and similar technical means such as a rotary valve distributor. The activator distribution component may be connected to a syringe pump system, for example, through one or more valves or valve assembly and may be further connected to the syringe pump via a loop line.

The **washing solvent distribution** component may provide the reaction vessel with one or more washing solvents or washing solutions. Exemplary washing solvents suitable for automated synthesis of oligo-, polysaccharides and peptides on a solid support include, but are not limited to dichloromethane (DCM), tetrahydrofuran (THF) and dimethylformamide (DMF). The washing solvent distribution component is connected to a reagent storing component, preferably a washing solvent storing component. Each of the washing solvents may be stored in a respective solvent container of the washing solvent storing component. Thus, the washing solvent distribution component may be connected to a washing solvent storing component comprising one or more solvent containers. The washing solvent distribution component may be further connected to a syringe pump. One or more of the solvent containers may be connected to the syringe pump through the washing solvent distribution component. The washing solvent distribution component may comprise one or more valves or valve assembly or similar technical means which may be connected to each of the solvent containers of the washing solvents storing component. As an example, the washing solvent distribution component may comprise a multi-port valve, such as a four way magnetic valve. The washing solvents may be delivered to the reaction vessel by the washing solvent distribution component. The washing solvents may be delivered to the reaction vessel through a washing solvent delivery line. The washing solvent containers of the washing solvent storing component may be connected to a gas delivery system to provide inert gas to the each washing solvent container. Thus, each of the washing solvent containers may be stored under anhydrous and inert atmosphere, such as under argon atmosphere. The magnetic valve may be further connected to the gas delivery system to receive inert gas and to provide and deliver inert gas to the reaction vessel. The one or more valves or valve assembly or similar technical means suitable for distribution of the washing solvents within the washing solvent distribution component may be under control of a processor of a computing device configured to control the distribution of the washing solvents and washing solution and preferably configured to control the one or more valves or valve assembly and similar technical means such as a magnetic valve distributor.

The **bottom distribution component** may comprise one or more valves such as one or more multi-port valves, or valve assemblies, or similar technical means. The bottom distribution component may comprise a deprotection distribution component for delivery of deprotection solutions to the reaction vessel and may further comprise a capping distribution component for delivery of capping solution to the reaction vessel. The bottom distribution component may be further connected to a waste container. The bottom distribution component may be further connected with a gas delivery component for delivery of inert gas, such as argon, to the reaction vessel. One or more of the components of the bottom distribution component may be connected to the reaction vessel through a loop line which allows for delivery of chemical solutions from the deprotection distribution component, the capping distribution component and delivery of inert gas from the gas delivery component. The loop lines may be also adapted for discharge of waste liquids from the reaction vessel. The bottom distribution component may comprise for example a multiple ways magnetic valve which may be electronically coupled to a computing device comprising at least one processor configured to control the delivery of deprotection solutions, capping solutions and inert gas to the reaction vessel and further configured to control the discharging of liquids and solutions of the reaction vessel after each reaction or washing step. The deprotection distribution component is connected to a reagent storing component, preferably a deprotection solution storing component. The deprotection storing component may comprise one or more containers for storing deprotection reagents. The deprotection storing component may be connected to a gas delivery system to provide inert gas to the each deprotection reagent container. Thus, each of the deprotection reagent containers may be stored under anhydrous and inert atmosphere, such as under argon atmosphere. The capping distribution component is connected to a reagent storing component, preferably a capping solution storing component. The capping storing component may comprise one or more containers for storing capping reagents. The capping storing component may be connected to a gas delivery system to provide inert gas to the each capping reagent container. Thus, each of the capping reagent containers may be stored under anhydrous and inert atmosphere, such as under argon pressure.

The synthesizer according to the present invention comprises **a cooling device** for cooling the reaction vessel. The cooling device therefore provides active cooling to the reaction vessel and thus provides active cooling of the reaction mixture inside of the reaction vessel. The cooling device is therefore preferably in thermal communication with the reaction vessel. The cooling device is preferably adapted for cooling the reaction vessel to temperatures of -80°C to +60 °C, preferably -40°C to +40°C, more preferably -40°C to +25°C, and more preferably -40°C to +20°C. Thus, the synthesizer of the present invention may comprise a cooling device for regulating the temperature of the reaction vessel. The cooling device may be under control of a thermal controller as a part of a computing device comprising at least one processor. The cooling device may be further adapted to maintain the temperature within the reaction vessel. The cooling device may be adapted to adjust the temperature within +1°C and -1°C of the reaction temperature. The cooling device may be equipped with a temperature sensor or thermometer, wherein the cooling device temperature may be adjusted either manually or preferably by a computing device, more preferably by a thermal controller. For example, the cooling device could be an external refrigerated circulator or a cooling block, or a chiller. The cooling block may be made of any heat transfer material. The block may have channels running through to pass microwave transparent coolant fluid through. The reaction vessel may be placed in a cavity of the cooling block. In a preferred embodiment of the present invention the microwave transparent coolant fluid may be circulated around the reaction vessel via a sleeve surrounding the reaction vessel. In preferred embodiments of the present invention the cooling device may comprise a cooling jacket. The cooling jacket may be in form of a cooling coil surrounding the reaction vessel. Preferably, the cooling jacket is in thermal communication with the reaction vessel. The cooling coil or cooling jacket may be further provided with a thermal isolation cover to increase the heat exchange efficiency of the cooling jacket or cooling coil.

Therefore the present invention relates to a synthesizer for automated multistep synthesis on a solid support comprising:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel, wherein the cooling device comprises a cooling jacket in thermal communication with the reaction vessel,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel, wherein the cooling device comprises a cooling jacket in thermal communication with the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

In one embodiment, the synthesizer for automated multistep synthesis on a solid support comprises:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel, comprising a cooling coil in thermal communication with the reaction vessel,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the microwave transparent reaction vessel comprising a cooling coil in thermal communication with the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

The cooling device may be connected to a coolant fluid reservoir. The cooling device may be connected to a cooling circuit pump. In preferred embodiments of the invention the cooling jacket surrounding the reaction vessel may be connected to a coolant fluid reservoir and a coolant circuit pump. The coolant fluid reservoir, the coolant circuit pump and the cooling jacket surrounding the reaction vessel may form a closed cooling circuit. The cooling jacket may be provided in form of a cooling coil surrounding the reaction vessel. The coolant circuit pump may be under control of a computer comprising at least one processor. The cooling device may comprise a cooling unit configured to control the temperature of the cooling device. The cooling unit may be electronically coupled to a computing device comprising at least one processor. The cooling circuit pump may be electronically coupled to a computing device comprising at least one processor to control the flow of the microwave transparent coolant fluid through the cooling circuit.

In one embodiment of the present invention the synthesizer comprises at least one **cooling device** for cooling the reaction vessel or more precisely for cooling the content of the reaction vessel and at least one **pre-cooling device** for pre-cooling the reagents to be supplied to the reaction vessel. The cooling device provides at least active cooling of the reaction vessel. The pre-cooling device provides at least active cooling of one or more of the reagents to be supplied to the reaction vessel.

Therefore the present invention relates to a synthesizer for automated multistep synthesis on a solid support comprising:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e1) a cooling device for cooling the reaction vessel,
(e2) a pre-cooling device for pre-cooling the reagents to be supplied,
(g) an inert gas delivery system comprising a gas container and a gas valve manifold establishing a fluid communication between each of the one or more reagent containers and the gas container, wherein at least two fluid communications are separate of each other.

In one embodiment the synthesizer for automated multistep synthesis on a solid support comprises:
(a) a microwave transparent reaction vessel equipped with a temperature sensor,
(b) a microwave generator component,
(c) a reagent storing component comprising one or more reagent containers,
(d) a reagent delivery system configured to establish a fluid communication between each of the one or more reagent containers and the reaction vessel, wherein at least two fluid communications are separate of each other,
(e1) a cooling device for cooling the reaction vessel,
(e2) a pre-cooling device for pre-cooling the reagents to be supplied,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device,
(g) an inert gas delivery system comprising a gas container and a gas valve manifold establishing a fluid communication between each of the one or more reagent containers and the gas container, wherein at least two fluid communications are separate of each other.

Current synthesizers of the prior art require expensive and large cooling systems, thereby allowing, in principle, active cooling of the reaction vessel during the coupling reactions. However, the synthesizers known in the art still suffer from low to moderate yields, decomposition of reagents and formation of side and decomposition products. These deficiencies could neither be remedied by cooling the reaction vessel to even lower temperatures or by a very slow addition and careful delivery of reagents to the reaction mixture. The first attempt did not result in the desired improvement of yield and reduction of decomposition and decrease of formation of side and decomposition products, and the latter approach led to an impractical increase of the total reaction time, while simultaneously also the side and decomposition products increased without the desired enhancement of the yield. In addition, up-scaling of this way running the reaction was almost impossible. Thus, the inventors have found that pre-cooling of the reagents prevents and avoids decomposition of the reagents during the synthesis cycles which otherwise could result in undesired side-products and decomposition products.

As an example, Fig. 6 shows a chart comparing temperature readings inside the reaction vessel, taken during a synthesis cycle, in the presence and absence of the pre-cooling device. The three thermal stages are identified. It can be clearly recognized that temperature spikes appear when a liquid is dispensed in the reaction vessel. The dashed curve shows the temperature profile for the device without pre-cooling of the reagents to be supplied. The thermal spikes are remarkable at the pre-coupling regime (subzero temperatures). The solid line depicts the temperature profile with active pre-cooling. The incoming solutions of reagents are effectively pre-cooled and this suppresses the undesired temperature spikes.

The inventive synthesizer comprising an improved cooling system comprising a cooling device and a pre-cooling device allows the prevention of fluctuations in temperature of a reaction mixture which may otherwise result in loss of sensitive intermediates and reagents, in particular in the coupling steps of the synthesis cycles. Thus, with the cooling system of the synthesizer comprising a cooling device and a pre-cooling device unfavorable temperature spikes may be effectively prevented during the transfer of building blocks and activators to the reaction vessel. Furthermore, the cooling system of the present invention allows for advantageous temperature adjustments of the reaction vessel in the pre-coupling regime and further of the reaction mixture to prevent decomposition of the reactants, sensitive intermediates and desired products at different thermal stages of the synthesis cycles. Thus, the improved cooling system of the inventive synthesizer consists of a cooling device and a pre-cooling device. Therefore the inventive synthesizer comprises a cooling device for cooling the reaction vessel and a pre-cooling device for pre-cooling the reagents to be supplied for control of the temperature of one or more reagents and/or the reaction mixture during one or more synthesis steps of the synthesis cycles to prevent decomposition of the sensitive intermediates and reagents. Of course, the cooling device for cooling the reaction vessel and the pre-cooling device for pre-cooling the reagents can be combined to a single cooling device for cooling both, the reaction vessel and the reagents to be supplied. Most preferably the one cooling device for both is a Peltier cooling element.

As used herein, the term >> **pre-cooling the reagents** << refers to the cooling of the reagent storing component and/or the cooling of the reagent delivery system to the reaction vessel and preferably to the cooling of the reagent delivery system and more preferably to the cooling of the reagent storing component and the cooling of the reagent delivery system.

Preferably the pre-cooling device is in thermal communication with the reagent delivery system. The pre-cooling device is preferably interposed between the reaction vessel and the reagent delivery device. The pre-cooling device is preferable positioned in close proximity to the one or more inlets of the reaction vessel. Thus, the pre-cooling device may be adapted to function as a pre-cooling inlet for pre-cooling the reagents to be supplied. The pre-cooling component may be adapted for active cooling of the reagents to be supplied. In other words the incoming solutions at room temperature from the reagent delivery system are actively cooled by the pre-cooling device to a temperature around the preset temperature of the reaction mixture in the reaction vessel. By pre-cooling the at room temperature incoming solutions to around the temperature of the reaction vessel the reagents to be supplied are added to the reaction mixture at a temperature that preferably corresponds to the temperature of the reaction mixture in the reaction vessel.

In one embodiment, all reagents and solvents, including, building blocks, activators and washing solvents, are pre-cooled before addition to the reaction mixture in the reaction vessel. In one embodiment, only the activators or activator solutions are pre-cooled before addition to the reaction mixture in the reaction vessel. Preferably at least the building blocks and activators (or a solution thereof) are pre-cooled before addition to the reaction mixture in the reaction vessel and preferably all reagents and solutions added to perform the coupling reaction are pre-cooled close to the temperature of the reaction mixture in the reaction vessel. Furthermore, the resulting intermediates are often temperature sensitive and may decompose at higher temperatures then the desired temperature of the reaction mixture. It should be clear that already formed temperature sensitive intermediates in the reaction mixture may decompose in case further warmer reagents or solvents are added to the reaction mixture, such as reagents or solvents at room temperature. In such a case it may be suitable to supply these reagents or solvents in a slow and careful manner, for example dropwise manner, by monitoring the temperature of the reaction mixture at the same time. However, in larger batch sizes a larger amount of the reagents to be supplied are added to the reaction mixture. The delayed addition, for example dropwise addition, and the period of time until the reaction mixture is adjusted to the desired and preset temperature may also result in decomposition of the reagents and in particular the sensitive intermediates. The inventors of the present invention have found that on the one hand the rapid addition of the reagents to the reaction mixture in the pre-coupling stage and impregnating of the resin to allow these reagents to diffuse through the porous solid for a period of time of 1 minute to 20 minutes, preferably 5 minutes has an advantageous effect on the overall yield of the desired oligo- and polysaccharide.

On the other hand the inventors have found that the monitoring of the reaction mixture temperature during the addition of reagents and solvents and the careful and slow addition of these reagents such that the reaction mixtures maintains the desired reaction mixture has also an advantageous effect on the overall yield of the desired oligo-, polysaccharide or peptide. The inventive synthesizer comprising a pre-cooling device for pre-cooling the reagents to be supplied has the advantage that the pre-cooled reagents to be supplied may be added in a faster and rapid manner to the reaction mixture without unfavorable temperature fluctuations in the reaction mixture in the pre-coupling stage at preferably -40°C to -10°C and has the advantages that the added pre-cooled reagents may be allowed to impregnate the resin to diffuse through the porous solid for a period of time of 1 minute to 20 minutes, preferably 5 minutes such that the overall time of the pre-coupling stage including the adding and impregnating of the reagents may be reduced such that decompositions of the reagents and the resulting intermediates may be effectively prevented in comparison to a slow and careful addition of the reagents to be supplied which may result in an overall time of the pre-coupling stage of over 25 minutes or 30 minutes or more.

The rapid addition of the pre-cooled reagents in the pre-coupling stage to allow effective impregnation of the resin and the prevention of the temperature fluctuation during the addition of the pre-cooled reagents has been shown to be particularly advantageous with regard to the glycosylation reactions in the synthesis of oligo- and polysaccharides.

The pre-cooling device is preferably adapted for pre-cooling of the reagents to be supplied to the corresponding temperature of the reaction mixture in the reaction vessel. For example, if the reaction vessel is actively cooled to a temperature of -30° in the pre-coupling stage of a synthesis cycle the reagents to be supplied may preferably be pre-cooled also to a temperature of -30°C by the pre-cooling device or preferably to -31°C or -32°C or -33°C or to a lower temperature. In such a case the pre-cooled reagents at a temperature of -30°C are added to the reaction mixture having a temperature of -30°C, wherein temperature fluctuations in the reaction mixture are prevented during addition of the reagents. However, the reagents to be supplied to the reaction vessel may be also added at a slightly higher temperature then the temperature of the reaction mixture in the reaction vessel.

Preferably the reagents are pre-cooled by the pre-cooling device to a temperature in the temperature range corresponding to the temperature range of -40°C to -8°C, preferably of -30°C to -9°C, and more preferably to the temperature range of -20°C to -10°C of the pre-coupling stage. In preferred embodiments of the present invention the pre-cooling device may be adapted for pre-cooling the reagents to be supplied at least to a temperature which is not higher than -3°C, preferably -6°C, more preferably -8°C or -10°C of the temperature of the reaction mixture in the transparent reaction vessel so that the temperature of the supplied reagents is not higher than 3°C, preferably 6°C, more preferably 8°C or 10°C below the temperature of the reaction mixture in the reaction vessel. Preferably the pre-cooling device may be adapted to cool the reagents to be supplied to a temperature in the range of 3°C to 6°C below the temperature of the reaction mixture in the reaction vessel. Thus, it is preferred that the reagents to be supplied are pre-cooled to the corresponding temperature of the reaction vessel with a maximum variance of 0°C to 10°C below the temperature of the reaction mixture in the reaction vessel. Most preferably the temperature of the reaction mixture in the reaction vessel does not exceed a temperature of -10°C in the pre-coupling stage of the synthesis cycle.

Thus, it is preferred that the reagents to be supplied are pre-cooled to the corresponding temperature of the reaction vessel with a maximum variance of 10°C or maximum deviation of 10°C, preferably with a maximum variance of 5°C or maximum deviation of 5°C, more preferably with a maximum variance of 3°C or maximum deviation of 3°C. In preferred embodiments it is preferred that the temperature of the reaction mixture in the reaction vessel does not exceed a temperature of -10°C in the pre-coupling stage of the synthesis cycle.

Most preferably, the temperature of the reagents to be supplied to the reaction vessel is in the range of -18°C to -8°C, preferably in the range of -16°C to -9°C, more preferably in the range of -14°C to -10°C and still more preferably in the range of -12°C to -10°C.

In preferred embodiments of the present invention various liquid lines such as washing solvents lines, building block lines, and activator solution lines may be pre-cooled before feeding the reaction vessel. In other words the pre-cooling device may be located upstream to the reaction vessel. The pre-cooling device may be interposed between the reaction vessel and the reagent delivery system.

Thus, the reaction vessel may be connected to a pre-cooling device which allows active pre-cooling of various liquid lines such as washing solvents, building blocks, activator solution before said washing solvents, building blocks, activator solution are delivered and supplied to the reaction vessel. In such embodiments the flow of liquid to the reaction vessel from the reagent delivery system is not interrupted as the reagents to be supplied to the reaction vessel are pre-cooled during the delivery to the reaction vessel. Preferably the flow speed of the liquids through the various liquid lines are taken into account as by lower flow speeds the reagents may be pre-cooled for a longer period of time to enable effective cooling to the desired temperatures. In one embodiment, all liquid lines between the reagent delivery system and the reaction vessel are pre-cooled by the pre-cooling device located between the reagent delivery system and the reaction vessel. In one embodiment, only one liquid line between the reagent delivery system and the reaction vessel is pre-cooled by the pre-cooling device located between the reagent delivery system and the reaction vessel. In a further embodiment, at least one liquid line between the reagent delivery system and the reaction vessel is pre-cooled by the pre-cooling device located between the reagent delivery system and the reaction vessel.

In preferred embodiments the pre-cooling device may comprise a metal cooling surface made of a metal heat exchange material. In such embodiment the building block delivery line and activator line may contact the metal cooling surface at a preset temperature. Thus, the metal cooling surface may be cooled to a predefined temperature. Preferably the predefined temperature corresponds to the temperature of the reaction mixture in the reaction vessel, thus preferably corresponds to the desired reaction temperature. The metal cooling surface may be made of any known metal heat exchange material. In further preferred embodiments the washing solvent delivery line may also contact the metal cooling surface at a preset temperature.

In preferred embodiments the pre-cooling device may comprise a thermoelectric contact-cooling device such as a Peltier cooler. The cooling effect provided by a Peltier cooler is based on the well-known Peltier effect. The Peltier cooler is adapted to provide the means to reach the preset temperature. Thus, the Peltier cooler may be in thermal communication with the metal cooling surface.

In preferred embodiments the thermoelectric contact-cooling device is assisted by a line of coolant fluid, such as for example cooling water, preferably at a temperature of 15°C to cool down the warm side of the thermoelectric contact-cooling device. The cooling water line could be provided by a central cooling system or a compact commercial cooling unit.

In preferred embodiments the pre-cooling device may also provide active cooling to the reaction vessel. In other words the pre-cooling device may be a part or component of the cooling device. In such embodiments the pre-cooling device may provide active cooling to the cooling jacket surrounding the reaction vessel. Thus, the pre-cooling device may be adapted to provide active cooling to the cooling circuit comprising the cooling jacket, cooling circuit pump and the coolant fluid reservoir. In such embodiments the cooling device for cooling the reaction vessel and the pre-cooling device for pre-cooling the reagents to be supplied are adjusted simultaneously to the same preset temperature. Thus, based on the preset temperature for cooling of the reaction vessel the reagents to be supplied will be pre-cooled to the temperature of the reaction vessel.

In embodiments of the inventive synthesizer comprising a pre-cooling device, the reagent delivery system may be connected to the pre-cooling device. With other words, the reagent delivery system may be connected to a pre-cooling device for pre-cooling the reagents to be supplied. The reagent delivery system device may be adapted for receiving reagents from the reagent storing device and may be further adapted for delivery of the received reagents to a pre-cooling device for pre-cooling the reagents to be supplied and may be further adapted for delivery of the pre-cooled reagents to the reaction vessel. Thus, in preferred embodiments the reagent delivery system may be adapted for receiving one or more reagents such as building blocks and activators and/or one or more reagent solutions such as building block solutions and activator solutions and/or one or more washing solutions and/or one or more deprotection solutions from a reagent storing component or one or more reagent storing components and may be further adapted for delivery of the received one or more reagents and/or one or more reagent solutions and/or one or more washing solution and/or one or more deprotection solutions to a pre-cooling device for pre-cooling the reagents to be supplied and may be further adapted to deliver the pre-cooled one or more reagents and/or one or more reagent solutions and/or one or more washing solutions and/or one or more deprotection solutions to the reaction vessel.

In embodiments of the inventive synthesizer comprising a pre-cooling device, reagent delivery system may be adapted for delivery of one or more of the reagents and/or reagents solutions and/or washing solutions and/or deprotection solution to the pre-cooling device for pre-cooling the reagents to be supplied. In such embodiments it is particularly preferred that at least the building blocks and the activators are delivered to the pre-cooling device for pre-cooling the reagents to be supplied. In further preferred embodiments the reagent delivery system may also be adapted for delivery of the washing solutions or washing solvents to the pre-cooling device for pre-cooling the reagents to be supplied.

In embodiments of the inventive synthesizer comprising a pre-cooling device, the reagent delivery system may be in thermal communication with a pre-cooling device for pre-cooling the reagents to be supplied. In further preferred embodiments of the present invention one or more delivery lines connecting the reagent delivery system and the reaction vessel may be in thermal communication with a pre-cooling device for pre-cooling the reagents to be supplied. Thus, in such embodiments the reagent delivery system may be adapted for delivery of one or more reagents to the reaction vessel, the reagent delivery system in thermal communication with a pre-cooling device for pre-cooling the reagents to be supplied, wherein the one or more reagents are pre-cooled by the pre-cooling device before entering the reaction vessel, and preferably wherein the one or more reagents are pre-cooled through contact cooling by the pre-cooling device for pre-cooling the reagents to be supplied.

In some embodiments of the inventive synthesizer comprising a pre-cooling device, the reagent delivery system may be interposed between a pre-cooling device and the reagent storing component. In such embodiments the reagent delivery system is connected to the reagent storing component and is further connected to a pre-cooling device. It is particularly preferred that the reagent storing component and the pre-cooling device are not directly connected to each other. In other words it is preferred that the reagent storing component and the pre-cooling device are connected through the reagent delivery system. It is further preferred that the pre-cooling device for pre-cooling the reagents to be supplied connects the reagent delivery system with the reaction vessel. Thus, in some embodiments the reaction vessel, the pre-cooling device, the reagent delivery system and the reagent storing device are successively connected in the following sequence: reaction vessel - pre-cooling device - reagent delivery system - reagent storing device.

In some embodiments of the inventive synthesizer comprising a pre-cooling device and a reagent delivery system comprising a first reagent distribution component and a second reagent distribution component, it is particularly preferred that the first distribution component or the top distribution component is connected to the pre-cooling device for pre-cooling the reagents to be supplied for delivery of pre-cooled building blocks or building block solutions and activators or activator solutions to the reaction vessel. In such embodiments the first distribution component or the top distribution component may also deliver the washing solvents or washing solution to the pre-cooling device and thereafter may supply pre-cooled washing solvents or washing solution to the reaction vessel. The top distribution component may be also adapted to provide a ventilation exit for gases.

In some embodiments of the inventive synthesizer comprising a pre-cooling device, the components of the reagent delivery system, particularly, building block distribution component, activator distribution component and the washing solvent distribution component are adapted to deliver the respective building blocks, activators and washing solvents to the pre-cooling device for pre-cooling the reagents to be supplied and are further adapted to deliver the respective pre-cooled building blocks, pre-cooled activators and pre-cooled washing solvents to the reaction vessel.

**Description of the figures:**
- **FIG. 1**: provides examples of interconnecting molecules for immobilizing saccharides to solid support.
- **FIG. 2**: provides examples of commercially available anchoring groups.
- **FIG. 3**: shows the structures of exemplarily saccharide building blocks which can be used in the present invention.
- **FIG. 4**: is a block diagram illustrating the automated synthesizer using the inventive methods.

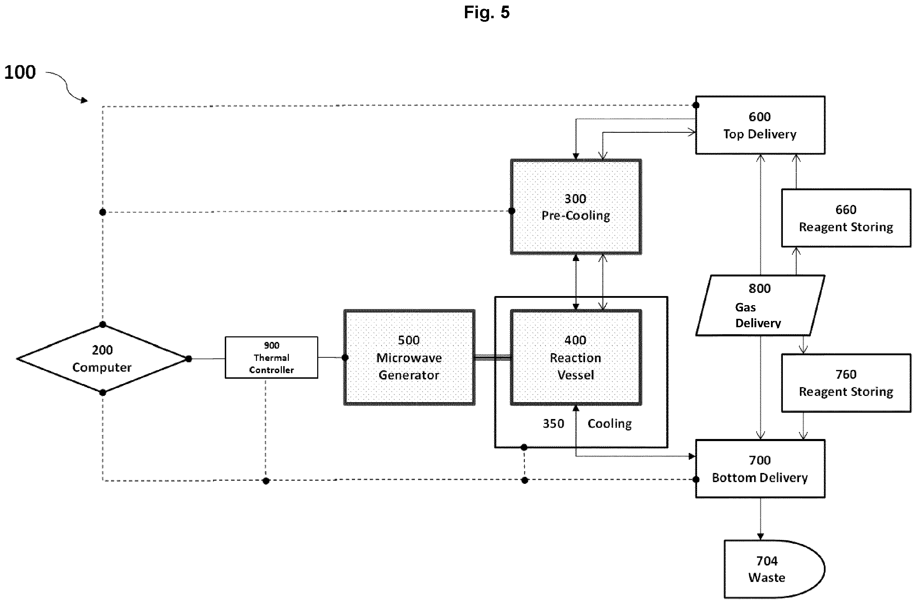

- **FIG. 5**: shows a block diagram illustrating the automated synthesizer with a pre-cooling device (300) using the inventive methods.
- **FIG. 6**: shows the temperature readings inside the reaction vessel, taken during a synthesis cycle of the inventive method, in the presence and absence of the pre-cooling device (300) and without microwave irradiation / heating.
- **FIG. 7**: shows the thermal profile during a blank experiment. The reaction vessel is made of PFA. The solid black line shows the temperature inside the reaction vessel; the solid gray line is the temperature at the wall of the jacket adjusted by the active cooling device. At the beginning, 3 mL of DCM were delivered in the reactor vessel. The temperature is adjusted by the active cooling device from room temperature to -12 °C. The increased to -2 °C by the active cooling device. The reaction vessel was discharged and the temperature of the jacket was adjusted again to cool down to -12 °C; while Fmoc deprotection module is performed using microwave heating against the cooling effect of the cooling device. The microwave radiation was set with a maximum power of 50 W and regulated to maintain a temperature around 45 °C or at least below 60 °C.
- **FIG. 8A**: shows a block diagram illustrating the automated synthesizer with a gas valve manifold using the inventive methods.
- **FIG. 8B**: shows in detail the manifold implemented in the inventive methods.
- **FIG. 9A**: shows charts comparing temperature readings inside the glass reaction vessel and the chiller side, taken during a synthesis cycle via phosphate-glycosylation, with the post coupling reactions assisted by microwave irradiation.
- **FIG. 9B**: shows charts comparing temperature readings inside the glass reaction vessel, taken during a synthesis cycle via phosphate-glycosylation, with and without the microwave irradiation during the post coupling reactions.
- **FIG. 9C**: shows analytical results by MALDI and HPLC of experiments from FIG.**9A**.
- **FIG. 10A**: shows charts comparing temperature readings inside the glass reaction vessel and the chiller side, taken during a synthesis cycle via phosphate-glycosylation, with temperature adjustment during the coupling and post coupling reactions via microwave irradiation.
- **FIG. 10B**: shows analytical results by MALDI and HPLC of experiments from FIG.**10A**.

### List of Reference Signs

- 100: device
- 200: computing device, processor
- 300: pre-cooling device / pre-cooling component
- 350: cooling device for reaction vessel
- 400: reaction vessel
- 500: microwave generator component / microwave generator
- 600: reagent delivery system, top delivery system
- 630-632: reagents containers
- 660, 760: reagent storing component
- 700: reagent delivery system, bottom delivery system
- 704: waste container / waste collector
- 800: inert gas delivery system
- 801: gas container
- 802: manifold
- 803-809: seven pressure valves
- 810: flow control valve
- 820: tubing connector
- 823-829: seven pressure sensors
- 833-839: seven checks or one way valves
- 900: thermal controller

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Example 1: Investigation of microwave-assisted capping and deprotection steps

The improvement in conditions and reaction time for the capping and deprotection steps with microwave radiation was investigated.

During a typical AGA cycle there is the glycosylation coupling step as well as several auxiliary steps (acetyl capping and temporary group deprotection). These auxiliary steps increase the overall yield of the final oligo- or polysaccharide (glycan) by terminating unglycosylated nucleophiles as well as they remove temporary protecting groups that allows the next coupling to occur. These auxiliary steps have been a bottleneck in the overall time required for one AGA cycle. The investigation of the overall time required for one AGA cycle by using microwave radiation during these auxiliary steps has shown that microwave assisted deprotection and capping steps drastically reduce the reaction time. The results shown in Table **1** demonstrate that the utilization of a microwave generator component is not only instrumental for hastening the cooling to heating process, microwave-assisted synthesis also drastically reduces chemical reaction time. Under these rapid microwave-assisted conditions, the steps remained orthogonal and few side reactions were observed. With shortened reaction times for these auxiliary steps the overall duration of a standard AGA cycle was successfully reduced from 100 minutes to below 60 minutes and even to 45 minutes.

**Table 1**

| **Module** | **Without Microwave** | | **With Microwave Radiation** | |
|---|---|---|---|---|
| | **Conditions** | **Time** | **Conditions** | **Time** |
| Capping | 2% MsOH in | 25 min | 2% MsOH in | 1 min |
| | CH₂Cl₂/Ac₂O | | CH₂Cl₂/Ac₂O | |
| | (5:1), 25 °C | | (5:1), 45 °C | |
| Deprotection: Lev | 7% N₂H₄HOAc in | 90 min | 7% N₂H₄HOAc in | 1 min |
| | CH₂Cl₂/Pyr/HOAc/H₂O | | CH₂Cl₂/Pyr/HOAc/H₂O | |
| | (20:16:4:1), 25 °C | | (20:16:4:1), 40 °C | |
| Deprotection: NAP | 2% DDQ in | 120 min | 0.5% MsOH in | 1 min |
| | DCE/MeOH/H₂O | | CH₂Cl₂/HFIP/TIS | |
| | (64:16:1), 40 °C | | (20:2:1), 40 °C | |
| Deprotection: Fmoc | 20% Piperidine in DMF, 25 °C | 5 min | 20% Piperidine in DMF, 40 °C | 1 min |
| Deprotection: ClAc | Thiourea in EGME, 80°C | 360 min | Thiourea in EGME, 80°C | 45 min |

### Example 2: Investigation of temperature fluctuations of the reaction mixture by addition of pre-cooled building block and activator solutions

The temperature development inside of the reaction vessel during a synthesis cycle in the presence and absence of pre-cooling device was investigated. FIG. **6** shows the results charts comparing temperature readings inside the reaction vessel, taken during a synthesis cycle, in the presence and absence of a pre-cooling device.

The three thermal stages are shown. Temperature spikes appear when a liquid is dispensed in the reaction vessel. The dashed curve shows the temperature profile for the device without pre-cooling of the reagents. The thermal spikes are remarkable at the pre-coupling regime (subzero temperatures). The solid line depicts the temperature profile with active pre-cooling. The incoming solution of reagents is pre-cooled and this suppresses the temperature spikes. Since there are three thermal stages during a glycosylation cycle: (1) The pre-coupling regime (-40 °C to -10 °C). The building block and activator are allowed to impregnate the resin and for diffusion through the porous solid. The low temperature prevents the early decomposition of the intermediate before the actual coupling; (2) the coupling regime (around 0°C). The increase in the temperature allows for initiating the coupling reaction by promoting the formation of the intermediates; and (3) the post-coupling regime (room temperate or above). The capping and deprotection reactions take place at higher temperature. These reactions close out the glycosylation cycle. Then, the next coupling or process termination takes place.

### Example 3: Synthesis of mannose tetramer via phosphate donor with auxiliary reactions (post-coupling) accelerated by microwave heating.

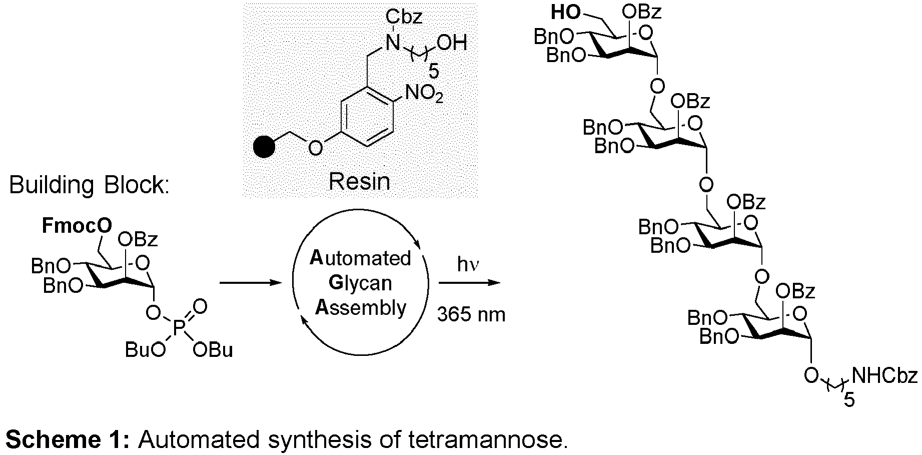

### Automated synthesis working modules

The timing and quantity of solvents/reagents transferred to the reaction vessel in each step is controlled by the software. The reagent delivery system is based on valve-pressured control in which the entire platform is constantly pressurized so that the specific solvent/reagent is transferred from the respective storage components by timing the opening and closing of the appropriate valves.

Module 1: Acidic Washing: The resin loaded into the reaction vessel is washed with DMF, THF, DCM (six times each with 3 mL for 15 s). The resin is swollen in 2 mL DCM, and the temperature of the reaction vessel was adjusted in the range of -22 °C to -20 °C by cooling device (when it is programmed to do so). For acidic washing 1 mL of the solution of 2% TMSOTf in DCM is delivered to the reaction vessel via the pre-cooling device which cools the solution to a temperature of -15 °C (when it is programmed to do so). After three minutes, the solution is drained. Finally, 3 mL DCM is added to the reaction vessel.

Module 2: Glycosylation: Phosphate building block is dissolved in the proper solvent mixture e.g. DCM (5 mL for one initial double cycle for the first coupling and three more single glycosylation cycles to build up the tetramer) in the designated building block storing component. The reaction vessel is set to reach the initial glycosylation temperature. During the adjustment of the temperature in the reactor vessel (when it is programmed to do so), the DCM in the reaction vessel is drained and 1 mL of phosphate building block (5.0 eq. in 1.0 mL DCM) is delivered from the building block storing component to the reaction vessel via the pre-cooling device which cools the solution of phosphate building block (when it is programmed to do so) to a temperature of -18 °C. After the set temperature in the range of -22 °C to -20 °C is reached the resin is incubated in the solution of phosphate building block for 10 min. Then 1.0 mL of the solution of 2% TMSOTf in DCM is delivered to the reaction vessel from the respective activator storing component via the pre-cooling device which cools the activator solution down to a temperature of -18 °C (when it is programmed to do so). The glycosylation mixture is incubated for 30 min in the temperature range of -22 °C to -20 °C, linearly ramped to 0 °C (when it is programmed to do so), and after reaching 0 °C the reaction mixture is incubated for an additional 10 min. Once incubation time is finished, the reaction mixture is drained and the resin is washed with DCE (once, 2 mL for 5 s).

Module 3: Capping: While the temperature of the active cooling element is adjusted to range of -22 °C to -20 °C preparing for the next coupling cycle. The temperature of the reactor vessel is adjusted between 70 and 20 °C by microwave irradiation of the washing solvents and reagents adjusting the irradiation power. The resin is washed twice with DMF (3 mL for 15 s). 2 mL of Pyridine solution (10% in volume in DMF) was delivered and the resin is incubated for one minute. Up to this point the microwave irradiation power is adjust at 50W. The microwave irradiation power is then adjusted 150-180W to proceed with the resin is washed three times with DCM (2 mL for 15). The capping of the unreacted acceptor groups is done by delivering 4 mL of methanesulfonic acid (2% in volume) and acetic anhydride (10% in volume) in DCM. The resin and the reagents are incubated for one minute; then 1 mL of DCM in added to dilute the solution and the incubation continues for another one minute. The solution is drained from the reactor vessel and the resin is washed 3 time with DCM (2 mL for 15 s).

Module 3: Fmoc Deprotection: The temperature of the reactor vessel is adjusted between 70 and 20 °C by microwave irradiation of the washing solvents and reagents adjusting the irradiation power (40 W). The resin is washed with DMF (three times with 3 mL for 15 s), swollen in 2 mL DMF and the temperature of the reaction vessel is adjusted between 70-20 °C. For Fmoc deprotection the DMF is drained and 2 mL of a solution of 20% piperidine in DMF was delivered to the reaction vessel. After 1 min the reaction solution is drained from the reactor vessel. Then, the resin is washed with DMF (three times with 3 mL for 15 s) and DCM (five times with 3 mL). After this module the resin is ready for the next glycosylation cycle.

Resin functionalized with a photo-cleavable linker (64 mg; loading 0.392 mmol/g; 25.1 µmol) was loaded into the reaction vessel of the synthesizer and swollen in 2 mL DCM. The sequence of reaction steps for the formation of tetramannose was as follows:
1. Module 1 was performed with 1 mL TMSOTf solution at the temperature range of -22 °C to -20 °C (when it is programed to do so) for 3 min.
2. Module 2 was performed with 5 equiv Building Block and 2% TMSOTf in DCM solution.
3. Module 3 was carried out in two steps; first 2 mL of Pyridine solution (10% in volume in DMF); then in a second step with 4 mL of methanesulfonic acid (2% in volume) and acetic anhydride (10% in volume) in DCM.
4. Module 4 was carried out with 20% piperidine in DMF.
5. After Module 1 took place the Module 2 repeated twice (for the first coupling). The Modules 3 and 4 were then performed.
6. Subsequently, modules 1-4 were repeated three times in the same manner as described in steps 1-4 in order to obtain a tetramer.

After buildup of the tetramer on the resin the oligosaccharide was cleaved from solid support in a photoreactor: A mercury lamp is turned on 30 min prior to the first cleavage event. The fluorinated-ethylene-propylene (FEP) tubing was washed with 20 mL DCM at a flow rate of 5 mL/min before cleavage. The solid support was pre-swelled in the dark in DCM for 30 min at least before being taken up with a 20 mL disposable syringe. The suspension of solid support in DCM was slowly injected from the disposable syringe (20 mL) into the FEP tubing using a syringe pump. The suspension was pushed through the FEP tubing into the photoreactor with additional 18 mL DCM (flow rate: 700 µL/min). The photocleavage took place inside the reactor while solid support travelled toward the exit point of the reactor. The suspension leaving the reactor was directed into a syringe equipped with polyethylene filter frit where the resin was filtered off and the solution containing the cleaved oligosaccharide is collected in a separate glass vial. The tubing as washed with 20 mL DCM (flow rate: 2 mL/min) until any remaining resin exited the reactor and the remaining oligosaccharide solution is collected. The tubing was reequilibrated with 20 mL DCM using a flow rate of 5 mL/min and the entire cleavage procedure was repeated. The combined solution that was collected in the photocleavage process was evaporated in vacuo and the crude material was analyzed by MALDI-TOF, and HPLC.

### Example 4: Synthesis of mannose tetramer via phosphate donor with temperature regulation via microwave heating.

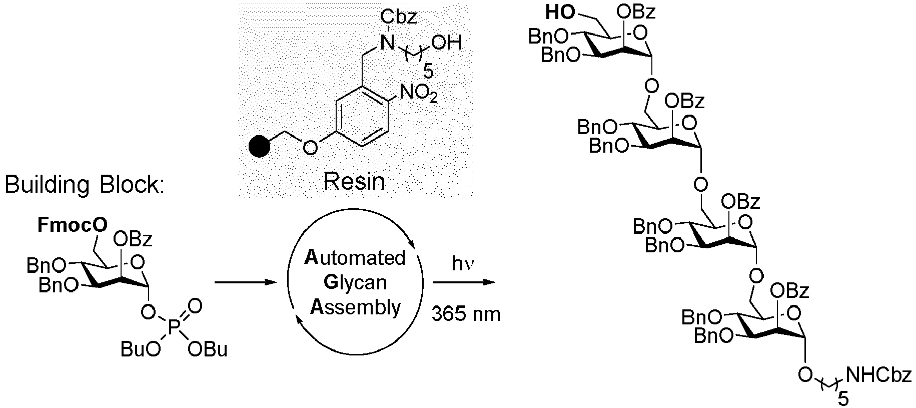

### Automated synthesis working modules

The timing and quantity of solvents/reagents transferred to the reaction vessel in each step is controlled by the software. The reagent delivery system is based on valve-pressured control in which the entire platform is constantly pressurized so that the specific solvent/reagent is transferred from the respective storage components by timing the opening and closing of the appropriate valves.

Module 1: Acidic Washing: The resin loaded into the reaction vessel is washed with DMF, THF, DCM (six times each with 3 mL for 15 s). The resin is swollen in 2 mL DCM, and the temperature of the reaction vessel was adjusted in the range of -22 °C to -20 °C by cooling device (when it is programmed to do so). For acidic washing 1 mL of the solution of 2% TMSOTf in DCM is delivered to the reaction vessel via the pre-cooling device which cools the solution to a temperature of -20 °C (when it is programmed to do so). After three minutes, the solution is drained. Finally, 3 mL DCM is added to the reaction vessel.

Module 2: Glycosylation: Phosphate building block is dissolved in the proper solvent mixture e.g. DCM (5 mL for one initial double cycle for the first coupling and three more single glycosylation cycles to build up the tetramer) in the designated building block storing component. The reaction vessel is set to reach the initial glycosylation temperature. During the adjustment of the temperature in the reactor vessel (when it is programmed to do so), the DCM in the reaction vessel is drained and 1 mL of phosphate building block (5.0 eq. in 1.0 mL DCM) is delivered from the building block storing component to the reaction vessel via the pre-cooling device which cools the solution of phosphate building block (when it is programmed to do so) to a temperature of -18 °C. After the set temperature in the range of -22 °C to -20 °C is reached the resin is incubated in the solution of phosphate building block for 10 min. Then 1.0 mL of the solution of 2% TMSOTf in DCM is delivered to the reaction vessel from the respective activator storing component via the pre-cooling device which cools the activator solution down to a temperature of -18 °C (when it is programmed to do so). The glycosylation mixture is incubated for 10 min in the temperature range of -22 °C to -20 °C. Keeping constant the active cooling action by microwave transparent coolant flowing in the jacket the linearly ramped to 0 °C (when it is programmed to do so) by the microwave radiation, adjusting the maximum radiation power to 180 W, and after reaching 0 °C the reaction mixture is incubated for an additional 10 min. Once incubation time is finished, the reaction mixture is drained and the resin is washed with DCE (once, 2 mL for 5 s).

Module 3: Capping: While the temperature of the active cooling element is kept in the range of -22 °C to -20 °C preparing for the next coupling cycle. The temperature of the reactor vessel is adjusted between 70 and 20 °C by microwave irradiation of the washing solvents and reagents adjusting the irradiation power. The resin is washed twice with DMF (3 mL for 15 s). 2 mL of Pyridine solution (10% in volume in DMF) was delivered and the resin is incubated for one minute. Up to this point the microwave irradiation power is adjust at 50W. The microwave irradiation power is then adjusted 150-180W to proceed with the resin is washed three times with DCM (2 mL for 15). The capping of the unreacted acceptor groups is done by delivering 4 mL of methanesulfonic acid (2% in volume) and acetic anhydride (10% in volume) in DCM. The resin and the reagents are incubated for 1 min; then 1 mL of DCM in added to dilute the solution and the incubation continues for another 1 min. The solution is drained from the reactor vessel and the resin is washed 3 times with DCM (2 mL for 15 s).

Module 3: Fmoc Deprotection: The temperature of the reactor vessel is adjusted between 70 and 20 °C by microwave irradiation of the washing solvents and reagents adjusting the irradiation power (40 W). The resin is washed with DMF (three times with 3 mL for 15 s), swollen in 2 mL DMF and the temperature of the reaction vessel is adjusted between 70-20 °C. For Fmoc deprotection the DMF is drained and 2 mL of a solution of 20% piperidine in DMF was delivered to the reaction vessel. After 1 min the reaction solution is drained from the reactor vessel. Then, the resin is washed with DMF (three times with 3 mL for 15 s) and DCM (five times with 3 mL). After this module the resin is ready for the next glycosylation cycle.

Resin functionalized with a photo-cleavable linker (64 mg; loading 0.392 mmol/g; 25.1 µmol) was loaded into the reaction vessel of the synthesizer and swollen in 2 mL DCM. The sequence of reaction steps for the formation of tetramannose was as follows:
1. Module 1 was performed with 1 mL TMSOTf solution at the temperature range of -22 °C to -20 °C (when it is programed to do so) for 3 min.
2. Module 2 was performed with 5 equiv Building Block and 2% TMSOTf in DCM solution.
3. Module 3 was carried out in two steps; first 2 mL of Pyridine solution (10% in volume in DMF); then in a second step with 4 mL of Methanesulfonic acid (2% in volume) and Acetic anhydride (10% in volume) in DCM.
4. Module 4 was carried out with 20% Piperidine in DMF.
5. After Module 1 took place the Module 2 repeated twice (for the first coupling). The Modules 3 and 4 were then performed.
6. Subsequently, modules 1-4 were repeated three times in the same manner as described in steps 1-4 in order to obtain a tetramer.

After buildup of the tetramer on the resin the oligosaccharide was cleaved from solid support in a photoreactor: A mercury lamp is turned on 30 min prior to the first cleavage event. The fluorinated-ethylene-propylene (FEP) tubing was washed with 20 mL DCM at a flow rate of 5 mL/min before cleavage. The solid support was pre-swelled in the dark in DCM for 30 min at least before being taken up with a 20 mL disposable syringe. The suspension of solid support in DCM was slowly injected from the disposable syringe (20 mL) into the FEP tubing using a syringe pump. The suspension was pushed through the FEP tubing into the photoreactor with additional 18 mL DCM (flow rate: 700 µL/min). The photocleavage took place inside the reactor while solid support travelled toward the exit point of the reactor. The suspension leaving the reactor was directed into a syringe equipped with polyethylene filter frit where the resin was filtered off and the solution containing the cleaved oligosaccharide is collected in a separate glass vial. The tubing as washed with 20 mL DCM (flow rate: 2 mL/min) until any remaining resin exited the reactor and the remaining oligosaccharide solution is collected. The tubing was reequilibrated with 20 mL DCM using a flow rate of 5 mL/min and the entire cleavage procedure was repeated. The combined solution that was collected in the photocleavage process was evaporated in vacuo and the crude material was analyzed by MALDI-TOF, and HPLC.

## Claims

1. A method for synthesizing oligo- and polysaccharides, comprising the following steps:
a) providing a solid support with at least one immobilized saccharide;
b) adding a further saccharide bearing at least one protecting group and a glycosylation reagent to the solid support in order to initiate a coupling reaction of the further saccharide to the saccharide immobilized on the solid support;
c) performing removal of the at least one protecting group of the further saccharide under microwave irradiation.

2. The method according to claim 1, wherein the saccharide immobilized on the solid support is a glycosyl acceptor comprising 1 to 20 monosaccharide units.

3. The method according to claim 1 or 2, wherein the further saccharide is a glycosyl donor comprising a glycal, epoxide or orthoester group or having a leaving group at the reducing end selected from halogen, -O-C(=NH)-CCl₃, -O-C(=NPh)-CF₃, -OAc, -SR⁵, -SO-Ph, -SO₂-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 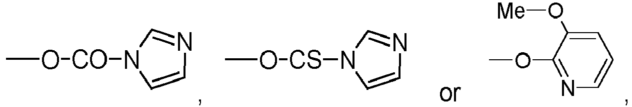 wherein R⁵ represents an alkyl or aryl group.

4. The method according to any one of claims 1 - 3, wherein the at least one protecting group of the further saccharide is a temporary protecting group selected from allyl, *p*-methoxybenzyl, 2-naphthylmethyl, tri-isopropylsilyl, *tert*-butyldimethylsilyl, *tert*-butylmethoxyphenylsilyl, triethylsilyl, trimethylsilyl, 2-trimethylsilylethoxymethyl, 9-fluorenylmethoxycarbonyl and levulinoyl.

5. The method according to any one of claims 1 - 4, wherein the immobilized saccharide comprises at least one permanent protecting group and/or the further saccharide comprises at least one permanent protecting group, in addition to the at least one protecting group, selected from acetyl, phenyl, benzyl, isopropylidene, benzylidene, benzoyl, *p*-methoxybenzyl, *p*-methoxybenzylidene, *p*-methoxyphenyl, *p*-bromobenzylidene, *p*-nitrophenyl, allyl, allyloxycarbonyl, monochloroacetyl, isopropyl, *p*-bromobenzyl, dimethoxytrityl, trityl, 2-naphthylmethyl, pivaloyl, triisopropylsilyl, *tert*-butyldimethylsilyl, *tert-*butyldiphenylsilyl, *tert*-butylmethoxyphenylsilyl, triethylsilyl, trimethylsilyl, 2-trimethylsilylethoxymethyl, 9-fluorenylmethoxycarbonyl, tert-butyloxycarbonyl, benzyloxymethyl, methyloxymethyl, *tert*-butyloxymethyl, methoxyethyl-oxymethyl, and levulinoyl.

6. The method according to any one of claims 1 - 5, wherein the solid support obtained in step b) is treated with a capping reagent.

7. The method according to claim 6, wherein the solid support obtained in step b) is treated with a capping reagent under microwave irradiation.

8. The method according to any one of claims 1 - 7, wherein the glycosylation reagent is a Lewis acid selected from: AgOTf, BF₃•OEt₂, trimethylsilyl trifluoromethanesulfonate, trifluoromethanesulfonic acid, trifluoromethanesulfonic anhydride, lanthanoid(III) triflates, NIS/AgOTf, NIS/TfOH or dimethyl(methylthio)sulfonium trifluoromethanesulfonate.

9. The method according to any one of claims 1 - 8, wherein the removal of the at least one protecting group is performed at a reaction temperature of at least 40°C.

10. The method according to any one of claims 1 - 9, wherein step b) is performed at a reaction temperature below 0°C and optionally under microwave irradiation.

11. The method according to any one of claims 1 - 10, wherein in step b) the glycosylation reagent is cooled to a temperature of at least 0°C before addition to the further saccharide and the solid support.

12. The method according to any one of claims 1 - 11 further comprising the steps:
d) cleaving the saccharide obtained in step c) from the solid support; e) purifying the saccharide obtained in step e).

13. A synthesizer for microwave-assisted automated multistep synthesis on a solid support comprising:
(a) a microwave transparent reaction vessel equipped with a temperature sensor,
(b) a microwave generator component,
(c) a reagent storing component,
(d) a reagent delivery system,
(e) a cooling device for cooling the microwave transparent reaction vessel,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.

14. A synthesizer for automated multistep synthesis on a solid support comprising:
(a) a reaction vessel,
(c) a reagent storing component comprising one or more reagent containers,
(d) a reagent delivery system configured to establish a fluid communication between each of the one or more reagent containers and the reaction vessel, wherein at least two fluid communications are separate of each other,
(e) a cooling device for cooling the reaction vessel,
(g) an inert gas delivery system comprising a gas container and a gas valve manifold establishing a fluid communication between each of the one or more reagent containers and the gas container, wherein at least two fluid communications are separate of each other.

15. The synthesizer for automated multistep synthesis on a solid support according to claim 14, wherein
the reaction vessel is a microwave transparent reaction vessel equipped with a temperature sensor,
the synthesizer for automated multistep synthesis on a solid support further comprising:
(b) a microwave generator component,
(f) a thermal controller for controlling the temperature inside the microwave transparent reaction vessel, the controller being connected to the cooling device, the temperature sensor and the microwave generator component, the controller being configured to control the temperature by adjusting the power output of the microwave generator component and by setting a fixed cooling temperature of the cooling device.
